# EUROPEAN PATENT SPECIFICATION

(11) **EP 3 887 370 B1**
(45) Date of publication and mention of the grant of the patent: **18.01.2023**
(21) Application number: 19809440.1
(22) Date of filing: 25.11.2019
(51) Int. Cl.: C07D 471/04, C07D 487/04, A61P 31/04, A61K 31/437

(54) **TRICYCLIC COMPOUNDS FOR THE TREATMENT AND PROPHYLAXIS OF BACTERIAL INFECTION**
TRICYCLISCHE VERBINDUNGEN ZUR BEHANDLUNG UND PROPHYLAXE EINER BAKTERIELLEN INFEKTION
COMPOSÉS TRICYCLIQUES POUR LE TRAITEMENT ET LA PROPHYLAXIE D'UNE INFECTION BACTÉRIENNE

(30) Priority: 27.11.2018 WO PCT/CN2018/117718
(43) Date of publication of application: 06.10.2021
(73) Proprietor: F. Hoffmann-La Roche AG, 4070 Basel (CH)
(72) Inventor: DEY, Fabian, 4070 Basel (CH); HU, Yimin, Shanghai, 201203 (CN); LIU, Yongqiang, Shanghai, 201203 (CN); SHEN, Hong, Shanghai, 201203 (CN); SHI, Houguang, Shanghai, 201203 (CN); TAN, Xuefei, Shanghai, 201203 (CN); YAN, Shixiang, Shanghai, 201203 (CN); ZHANG, Weixing, Shanghai, 201203 (CN); ZHANG, Zhiwei, Shanghai, 201203 (CN); ZHOU, Chengang, Shanghai, 201203 (CN); ZHOU, Mingwei, Shanghai, 201203 (CN); ZHU, Wei, Shanghai, 201203 (CN)
(74) Representative: Jochnowitz, Evan
(86) International application number: PCT/EP2019/082335
(87) International publication number: WO 2020/109190

(56) References cited:
- WO-A1-2012/125746
- WO-A1-2018/178041

## Description

The present invention relates to organic compounds useful for therapy and/or prophylaxis in a mammal, and in particular to inhibitors of DNA gyrase and/or topoisomerase IV useful for treatment and/or prophylaxis of bacterial infection.

### FIELD OF THE INVENTION

Bacterial infections pose a continuing medical problem because anti-bacterial drugs eventually engender resistance in the bacteria on which they are used. Bacterial resistance against virtually all current antibiotic drugs are increasing. Many forms of antibiotic resistance can even cross international boundaries and spread with remarkable speed. Thus novel classes of antibacterial compounds are urgently needed.

One target for development of anti-bacterial drugs has been DNA gyrase and topoisomerase IV (bacterial type IIA topoisomerases), which are essential to cell life, that solve DNA topological problems resulting from the replication, transcription, and recombination of DNA. DNA Gyrase controls DNA supercoiling and relieves topological stress that occurs when the DNA strands are untwisted such as during replication. Topoisomerase IV primarily resolves linked chromosome dimers at the conclusion of DNA replication. Both enzymes can introduce double stranded DNA breaks; pass a second DNA strand through the break and rejoining the broken strands. The activity of both enzymes is driven by the binding and hydrolysis of ATP. Bacterial DNA gyrase consists of two A (GyrA) and two B (GyrB) subunits. Binding and cleavage of the DNA is associated with GyrA, whereas ATP is bound and hydrolyzed by GyrB. Bacterial Topoisomerase IV is also a hetero-tetramer that consists of two C (ParC) and two E (ParE) subunits. The latter subunits bind ATP like GyrB in order to supply energy necessary for catalytic turnover of the enzymes.

Inhibition of DNA gyrase and topoisomerase IV has potential for the development of broad-spectrum antibiotics. The enzymes are highly conserved across a broad range of gram-positive and gram-negative pathogens. There are two classes of antibiotics that demonstrated such mechanism of action. The first, well-represented by the quinolones, inhibits GyrA and ParC subunits by stabilizing the cleaved DNA-enzyme complex, thus inhibiting overall gyrase function, leading to cell death. Novobiocin, the only marketed drug in the second class, exerts its effect by blocking the ATPase activity of the enzymes. Novobiocin was identified in 1950s. But its use declined rapidly and it was eventually withdrawn from the market, mainly due to its low permeability in many bacteria strains, rise of spontaneous resistance development, and the development of more effective drugs, such as penicillinase-stable penicillins and the first cephalosporins in 1960s and 1970s.

Recently, strong inhibition of both DNA gyrase and/or topoisomerase IV has been recognized to be important for low resistance development in bacterial strains treated by inhibitors of the enzymes. Inhibitors of bacterial DNA gyrase and/or topoisomerase IV with different mechanism of action compare to the widely used quinolones will exhibit minimal cross resistance, and will be potentially useful in combating quinolone resistance that has increased significantly in the past few years.

### SUMMARY OF THE INVENTION

The present invention relates to novel compounds of formula (I), 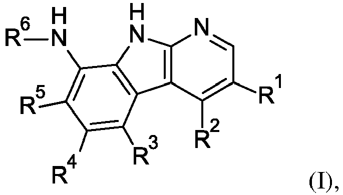 wherein
R¹ is oxopyridinyl; pyrazolo[1,5-a]pyrimidinyl; pyridinyl substituted by cyano, C₁₋₆alkoxycarbonyl or C₁₋₆alkylaminocarbonylC₁₋₆alkyl; pyrimidinyl substituted by C₁₋₆alkoxy or carboxyC₃₋₇cycloalkyl;
R² is (aminoC₁₋₆alkyl)C₃₋₇cycloalkyl;
   (C₁₋₆alkyl)₂amino;
   2,3,3a,4,6,6a-hexahydropyrrolo[2,3-c]pyrrolyl substituted by C₁₋₆alkyl;
   3,6-diazabicyclo[3.1.1]heptanyl;
   azabicyclo[3.1.0]hexanyl substituted by aminoC₁₋₆alkyl;
   azaspiro[2.4]heptanyl substituted by amino;
   azaspiro[3.3]heptanyl substituted by amino;
   azaspiro[3.4]octanyl substituted by amino;
   diazabicyclo[3.1.1]heptanyl;
   morpholinyl substituted by aminoC₁₋₆alkyl;
   piperidinyl substituted once or twice by substituents independently selected from amino, aminoC₁₋₆alkyl, C₁₋₆alkyl and halogen; or
   pyrrolidinyl substituted once, twice or three times by substituents independently selected from amino, aminoC₁₋₆alkyl, C₁₋₆alkyl and halogen;
R³ is H, C₁₋₆alkyl or halogen;
R⁴ is halogen or haloC₁₋₆alkyl;
R⁵ is H or halogen;
R⁶ is C₁₋₆alkyl;
or pharmaceutically acceptable salt thereof.

Objects of the present invention are novel compounds of formula (I), their manufacture, medicaments based on a compound in accordance with the invention and their production as well as the compounds of formula (I) for use as DNA gyrase and/or topoisomerase IV inhibitors and for use in the treatment or prophylaxis of bacterial infection. The compounds of formula (I) showed superior anti-bacterial activity. In addition, the compounds of formula (I) also showed good solubility and CC₅₀ profiles.

### DETAILED DESCRIPTION OF THE INVENTION

Unless otherwise defined, all technical and scientific terms used herein have the same meaning as commonly understood by one of ordinary skill in the art to which this invention belongs. Furthermore, the following definitions are set forth to illustrate and define the meaning and scope of the various terms used to describe the invention.

Any references in the description to methods of treatment refer to compounds, pharmaceutical compositions and medicaments of the present invention for use in a method for treatment of the human (or animal) body by therapy (or for diagnosis).

### DEFINITIONS

The term "C₁₋₆alkyl" denotes a saturated, linear or branched chain alkyl group containing 1 to 6, particularly 1 to 4 carbon atoms, for example methyl, ethyl, propyl, isopropyl, *n*-butyl, isobutyl, *tert-butyl* and the like. Particular "C₁₋₆alkyl" groups are methyl, ethyl and propyl.

The term "C₃₋₇cycloalkyl" denotes to a saturated carbon ring containing from 3 to 7 carbon atoms, particularly from 3 to 6 carbon atoms, for example, cyclopropyl, cyclobutyl, cyclopentyl, cyclohexyl, cycloheptyl and the like. Particular "C₃₋₇cycloalkyl" group is cyclopropyl.

The term "C₁₋₆alkoxy" denotes a group of the formula C₁₋₆alkyl-O-. Examples of C₁₋₆alkoxy group include, but not limited to, methoxy, ethoxy, n-propoxy, isopropoxy, n-butoxy, isobutoxy and *tert*-butoxy. Particular "C₁₋₆alkoxy" groups are methoxy, ethoxy and isopropoxy.

The term "halogen" and "halo" are used interchangeably herein and denote fluoro, chloro, bromo, or iodo.

The term "haloC₁₋₆alkyl" denotes an alkyl group wherein at least one of the hydrogen atoms of the alkyl group has been replaced by same or different halogen atoms, particularly fluoro atom. Examples of haloC₁₋₆alkyl include monofluoro-, difluoro-or trifluoro-methyl, -ethyl or -propyl, for example 3,3,3-trifluoropropyl, 2-fluoroethyl, 2,2,2-trifluoroethyl, fluoromethyl, difluoromethyl, trifluoromethyl and trifluoroethyl.

The term "carbonyl" denotes the group -C(O)-.

The term "pharmaceutically acceptable salts" denotes salts which are not biologically or otherwise undesirable. Pharmaceutically acceptable salts include both acid and base addition salts.

The term "pharmaceutically acceptable acid addition salt" denotes those pharmaceutically acceptable salts formed with inorganic acids such as hydrochloric acid, hydrobromic acid, sulfuric acid, nitric acid, carbonic acid, phosphoric acid, and organic acids selected from aliphatic, cycloaliphatic, aromatic, araliphatic, heterocyclic, carboxylic, and sulfonic classes of organic acids such as formic acid, acetic acid, propionic acid, glycolic acid, gluconic acid, lactic acid, pyruvic acid, oxalic acid, malic acid, maleic acid, maloneic acid, succinic acid, fumaric acid, tartaric acid, citric acid, aspartic acid, ascorbic acid, glutamic acid, anthranilic acid, benzoic acid, cinnamic acid, mandelic acid, embonic acid, phenylacetic acid, methanesulfonic acid, ethanesulfonic acid, *p*-toluenesulfonic acid, and salicyclic acid.

The term "pharmaceutically acceptable base addition salt" denotes those pharmaceutically acceptable salts formed with an organic or inorganic base. Examples of acceptable inorganic bases include sodium, potassium, ammonium, calcium, magnesium, iron, zinc, copper, manganese, and aluminum salts. Salts derived from pharmaceutically acceptable organic nontoxic bases includes salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperizine, piperidine, *N*-ethylpiperidine, and polyamine resins.

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, e.g., a human in need thereof.

### INHIBITORS OF DNA GYRASE AND/OR TOPOISOMERASE IV

The present invention relates to a compound of formula (I), 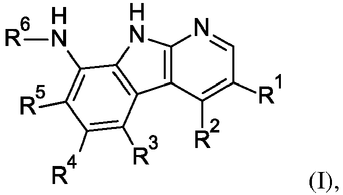 wherein
R¹ is oxopyridinyl; pyrazolo[1,5-a]pyrimidinyl; pyridinyl substituted by cyano, C₁₋₆alkoxycarbonyl or C₁₋₆alkylaminocarbonylC₁₋₆alkyl; pyrimidinyl substituted by C₁₋₆alkoxy or carboxyC₃₋₇cycloalkyl;
R² is (aminoC₁₋₆alkyl)C₃₋₇cycloalkyl;
   (C₁₋₆alkyl)₂amino;
   2,3,3a,4,6,6a-hexahydropyrrolo[2,3-c]pyrrolyl substituted by C₁₋₆alkyl;
   3,6-diazabicyclo[3.1.1]heptanyl;
   azabicyclo[3.1.0]hexanyl substituted by aminoC₁₋₆alkyl;
   azaspiro[2.4]heptanyl substituted by amino;
   azaspiro[3.3]heptanyl substituted by amino;
   azaspiro[3.4]octanyl substituted by amino;
   diazabicyclo[3.1.1]heptanyl;
   morpholinyl substituted by aminoC₁₋₆alkyl;
   piperidinyl substituted once or twice by substituents independently selected from amino, aminoC₁₋₆alkyl, C₁₋₆alkyl and halogen; or
   pyrrolidinyl substituted once, twice or three times by substituents independently selected from amino, aminoC₁₋₆alkyl, C₁₋₆alkyl and halogen;
R³ is H, C₁₋₆alkyl or halogen;
R⁴ is halogen or haloC₁₋₆alkyl;
R⁵ is H or halogen;
R⁶ is C₁₋₆alkyl;
or pharmaceutically acceptable salt thereof.

A further embodiment of present invention is (ii) a compound of formula (I), wherein
R¹ is oxopyridinyl; pyrazolo[1,5-a]pyrimidinyl; cyanopyridinyl; methoxycarbonylpyridinyl; methylaminocarbonylmethylpyridinyl; ethoxypyrimidinyl; methoxypyrimidinyl or carboxycyclopropylpyrimidinyl;
R² is (aminomethyl)azabicyclo[3.1.0]hexanyl; (aminomethyl)cyclopropyl; (aminomethyl)morpholinyl; (aminomethyl)piperidinyl; (aminomethyl)pyrrolidinyl; 3,6-diazabicyclo[3.1.1]heptanyl; aminoazaspiro[2.4]heptanyl; aminoazaspiro [3.3]heptanyl; aminoazaspiro [3.4] octanyl; aminopiperidinyl; aminopyrrolidinyl; diazabicyclo[3.1.1]heptanyl; difluoro(aminomethyl)pyrrolidinyl; dimethylamino; fluoro(aminomethyl)piperidinyl; fluoro(aminomethyl)pyrrolidinyl; methyl(aminomethyl)piperidinyl; methyl(aminomethyl)pyrrolidinyl or methyl-2,3,3a,4,6,6a-hexahydropyrrolo[2,3-c]pyrrolyl;
R³ is H, methyl or fluoro;
R⁴ is chloro, fluoro or trifluoromethyl;
R⁵ is H or fluoro;
R⁶ is ethyl or methyl;
or pharmaceutically acceptable salt thereof.

A further embodiment of present invention is (iii) a compound of formula (I) according to (i), or pharmaceutically acceptable salt thereof, wherein R¹ is cyanopyridinyl or C₁₋₆alkoxypyrimidinyl.

A further embodiment of present invention is (iv) a compound of formula (I) according to (i) to (iii), wherein R¹ is cyanopyridinyl or methoxypyrimidinyl.

A further embodiment of present invention is (v) a compound of formula (I) according to (i) to (iv), wherein R² is (C₁₋₆alkyl)₂amino; aminoazaspiro[2.4]heptanyl; piperidinyl substituted once or twice by substituents independently selected from amino and aminoC₁₋₆alkyl; or pyrrolidinyl substituted once or twice by substituents independently selected from amino and aminoC₁₋₆alkyl.

A further embodiment of present invention is (vi) a compound of formula (I), according to (i) to (v), wherein R² is dimethylamino, aminopiperidinyl, (aminomethyl)piperidinyl, methyl(aminomethyl)piperidinyl, aminopyrrolidinyl, (aminomethyl)pyrrolidinyl or methyl(aminomethyl)pyrrolidinyl.

A further embodiment of present invention is (vii) a compound of formula (I) according to (i) to (vi), wherein
R¹ is cyanopyridinyl or C₁₋₆alkoxypyrimidinyl;
R² is (C₁₋₆alkyl)₂amino; aminoazaspiro[2.4]heptanyl; piperidinyl substituted once or twice by substituents independently selected from amino and aminoC₁₋₆alkyl; or pyrrolidinyl substituted once or twice by substituents independently selected from amino and aminoC₁₋₆alkyl;
R³ is halogen;
R⁴ is halogen;
R⁵ is H;
R⁶ is C₁₋₆alkyl;
or pharmaceutically acceptable salt thereof.

A further embodiment of present invention is (viii) a compound of formula (I), according to (i) to (vii), wherein
R¹ is cyanopyridinyl or methoxypyrimidinyl;
R² is dimethylamino, aminopiperidinyl, (aminomethyl)piperidinyl, methyl(aminomethyl)piperidinyl, aminopyrrolidinyl, (aminomethyl)pyrrolidinyl or methyl(aminomethyl)pyrrolidinyl;
R³ is fluoro;
R⁴ is fluoro or chloro;
R⁵ is H;
R⁶ is methyl or ethyl;
or pharmaceutically acceptable salt thereof.

Another embodiment of present invention is that (ix) particular compounds of formula (I) are the following:
5-[4-[(3*R*)-3-Aminopyrrolidin-1-yl]-6-fluoro-8-(methylamino)-9H-pyrido[2,3-b]indol-3-yl]pyridine-3-carbonitrile;
5-[4-(3-Aminopyrrolidin-1-yl)-8-(ethylamino)-5,6-difluoro-9H-pyrido[2,3-b]indol-3-yl] pyridine-3-carbonitrile;
5-[4-(3-Aminopyrrolidin-1-yl)-6-chloro-8-(ethylamino)-9H-pyrido[2,3-b]indol-3-yl]pyridine-3-carbonitrile;
5-[4-[(3*R*)-3-aminopyrrolidin-1-yl]-8-(ethylamino)-5,6-difluoro-9H-pyrido[2,3-b]indol-3-yl]pyridine-3-carbonitrile;
5-[4-[(3*R*)-3-aminopyrrolidin-1-yl]-6-chloro-8-(ethylamino)-9H-pyrido[2,3-b]indol-3-yl]pyridine-3-carbonitrile;
5-[4-[2-(Aminomethyl)pyrrolidin-1-yl]-8-(ethylamino)-6-fluoro-9H-pyrido[2,3-b]indol-3-yl]pyridine-3-carbonitrile;
5-[4-[3-(Aminomethyl)pyrrolidin-1-yl]-8-(ethylamino)-5,6-difluoro-9H-pyrido[2,3-b]indol-3-yl]pyridine-3-carbonitrile;
4-[3-(aminomethyl)pyrrolidin-1-yl]-N-ethyl-5,6-difluoro-3-(2-methoxypyrimidin-5-yl)-9H-pyrido[2,3-b]indol-8-amine;
4-(3-Aminopyrrolidin-1-yl)-N-ethyl-5,6-difluoro-3-pyrazolo[1,5-a]pyrimidin-6-yl-9H-pyrido[2,3-b]indol-8-amine;
5-[4-[3-(aminomethyl)-3-fluoro-pyrrolidin-1-yl]-8-(ethylamino)-6-fluoro-9H-pyrido[2,3-b]indol-3-yl]pyridine-3-carbonitrile;
5-[8-(ethylamino)-6-fluoro-4-[*cis*-2-amino-5-azaspiro[2.4]heptan-5-yl]-9H-pyrido[2,3-b]indol-3-yl]pyridine-3-carbonitrile;
5-[8-(ethylamino)-6-fluoro-4-[*trans*-2-amino-5-azaspiro[2.4]heptan-5-yl]-9H-pyrido[2,3-b]indol-3-yl]pyridine-3-carbonitrile;
5-[4-[3-(Aminomethyl)pyrrolidin-1-yl]-5,6-difluoro-8-(methylamino)-9H-pyrido[2,3-b]indol-3-yl]pyridine-3-carbonitrile;
5-[4-[3-(Aminomethyl)-1-piperidyl]-8-(ethylamino)-6-fluoro-9H-pyrido[2,3-b]indol-3-yl]pyridine-3-carbonitrile;
5-[4-[(3*R*)-3-Aminopyrrolidin-1-yl]-5,6-difluoro-8-(methylamino)-9H-pyrido[2,3-b]indol-3-yl]pyridine-3-carbonitrile;
5-[6-fluoro-8-(methylamino)-4-[*cis*-2-amino-5-azaspiro[2.4]heptan-5-yl]-9H-pyrido[2,3-b]indol-3-yl]pyridine-3-carbonitrile;
5-[6-fluoro-8-(methylamino)-4-[*trans*-2-amino-5-azaspiro[2.4]heptan-5-yl]-9H-pyrido[2,3-b]indol-3-yl]pyridine-3-carbonitrile;
4-[(3*R*)-3-Aminopyrrolidin-1-yl]-N-ethyl-5,6-difluoro-3-(2-methoxypyrimidin-5-yl)-9H-pyrido[2,3-b]indol-8-amine;
4-[3-(Aminomethyl)pyrrolidin-1-yl]-5,6-difluoro-3-(2-methoxypyrimidin-5-yl)-N-methyl-9H-pyrido[2,3-b]indol-8-amine;
5-[4-(3-Aminopyrrolidin-1-yl)-8-(ethylamino)-6-(trifluoromethyl)-9H-pyrido[2,3-b]indol-3-yl]pyridine-3-carbonitrile;
5-[4-[3-(Aminomethyl)-1-piperidyl]-5,6-difluoro-8-(methylamino)-9H-pyrido[2,3-b]indol-3-yl]pyridine-3-carbonitrile;
5-[4-[3-(Aminomethyl)-3-fluoro-pyrrolidin-1-yl]-5,6-difluoro-8-(methylamino)-9H-pyrido[2,3-b]indol-3-yl]pyridine-3-carbonitrile;
5-[4-[3-(Aminomethyl)-3-fluoro-pyrrolidin-1-yl]-6-fluoro-8-(methylamino)-9H-pyrido[2,3-b]indol-3-yl]pyridine-3-carbonitrile;
4-[3-(aminomethyl)-3-fluoro-pyrrolidin-1-yl]-6-fluoro-3-(2-methoxypyrimidin-5-yl)-N-methyl-9H-pyrido[2,3-b]indol-8-amine;
4-[(3*S*)-3-(aminomethyl)-1-piperidyl]-5,6-difluoro-3-(2-methoxypyrimidin-5-yl)-N-methyl-9H-pyrido[2,3-b]indol-8-amine;
4-[3-(aminomethyl)-3-methyl-1-piperidyl]-5,6-difluoro-3-(2-methoxypyrimidin-5-yl)-N-methyl-9H-pyrido[2,3-b]indol-8-amine;
4-[1-(aminomethyl)-3-azabicyclo[3.1.0]hexan-3-yl]-5,6-difluoro-3-(2-methoxypyrimidin-5-yl)-N-methyl-9H-pyrido[2,3-b]indol-8-amine;
4-[3-(aminomethyl)-3-fluoro-pyrrolidin-1-yl]-5,6-difluoro-3-(2-methoxypyrimidin-5-yl)-N-methyl-9H-pyrido[2,3-b]indol-8-amine;
5-[4-[*trans*-2-amino-5-azaspiro[2.4]heptan-5-yl]-5,6-difluoro-8-(methylamino)-9H-pyrido[2,3-b]indol-3-yl]pyridine-3-carbonitrile;
5-[4-[*cis*-2-amino-5-azaspiro[2.4]heptan-5-yl]-5,6-difluoro-8-(methylamino)-9H-pyrido[2,3-b]indol-3-yl]pyridine-3-carbonitrile;
5-[4-[2-(aminomethyl)morpholin-4-yl]-5,6-difluoro-8-(methylamino)-9H-pyrido[2,3-b]indol-3-yl]pyridine-3-carbonitrile;
5-[4-[1-(aminomethyl)-3-azabicyclo[3.1.0]hexan-3-yl]-5,6-difluoro-8-(methylamino)-9H-pyrido[2,3-b]indol-3-yl]pyridine-3-carbonitrile;
4-[2-(aminomethyl)morpholin-4-yl]-5,6-difluoro-3-(2-methoxypyrimidin-5-yl)-N-methyl-9H-pyrido[2,3-b]indol-8-amine;
4-[*cis*-2-amino-5-azaspiro[2.4]heptan-5-yl]-5,6-difluoro-3-(2-methoxypyrimidin-5-yl)-N-methyl-9H-pyrido[2,3-b]indol-8-amine;
4-[*trans-*2-amino-5-azaspiro[2.4]heptan-5-yl]-5,6-difluoro-3-(2-methoxypyrimidin-5-yl)-N-methyl-9H-pyrido[2,3-b]indol-8-amine;
4-[3-(aminomethyl)-3-methyl-pyrrolidin-1-yl]-5,6-difluoro-3-(2-methoxypyrimidin-5-yl)-N-methyl-9H-pyrido[2,3-b]indol-8-amine;
4-[2-(aminomethyl)pyrrolidin-1-yl]-5,6-difluoro-3-(2-methoxypyrimidin-5-yl)-N-methyl-9H-pyrido[2,3-b]indol-8-amine;
5-[4-[2-(aminomethyl)pyrrolidin-1-yl]-5,6-difluoro-8-(methylamino)-9H-pyrido[2,3-b]indol-3-yl]pyridine-3-carbonitrile;
5-[4-[(3*S*)-3-(aminomethyl)-1-piperidyl]-5,6-difluoro-8-(methylamino)-9H-pyrido[2,3-b]indol-3-yl]pyridine-3-carbonitrile;
Methyl 5-[4-[(3*S*)-3-(aminomethyl)-1-piperidyl]-5,6-difluoro-8-(methylamino)-9H-pyrido[2,3-b]indol-3-yl]pyridine-3-carboxylate;
5-(4-(3,6-diazabicyclo[3.1.1]heptan-3-yl)-5,6-difluoro-8-(methylamino)-9H-pyrido[2,3-b]indol-3-yl)nicotinonitrile;
5-(4-(3-(aminomethyl)pyrrolidin-1-yl)-8-(ethylamino)-6-fluoro-5-methyl-9H-pyrido[2,3-b]indol-3-yl)nicotinonitrile;
5-(4-(3-aminopiperidin-1-yl)-5,6-difluoro-8-(methylamino)-9H-pyrido[2,3-b]indol-3-yl)nicotinonitrile;
4-(3-aminopiperidin-1-yl)-5,6-difluoro-3-(2-methoxypyrimidin-5-yl)-N-methyl-9H-pyrido[2,3-b]indol-8-amine;
5-[4-(7-amino-2-azaspiro[3.3]heptan-2-yl)-5,6-difluoro-8-(methylamino)-9H-pyrido[2,3-b]indol-3-yl]pyridine-3-carbonitrile;
5-(4-(1-amino-6-azaspiro[3.4]octan-6-yl)-5,6-difluoro-8-(methylamino)-9H-pyrido[2,3-b]indol-3-yl)nicotinonitrile;
4-[*cis-*5-methyl-2,3,3a,4,6,6a-hexahydropyrrolo[2,3-c]pyrrol-1-yl]-5,6-difluoro-N-methyl-3-pyrazolo[1,5-a]pyrimidin-6-yl-9H-pyrido[2,3-b]indol-8-amine;
4-[4-(dimethylamino)-5,6-difluoro-8-(methylamino)-9H-pyrido[2,3-b]indol-3-yl]-1H-pyridin-2-one;
(*S*)-4-(3-(aminomethyl)piperidin-1-yl)-N-ethyl-6-fluoro-3-(2-methoxypyrimidin-5-yl)-9H-pyrido[2,3-b]indol-8-amine;
(*S*)-4-(3-(aminomethyl)piperidin-1-yl)-N-ethyl-5,6-difluoro-3-(2-methoxypyrimidin-5-yl)-9H-pyrido[2,3-b]indol-8-amine;
5-[4-[3-(aminomethyl)-3-fluoro-1-piperidyl]-5,6-difluoro-8-(methylamino)-9H-pyrido[2,3-b]indol-3-yl]pyridine-3-carbonitrile;
2-[4-[4-(dimethylamino)-5,6-difluoro-8-(methylamino)-9H-pyrido[2,3-b]indol-3-yl]-2-pyridyl]-N-methyl-acetamide;
4-(3,6-diazabicyclo[3.1.1]heptan-6-yl)-5,6-difluoro-3-(2-methoxypyrimidin-5-yl)-N-methyl-9H-pyrido[2,3-b]indol-8-amine;
5-(4-(3,6-diazabicyclo[3.1.1]heptan-6-yl)-5,6-difluoro-8-(methylamino)-9H-pyrido[2,3-b]indol-3-yl)nicotinonitrile;
4-(3,6-diazabicyclo[3.1.1]heptan-6-yl)-3-(2-ethoxypyrimidin-5-yl)-6-fluoro-N-methyl-9H-pyrido[2,3-b]indol-8-amine;
5-(4-(3,6-diazabicyclo[3.1.1]heptan-6-yl)-6-fluoro-8-(methylamino)-9H-pyrido[2,3-b]indol-3-yl)nicotinonitrile;
4-(3,6-diazabicyclo[3.1.1]heptan-6-yl)-6-fluoro-3-(2-methoxypyrimidin-5-yl)-N-methyl-9H-pyrido[2,3-b]indol-8-amine;
5,6-difluoro-N4,N4,N8-trimethyl-3-pyrazolo[1,5-a]pyrimidin-6-yl-9H-pyrido[2,3-b]indole-4,8-diamine;
5,6-Difluoro-3-(2-methoxypyrimidin-5-yl)-N-methyl-4-[(3aS,6aS)-5-methyl-2,3,3a,4,6,6a-hexahydropyrrolo[2,3-c]pyrrol-1-yl]-9H-pyrido[2,3-b]indol-8-amine;
1-[5-[4-(dimethylamino)-8-(ethylamino)-6-fluoro-9H-pyrido[2,3-b]indol-3-yl]pyrimidin-2-yl]cyclopropanecarboxylic acid;
1-[5-[4-(dimethylamino)-5,6-difluoro-8-(methylamino)-9H-pyrido[2,3-b]indol-3-yl]pyrimidin-2-yl]cyclopropanecarboxylic acid;
1-(5-(4-(dimethylamino)-6,7-difluoro-8-(methylamino)-9H-pyrido[2,3-b]indol-3-yl)pyrimidin-2-yl)cyclopropanecarboxylic acid;
5-[4-[4-(Aminomethyl)-3,3-difluoro-pyrrolidin-1-yl]-6-fluoro-8-(methylamino)-9H-pyrido[2,3-b]indol-3-yl]pyridine-3-carbonitrile;
4-[4-(aminomethyl)-3,3-difluoro-pyrrolidin-1-yl]-6-fluoro-3-(2-methoxypyrimidin-5-yl)-N-methyl-9H-pyrido[2,3-b]indol-8-amine;
4-[4-(aminomethyl)-3,3-difluoro-pyrrolidin-1-yl]-5,6-difluoro-3-(2-methoxypyrimidin-5-yl)-N-methyl-9H-pyrido[2,3-b]indol-8-amine;
5-[4-[4-(aminomethyl)-3,3-difluoro-pyrrolidin-1-yl]-5,6-difluoro-8-(methylamino)-9H-pyrido[2,3-b]indol-3-yl]pyridine-3-carbonitrile;
4-(1-(aminomethyl)cyclopropyl)-6-fluoro-3-(2-methoxypyrimidin-5-yl)-N-methyl-9H-pyrido[2,3-b]indol-8-amine;
*Cis*-4-(2-amino-5-azaspiro[2.4]heptan-5-yl)-5,6-difluoro-N-methyl-3-pyrazolo[1,5-a]pyrimidin-6-yl-9H-pyrido[2,3-b]indol-8-amine;
*Trans*-4-(2-Amino-5-azaspiro[2.4]heptan-5-yl)-5,6-difluoro-N-methyl-3-pyrazolo[1,5-a]pyrimidin-6-yl-9H-pyrido[2,3-b]indol-8-amine;
4-[(2R,3R)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine;
4-[(2S,3S)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine;
4-[(2S,3R)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine; and
4-[(2R,3S)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine;
or pharmaceutically acceptable salt thereof.

### SYNTHESIS

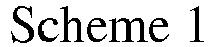

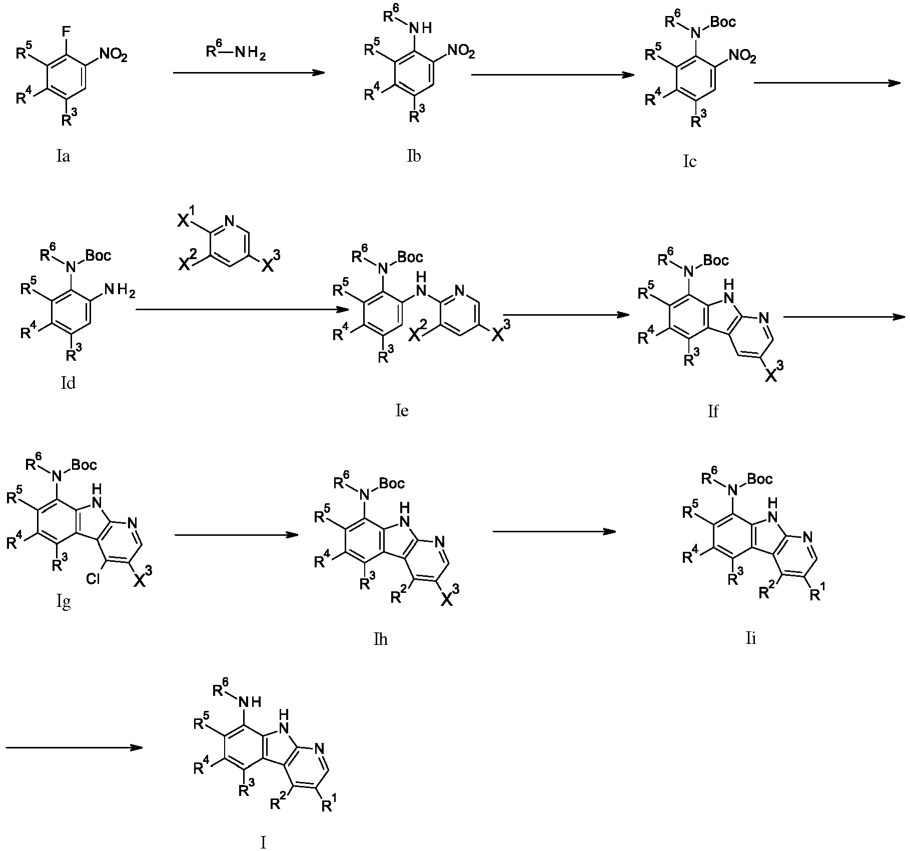

X¹, X² and X³ are halogen.

Compound of formula (I) can be prepared according to Scheme 1. Nucleophilic substitution of ortho-fluoro nitrobenzene (Ia) with amine R⁶-NH₂ affords aniline (Ib). The aniline (Ib) can be protected with di-tert-butyl carbonate to give the protected aniline (Ic). The nitro group in aniline (Ic) can be reduced by a reducing agent, such as H₂ with palladium catalysts, to give the compound of formula (Id). Coupling of the compound of formula (Id) with tri-halogenated pyridine can be achieved using palladium catalysts and phosphine ligands to give compound of formula (Ie). Cyclization of compound of formula (Ie) using palladium catalysts and phosphine ligands gives compound of formula (If). Compound of formula (Ig) can be obtained by chloronation of compound of formula (If) through oxidation of the pyridine followed by treatment of POCl₃. Coupling of compound of formula (Ig) to introduce R² can be achieved either through nucleophilic substitution with amine and a base for certain C-N bond formation (with R² bearing a nucleophilic N), or a Buchwald-Hartwig Cross Coupling Reaction for certain C-N bond formation (with R² bearing a basic N), or a palladium catalyzed Suzuki coupling for C-C bond formation (with R² as a boronic ester or boronic acid), to give compound of formula (Ih). Further coupling of compound of formula (Ih) to introduce R¹ can be achieved using a palladium catalyzed Suzuki coupling to give compound of formula (Ii). Deprotection of compound of formula (Ii) in the presence of an acid, such as trifluoroacetic acid, affords compound of formula (I).

This invention also relates to a process for the preparation of a compound of formula (I) comprising the reaction of compound of formula (Ii), 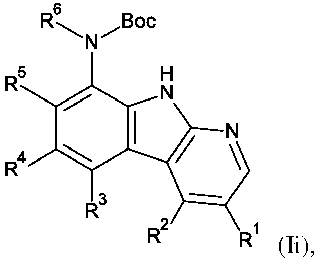
with an acid, which can be for example trifluoroacetic acid;
wherein R¹ to R⁶ are defined above, X¹, X² and X³ are halogen. A compound of formula (I) when manufactured according to the above process is also an object of the invention.

### PHARMACEUTICAL COMPOSITIONS AND ADMINISTRATION

Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compounds of formula (I) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to reduced bacterial load or improve host survival through the inhibition of bacterial DNA gyrase and/or Topoisomerase IV. For example, such amount may be below the amount that is toxic to normal cells, or the mammal as a whole.

In one example, the pharmaceutically effective amount of the compound of the invention administered parenterally per dose will be in the range of about 0.1 to 1000 mg/kg, alternatively about 1 to 100 mg/kg of patient body weight per day. In another embodiment, oral unit dosage forms, such as tablets and capsules, preferably contain from about 5 to about 5000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, e.g., Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 10 to 500 mg of the compound of the invention compounded with about 40 to 400mg anhydrous lactose, about 5 to 50 mg sodium croscarmellose, about 5 to 50 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 to 1000 mg) of the invention in a suitable buffer solution, e.g. a phosphate buffer, adding a tonicifier, e.g. a salt such sodium chloride, if desired. The solution may be filtered, e.g., using a 0.2 micron filter, to remove impurities and contaminants.

An embodiment, therefore, includes a pharmaceutical composition comprising a compound of formula (I), or a stereoisomer or pharmaceutically acceptable salt thereof. In a further embodiment includes a pharmaceutical composition comprising a compound of formula (I), or a stereoisomer or pharmaceutically acceptable salt thereof, together with a pharmaceutically acceptable carrier or excipient.

Another embodiment includes a pharmaceutical composition comprising a compound of formula (I) for use in the treatment and/or prophylaxis of bacterial infections.

In some embodiments, the compounds of this invention may be administered, as part of a single or multiple dosage regimen, orally, parenterally, by inhalation spray, topically, rectally, nasally, buccally, vaginally, or via an implanted reservoir. The term parenteral as used includes subcutaneous, intracutaneous, intravenous, intramuscular, intra-articular, intrasynovial, intrasternal, intrathecal, intralesional and intracranial injection or infusion techniques. Typically, the pharmaceutical compositions of the invention will be administered from about 1 to 5 times per day or alternatively, as a continuous infusion upon improvement of a patient's condition.

### INDICATIONS AND METHODS OF TREATMENT

The compounds of the invention are useful for treatment and/or prophylaxis of bacterial infection in humans or other animals by administering to the subject in need of a therapeutically effective amount of compound of formula (I), or a pharmaceutically acceptable salt, or enantiomer or diastereomer thereof. The compounds and methods of the invention are particularly well suited for human patients infected by pathogens that include *Staphylococcus aureus, Escherichia coli, Klebsiella pneumoniae, Acinetobacter baumannii* and *Pseudomonas aeruginosa.* Examples of bacterial organisms that may also be controlled by the compounds of the invention include, but not limited to, the following Gram-Positive and Gram-Negative organisms: *Streptococcus pneumoniae, Streptococcus pyogenes, Enterococcus faecalis, Enterococcus faecium, Enterobacter spp.* species, *Proteus spp.* species, *Serratia marcescens, Staphylococcus aureus, Coag. Neg. Staphylococci, Haemophilus influenzae, Bacillus anthraces, Mycoplasma pneumoniae, Moraxella catarrhalis, Chlamydophila pneumoniae, Chlamydia trachomatis, Legionella pneumophila, Mycobacterium tuberculosis, Helicobacter pylori, Staphylococcus saprophyticus, Staphylococcus epidermidis, Francisella tularensis, Yersinia pestis, Clostridium difficile, Bacteroides spp.* species *Neisseria gonorrhoeae, Neisseria meningitidis, Burkholderia pseudomallei, Burkholderia mallei, Borrelia burgdorferi, Mycobacterium avium complex, Mycobacterium abscessus, Mycobacterium kansasii and Mycobacterium ulcerans.*

Examples of bacterial infections may include, but not limited to, upper respiratory infections, lower respiratory infections, ear infections, pleuropulmonary and bronchial infections, complicated urinary tract infections, uncomplicated urinary tract infections, intra-abdominal infections, cardiovascular infections, a blood stream infection, sepsis, bacteremia, CNS infections, skin and soft tissue infections, GI infections, bone and joint infections, genital infections, eye infections, or granulomatous infections. Examples of specific bacterial infections include, but not limited to, uncomplicated skin and skin structure infections (uSSSI), complicated skin and skin structure infections (cSSSI), catheter infections, pharyngitis, sinusitis, otitis extema, otitis media, bronchitis, empyema, pneumonia, community-acquired bacterial pneumoniae (CABP), hospital-acquired pneumonia (HAP), hospital-acquired bacterial pneumonia, ventilator-associated pneumonia (VAP), diabetic foot infections, vancomycin resistant enterococci infections, cystitis and pyelonephritis, renal calculi, prostatitis, peritonitis, complicated intra-abdominal infections (cIAI) and other inter-abdominal infections, dialysis-associated peritonitis, visceral abscesses, endocarditis, myocarditis, pericarditis, transfusion-associated sepsis, meningitis, encephalitis, brain abscess, osteomyelitis, arthritis, genital ulcers, urethritis, vaginitis, cervicitis, gingivitis, conjunctivitis, keratitis, endophthalmitisa, an infection in cystic fibrosis patients or an infection of febrile neutropenic patients.

Furthermore, the invention relates to a compound of formula (I) for the treatment and/or prophylaxis of bacterial infection. The invention relates to the use of a compound of formula (I) for the preparation of a medicament for the treatment and/or prophylaxis of bacterial infection. Also disclosed is a method for the treatment or prophylaxis of bacterial infection which method comprises administering an effective amount of a compound of formula (I), or pharmaceutically acceptable salt, or enantiomer or diastereomer thereof.

### EXAMPLES

The invention will be more fully understood by reference to the following examples. They should not, however, be construed as limiting the scope of the invention.

### ABBREVIATIONS

Abbreviations used herein are as follows:
- [α]D₂₀: optical rotation at 20 degrees Celsius
- BINAP: 2,2'-Bis(diphenylphosphino)-1,1'-binaphthalene
- BOMC1: benzylchloromethyl ester
- calc'd: calculated
- CC₅₀: concentration results in the death of 50 percent of the cells
- Ct: cycle threshold
- d: day
- DBU: 1,8-Diazabicyclo[5.4.0]undec-7-ene
- DCM: dichloromethane
- DIAD: diisopropyl azodicarboxylate
- DIPEA: N,N-diisopropylethylamine
- EtOAc or EA: ethyl acetate
- h(s) or hr(s): hour
- HATU:: 1-[Bis(dimethylamino)methylene]-1H-1,2,3-triazolo[4,5-b]pyridinium 3-oxid hexafluorophosphate
- HPLC:: high performance liquid chromatography
- HPLC-UV:: high performance liquid chromatography with ultraviolet detector
- LAH: lithium aluminum hydride
- LDA: lithium diisopropylamide
- MIC: minimum inhibitory concentration
- OD: optical density
- PBS: phosphate buffered saline
- prep-HPLC: preparative high performance liquid chromatography
- qPCR: quantitative polymerase chain reaction
- qRT-PCR: quantitative real-time polymerase chain reaction
- rel: relative
- rt: room temperature
- SFC: supercritical fluid chromatography
- TFA: trifluoroacetic acid
- UV: ultraviolet detector

### GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel Brand and pore size: i) KP-SIL 60 A, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using X Bridge^{™} Perp C₁₈ (5 µm, OBD^{™} 30 × 100 mm) column or SunFire^{™} Perp C₁₈ (5 µm, OBD^{™} 30 × 100 mm) column.

Chiral Separation was conducted on Thar 350 preparative SFC using ChiralPak AD-10µ (200 × 50 mm I.D.) with mobile phase A for CO₂ and B for ethanol.LC/MS spectra were obtained using a Waters UPLC-SQD Mass. Standard LC/MS conditions were as follows (running time: 3 minutes):
Acidic condition: A: 0.1% formic acid and 1% acetonitrile in H₂O; B: 0.1% formic acid in acetonitrile;
Basic condition: A: 0.05% NH₃·H₂O in H₂O; B: acetonitrile.
Mass spectra (MS): generally only ions which indicate the parent mass are reported, and unless otherwise stated the mass ion quoted is the positive mass ion (M+H)⁺.

NMR Spectra were obtained using Bruker Avance 400MHz.

All reactions involving air-sensitive reagents were performed under an argon atmosphere. Reagents were used as received from commercial suppliers without further purification unless otherwise noted.

### PREPARATIVE EXAMPLES

### Intermediate A1 tert-Butyl N-(3,4-dichloro-6-fluoro-9H-pyrido[2,3-b]indol-8-yl)-N-methyl-carbamate

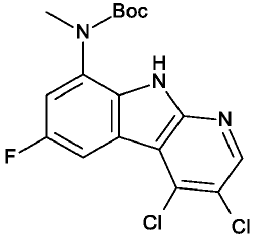

The titled compound was synthesized according to the following scheme:

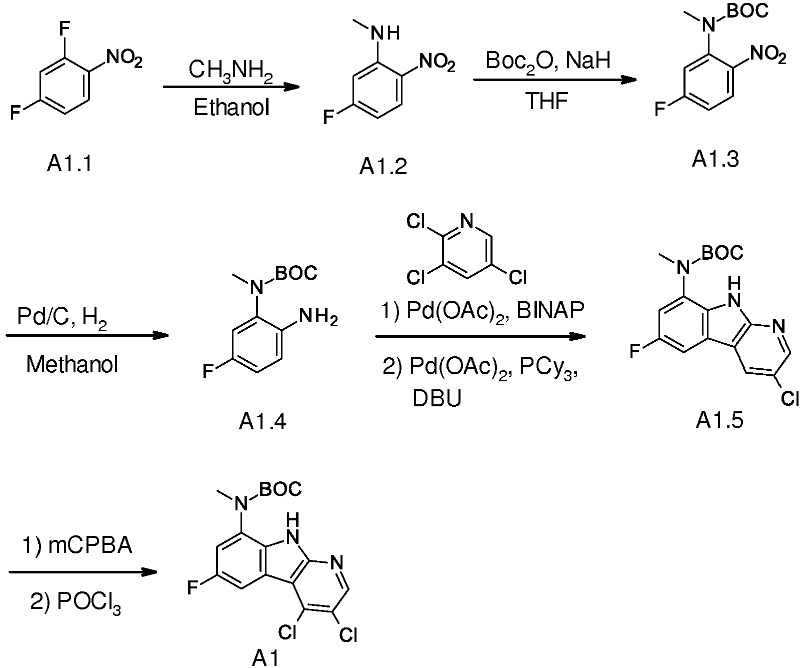

### Step (a) Preparation of 5-Fluoro-N-methyl-2-nitro-aniline (compound A1.2)

Methylamine solution (355 g, 2.866 mol, 25% in ethanol) was added dropwise to 2,4-difluoronitrobenzene (147 g, 0.924 mol) at 0 °C over 15 min. After completion of the addition, the reaction mixture was stirred at 0 °C for 2 hrs. The solution was diluted with ethanol (500 mL) and poured into 2 L of ice-water. The resulting precipitates were collected by filtration and dried *in vacuo* to give 5-fluoro-N-methyl-2-nitro-aniline (143 g, 63% yield) as a yellow solid.

### Step (b) Preparation of tert-Butyl N-(5-fluoro-2-nitro-phenyl)-N-methyl-carbamate (compound A1.3)

To the suspension of sodium hydride (141 g, 3.5 mol, 60 % dispersion in mineral oil) in dry tetrahydrofuran (2.0 L) was added 5-fluoro-N-methyl-2-nitro-aniline (60 g, 0.35 mol, compound A1.2) portion wise at 0 °C. The solution was stirred at 0 °C for 1 h. Then the solution of di-*tert*-butyl dicarbonate (115 g, 0.53 mol) in tetrahydrofuran (0.5 L) was added dropwise and the reaction continued for another 15 hrs at 15 °C. The reaction mixture was poured into 1.6 L of ice-water and extracted with EtOAc (1.6 L) two times. The combined organic layer was dried over sodium sulfate and concentrated *in vacuo.* The crude product was purified by flash chromatography (silica gel, 1-5% ethyl acetate in petroleum ether) to give *tert-butyl* N-(5-fluoro-2-nitro-phenyl)-N-methyl-carbamate (70 g, 73% yield) as a yellow solid. MS (ESI): 293.0 ([M+Na]⁺), 171.0 ([M-C₄H₈-CO₂+H]⁺).

### Step (c) Preparation of tert-Butyl N-(2-amino-5-fluoro-phenyl)-N-methyl-carbamate (compound A1.4)

To the solution of *tert-butyl* N-(5-fluoro-2-nitro-phenyl)-N-methyl-carbamate (70.0 g, 259 mmol, compound A1.3) in methanol (1.0 L) was added palladium on carbon (5.0 g, 10 wt. % loading). The reaction mixture was stirred at 16 °C for 18 h under H₂ atmosphere (50 psi). The remaining palladium catalyst was removed by filtration. The filtrate was concentrated *in vacuo* to give *tert-butyl* N-(2-amino-5-fluoro-phenyl)-N-methyl-carbamate (60 g, 96% yield) as a white solid. MS (ESI): 263.1 ([M+Na]⁺), 185.0 ([M-C₄H₈ +H]⁺), 141.0 ([M-C₄H₈-CO₂+H]⁺).

### Step (d) Preparation of tert-Butyl N-(3-chloro-6-fluoro-9H-pyrido[2,3-blindol-8-yl)-N-methylcarbamate (compound A1.5)

To the solution of *tert-butyl* N-(2-amino-5-fluoro-phenyl)-N-methyl-carbamate (80.0 g, 333 mmol, compound A1.4) and 2,3,5-trichloropyridine (66.8 g, 366 mmol, CAS: 16063-70-0) in dioxane (2.0 L) were added cesium carbonate (217 g, 666 mmol), palladium(II) acetate (3.74 g, 16.7 mmol), and BINAP (20.7 g, 33.3 mmol, CAS: 98327-87-8). Reaction continued at 120 °C for 16 hrs under nitrogen atmosphere. Then the reaction mixture was cooled to room temperature and diluted with EtOAc (800 mL). The precipitate was removed by filtration. The filtrate was concentrated *in vacuo* and the residue was purified by flash chromatography (silica gel, 0.2% to 5% EtOAc in petroleum ether) to give *tert-butyl* N-[2-[(3,5-dichloro-2-pyridyl)amino]-5-fluoro-phenyl]-N-methyl-carbamate (75 g, 58% yield). MS (ESI): 390.1 ([{³⁷C1}M+H]⁺), 388.1 ([1³⁷Cl+³¹Cl }M+H]⁺), 386.1 ([{³⁵Cl}M+H]⁺).

To the solution of *tert-butyl* N-[2-[(3,5-dichloro-2-pyridyl)amino]-5-fluoro-phenyl]-N-methyl-carbamate (5.0 g, 12.95 mmol) and DBU (3.94 g, 25.9 mmol, CAS: 6674-22-2) in the mixture of o-xylene (7.5mL) and N,N-Dimethylacetamide (7.5 mL) were added palladium(II) acetate (727 mg, 3.24 mmol) and tricyclohexylphosphine tetrafluoroborate (2.38 g, 6.48 mmol) under nitrogen atmosphere. The reaction mixture was stirred at 160 °C for 6 h, then cooled to room temperature and poured into water (100 mL). Then the mixture was extracted with EtOAc (200 mL). The organic layer was collected and washed with water (50 ml) two times and brine (30 ml) two times, dried over sodium sulfate. The organic layer was concentrated *in vacuo* and the residue was purified by flash chromatography (silica gel, 0.5% to 20% EtOAc in dichloromethane) to give *tert-butyl* N-(3-chloro-6-fluoro-9H-pyrido[2,3-b]indol-8-yl)-N-methylcarbamate (873 mg, 19.3% yield) as a yellow solid. MS (ESI): 352.1 ([{³⁷Cl}M+H]⁺), 350.1 ([{³⁵Cl}M+H]⁺).

### Step (e) Preparation of tert-Butyl N-(3,4-dichloro-6-fluoro-9H-pyrido[2,3-b]indol-8-yl)-N-methyl-carbamate (compound A1)

To the solution of *tert-butyl* N-(3-chloro-6-fluoro-9H-pyrido[2,3-b]indol-8-yl)-N-methylcarbamate (4.8 g, 13.7 mmol, compound A1.5) in dichloromethane (200 mL) was added 3-chloroperbenzoic acid (9.47 g, 54.9 mmol) at 0 °C under nitrogen atmosphere. Reaction continued at 30 °C for 12 h. The reaction mixture was poured into sodium sulfite aqueous solution (10%, 150 mL) and stirred for 1 h followed by extraction with ethyl acetate (750 mL) three times. The combined organic layer was washed by sodium bicarbonate aqueous solution (5N, 200 mL) and brine (250 mL), then dried over sodium sulfate and concentrated *in vacuo* to give *tert-butyl* N-(3-chloro-6-fluoro-1-oxido-9H-pyrido[2,3-b]indol-1-ium-8-yl)-N-methylcarbamate (4.8 g, yield: 96% yield) as a brown solid. MS (ESI): 368.1 ([{³⁷Cl}M+H]⁺), 366.1 ([{³⁵Cl}M+H]⁺).

To the solution of *tert-butyl* N-(3-chloro-6-fluoro-1-oxido-9H-pyrido[2,3-b]indol-1-ium-8-yl)-N-methyl-carbamate (4.8 g, 13.1 mmol) in dimethylformamide (100 mL) was added phosphorus(V) oxychloride (22.1 g, 144 mmol) dropwise at -5 °C. The mixture was stirred at - 5 °C to 0 °C for 1 h then poured into sodium bicarbonate aqueous solution (saturated, 350 mL) at 0 °C. The mixture was extracted by EtOAc (500 mL) three times and the combined organic layer was washed with water (200 mL) three times and brine (150 ml) two times, dried over sodium sulfate and concentrated *in vacuo.* The crude product was washed with methanol (120 mL) to give *tert-butyl* N-(3,4-dichloro-6-fluoro-9H-pyrido[2,3-b]indol-8-yl)-N-methyl-carbamate (2.16 g, 42.9% yield) as a pale yellow solid. MS (ESI): 388.1 ([{³⁷Cl+³⁷Cl }M+H]⁺), 386.1 ([{³⁷Cl+³⁵Cl }M+H]⁺), 384.1 ([{³⁵Cl+³⁵Cl }M+H]⁺). ¹H NMR (400 MHz, DMSO-d⁶) δppm: 12.55 (s, 1H), 8.65 (s, 1H), 8.05 (d, J=7.0 Hz, 1H) 7.49 (dd, J=10.3, 2.5 Hz, 1H) 3.26 (s, 3 H) 1.19 - 1.62 (m, 9H).

### Intermediate A2 tert-Butyl N-(3,4-dichloro-5,6-difluoro-9H-pyrido[2,3-b]indol-8-yl)-N-ethyl-carbamate

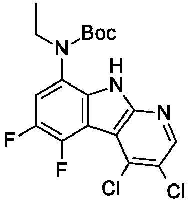

*tert*-Butyl N-(3,4-dichloro-5,6-difluoro-9H-pyrido[2,3-b]indol-8-yl)-N-ethyl-carbamate (intermediate A2) was prepared in analogy to **Intermediate A1,** by replacing methylamine solution with ethylamine solution (30% in ethanol), and 2,4-difluoronitrobenzene with 2,4,5-trifluoronitrobenzene in step (a). MS (ESI): 420.1 ([{³⁷Cl+³⁷Cl }M+H]⁺), 418.1 ([{¹⁷Cl+¹⁵Cl }M+H]⁺), 416.2 ([{³⁵Cl+³⁵Cl }M+H]⁺). ¹H NMR (400 MHz, CDCl₃) δppm: 8.63 (s, 1H), 7.30 (d, J=6.8Hz, 1H), 3.79 (q, J=7.2Hz, 2H), 1.42 (s, 9H), 1.17 (t, J=7.2Hz, 3H).

### Intermediate A3 tert-Butyl N-(3-bromo-4,6-dichloro-9H-pyrido[2,3-b]indol-8-yl)-N-ethyl-carbamate

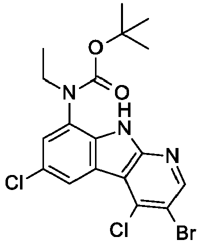

*tert-*Butyl N-(3-bromo-4,6-dichloro-9H-pyrido[2,3-b]indol-8-yl)-N-ethyl-carbamate (intermediate A3) was prepared in analogy to **Intermediate A1,** by replacing methylamine solution with ethylamine solution (30% in ethanol,), and 2,4-difluoronitrobenzene with 4-chloro-2-fluoronitrobenzene in step (a), and replacing 2,3,5-trichloropyridine with 2,3,5-tribromopyridine in step (d). MS (ESI): 463.9 ([{³⁷Cl+³⁷Cl+⁸¹Br}M+H]⁺), 461.9 ([{³⁷Cl+³⁷Cl+⁷⁹Br}/{³⁵Cl+³⁷Cl+⁸¹Br}M+H]⁺), 459.9 ([{³⁵Cl+³⁷Cl+⁷⁹Br}/{³⁵Cl+³⁵Cl+⁸¹Br}M+H]⁺), 457.9 ([P³¹Cl+³¹Cl⁺⁷⁹Br }M+H]⁺).

### Intermediate A4 tert-Butyl N-(3,4-dichloro-6-fluoro-9H-pyrido[2,3-b]indol-8-yl)-N-ethyl-carbamate

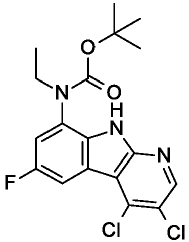

*tert-*Butyl N-(3,4-dichloro-6-fluoro-9H-pyrido[2,3-b]indol-8-yl)-N-ethyl-carbamate (intermediate A4) was prepared in analogy to **Intermediate A1,** by replacing methylamine solution in step (a) with ethylamine solution (30% in ethanol). MS (ESI): 402.0 ([{³⁷Cl+³⁷Cl }M+H]⁺), 400.0 ([{³⁷Cl+³⁵Cl }M+H]⁺), 398.0 ([{³⁵Cl+³⁵Cl }M+H]⁺). ¹HNMR (400 MHz, DMSO-d⁶) δ ppm: 12.57 (s, 1H), 8.65 (s, 1H), 8.08 (d, J=7.2Hz, 1H) 7.42 (dd, J=10.4, 2.4Hz, 1H) 3.67 (br, 2 H) 1.06 - 1.51 (m, 12H).

### Intermediate A5 tert-Butyl N-(3,4-dichloro-5,6-difluoro-9H-pyrido[2,3-b]indol-8-yl)-N-methyl-carbamate

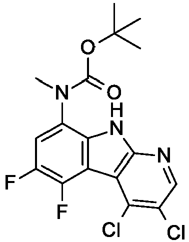

*tert-Butyl N-(3,4-dichloro-5,6-difluoro-9H-pyrido[2,3-b]indol-8-yl)-N-methyl-carbamate* **(Intermediate** A5) was prepared in analogy to **Intermediate A1,** by replacing 2,4-difluoronitrobenzene with 2,4,5-trifluoronitrobenzene in step (a). MS (ESI): 406.1 ([{³⁷Cl+³⁷Cl }M+H]⁺), 404.1 ([{³⁷Cl+³⁵Cl }M+H]⁺), 402.1 ([{³⁵Cl+³⁵Cl }M+H]⁺). ¹H NMR (400 MHz, DMSO-d⁶) δ ppm: 12.84 (br. s, 1H), 8.64 (s, 1H), 7.70 (m, 1H), 3.22 (s, 3H), 1.22-1.50 (m, 9H).

### Intermediate A6 tert-Butyl N-[3,4-dichloro-6-(trifluoromethyl)-9H-pyrido[2,3-b]indol-8-yl]-N-ethylcarbamate

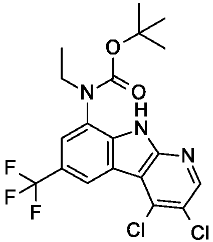

*tert-Butyl N-[3,4-dichloro-6-(trifluoromethyl)-9H-pyrido[2,3-b]indol-8-yl]-N-ethyl*carbamate **(Intermediate A6**) was prepared in analogy to **Intermediate A1,** by replacing methylamine solution with ethylamine solution (30% in ethanol), and 2,4-difluoronitrobenzene with 3-fluoro-4-nitrobenzotrifluoride in step (a). MS (ESI): 452.1 ([{³⁷Cl+³⁷Cl }M+H]⁺), 450.1 ([{³⁷Cl+³⁵Cl }M+H]⁺), 448.1 ([{³⁵Cl+³⁵Cl }M+H]⁺).

### Intermediate A7 tert-butyl (3,4-dichloro-6-fluoro-5-methyl-9H-pyrido[2,3-b]indol-8-yl)(ethyl)carbamate

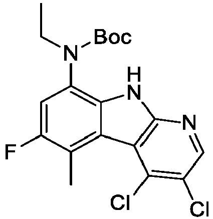

*tert-*butyl (3,4-dichloro-6-fluoro-5-methyl-9H-pyrido[2,3-b]indol-8-yl)(ethyl)carbamate **(Intermediate A7**) was prepared in analogy to **Intermediate A1,** by replacing methylamine solution with ethylamine solution (30% in ethanol), and 2,4-difluoronitrobenzene 1,5-difluoro-2-methyl-4-nitro-benzene in step (a). MS (ESI): 412.1 ([{³⁵Cl}M+H]⁺), 414.1 ([{³⁷Cl}M+H]⁺).

### Intermediate A8 tert-Butyl N-(3,4-dichloro-6,7-difluoro-9H-pyrido[2,3-b]indol-8-yl)-N-methyl-carbamate

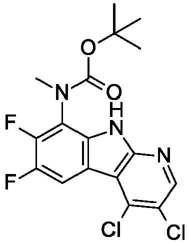

*tert-Butyl* N-(3,4-dichloro-6,7-difluoro-9H-pyrido[2,3-b]indol-8-yl)-N-methyl-carbamate **(Intermediate A8**) was prepared in analogy to **Intermediate A1,** by replacing 2,4-difluoronitrobenzene with 2,5,6-trifluoronitrobenzene in step (a). MS (ESI): 406.1 ([{³⁷Cl+³⁷Cl}M+H]⁺), 404.1 ([{³⁷Cl+³⁵Cl }M+H]⁺), 402.1 ([{³⁵Cl+³⁵Cl }M+H]⁺). ¹H NMR (400 MHz, DMSO-d⁶) δ ppm: 12.84 (br. s, 1H), 8.64 (s, 1H), 7.72 (m, 1H), 3.22 (s, 3H), 1.22-1.50 (m, 9H).

### Intermediate A9 tert-Butyl N-(3-bromo-4-chloro-5,6-difluoro-9H-pyrido[2,3-b]indol-8-yl)-N-methylcarbamate

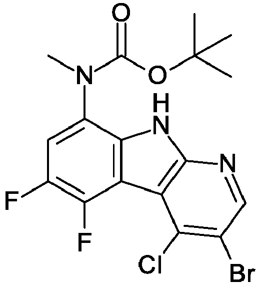

The title compound was synthesized according to the following scheme:

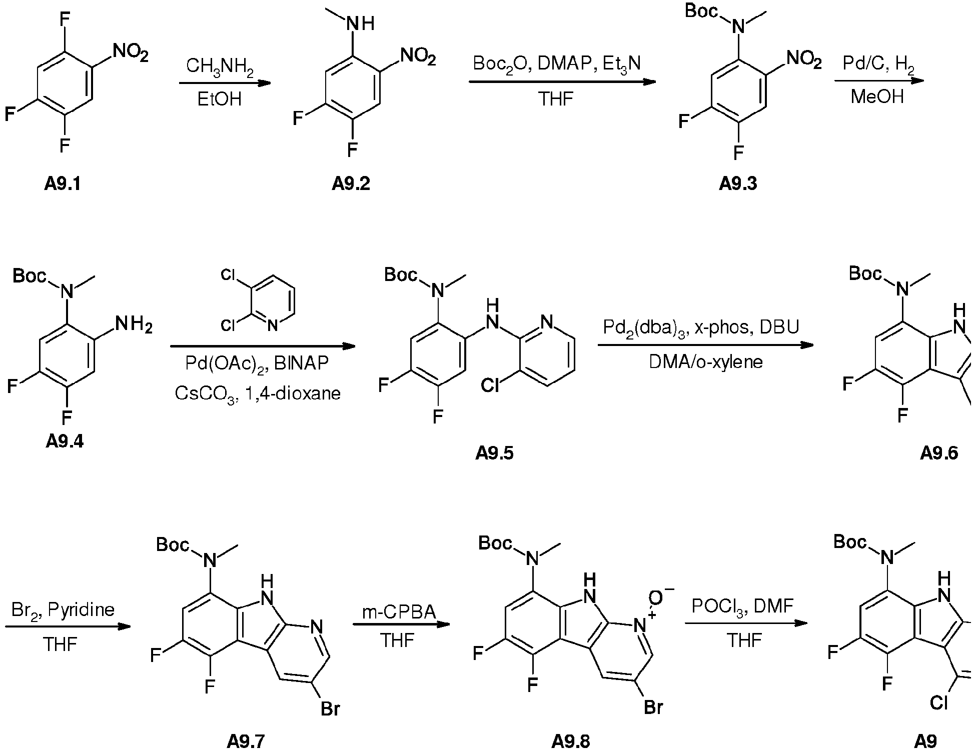

### Step (a) Preparation of 4,5-difluoro-N-methyl-2-nitro-aniline (compound A9.2)

To a solution of 1,2,4-trifluoro-5-nitro-benzene (1.0 kg, 5.65 mol) in EtOH (2.0 L) was added methylamine solution (1.06 kg, 11.29 mmol, 33% in EtOH) dropwise at 0 °C over 3 h. After completion of the addition, the reaction mixture was stirred at 0 °C for additional 1 h before poured into ice-water (15.0 L). The resulting precipitates were collected by filtration, and re-suspended in petroleum ether / EtOAc (10.0 L, v/v=4:1). The by-products were removed by filtration and the filtrate was dried over anhy. Na₂SO₄, filtered, and concentrated *in vacuo* to give a crude product. It was then purified by silica gel flash chromatography (petroleum ether:EtOAc = 100:1-20:1) to give 4,5-difluoro-N-methyl-2-nitro-aniline_(250.0 g, 23.5% yield) as a yellow solid. MS (ESI): 189.1 ([M+H]⁺).

### Step (b) Preparation of tert-butyl N-(4,5-difluoro-2-nitro-phenyl)-N-methyl-carbamate (compound A9.3)

To a mixture solution of 4,5-difluoro-N-methyl-2-nitro-aniline (250.0 g, 1.33 mol), Boc₂O (580.0 g, 2.66 mol), and Et₃N (403.0 g, 3.99 mol) in THF (1.0 L) was added DMAP (32.5 g, 0.266 mmol) and the resulting reaction mixture was stirred at 60 °C for 3 h. After TLC (petroleum ether:EtOAc = 10:1) showed the reaction was completed, MeOH (100.0 mL) was added. The mixture solution was concentrated *in vacuo* to give a crude product, which was purified by silica gel flash chromatography (petroleum ether:EtOAc = 100:1~10:1) to give *tert-*butyl N-(4,5-difluoro-2-nitro-phenyl)-N-methyl-carbamate (305.0 g, 79.6% yield) as a white solid. MS (ESI): 189.1 ([M-Boc+H]⁺), 233.1 ([M-*^{t}*Bu+H]⁺), 311.1 ([M+Na]⁺).

### Step (c) Preparation of tert-butyl N-(2-amino-4,5-difluoro-phenyl)-N-methyl-carbamate (compound A9.4)

To a solution of *tert-butyl* N-(4,5-difluoro-2-nitro-phenyl)-N-methyl-carbamate (104.0 g, 360.8 mmol) in MeOH (1.2 L) was added Pd/C (10.0 g, 50% H₂O) under N₂. After the suspension was degassed under vacuum and purged with H₂ several times, the reaction mixture was stirred at 30 °C for 18 h under H₂ (50 psi). The reaction mixture was filtered through Celite and the filterate was concentrated *in vacuo* to give a crude product of *tert*-butyl N-(2-amino-4,5-difluoro-phenyl)-N-methyl-carbamate (92.0 g, 98.7% yield) as a white solid. MS (ESI):159.1 ([M-Boc+H]⁺), 203.1 ([M-*^{t}*Bu+H]⁺), 281.1 ([M+Na]⁺). It was used directly in the next step without further purification.

### Step (a) Preparation of tert-butyl N-[2-[(3-chloro-2-pyridyl)amino]-4,5-difluoro-phenyl]-N-methyl-carbamate (compound A9.5)

To a stirred solution of *tert-butyl* N-(2-amino-4,5-difluoro-phenyl)-N-methyl-carbamate (142.0 g, 0.55 mol, compound A1.4) and 2,3-dichloropyridine (81.4 g, 0.55 mol) in dioxane (1.5 L) was added cesium carbonate (358.6 g, 1.10 mol). The mixture was degassed with nitrogen for 2 min before palladium(II) acetate (12.3 g, 0.055 mol) and BINAP (68.5 g, 0.110 mmol, CAS: 98327-87-8) were added under nitrogen. The resulting suspension was then heated to 110°C and stirred for 3 h. The reaction mixture was then cooled back to r.t., diluted with EtOAc (1.0 L) and filtered. The filtrate was concentrated *in vacuo* to give a crude product, which was purified by silica gel flash chromatography (petroleum ether:EtOAc = 500:1~20:1) to give *tert*-butyl N-[2-[(3-chloro-2-pyridyl)amino]-4,5-difluoro-phenyl]-N-methyl-carbamate (175.0 g, 86.1% yield) as a white solid. MS (ESI): 370.1 ([{³⁵Cl}M+H]⁺), 372.1 ([{³⁷Cl}M+H]⁺).

### Step (b) Preparation of tert-butyl N-(5,6-difluoro-9H-pyrido[2,3-b]indol-8-yl)-N-methylcarbamate (compound A9.6)

*tert*-Butyl N-[2-[(3-chloro-2-pyridyl)amino]-4,5-difluoro-phenyl]-N-methyl-carbamate (175.0g, 473.24 mmol), Pd₂(dba)₃ (65.0 g, 71.0 mmol, CAS: 51364-51-3), x-phos (67.7 g, 142.0 mmol, CAS: 564483-18-7), and DBU (144 g, 946.5 mmol, CAS: 6674-22-2) were dissolved in a mixture solution of o-xylene / DMA (v/v=1:1, 260.0 mL). After the mixture solution was degassed with nitrogen for 5 min, it was heated at 130°C for 3 h before cooled back to r.t. and diluted with EtOAc (500 mL). The mixture was filtered to remove part of catalyst and ligand, and the filtrate was poured into water (1.0 L) and extracted with EtOAc (2.0 L) three times. Combined organics was then washed with aq. Ca₂Cl₂ solution (1N, 500 mL) four times, brine (500 mL) twice, dried over anhy. Na₂SO₄, filtered, and concentrated *in vacuo* to give a crude product. It was then recrystallized in MeOH (800 mL) to give *tert*-butyl N-(5,6-difluoro-9H-pyrido[2,3-b]indol-8-yl)-N-methyl-carbamate (110 g, 69.7% yield) as a white solid. The mother liquid was purified by silica gel flash chromatography (petroleum ether: EtOAc = 50:1~1:1) to give additional *tert-butyl* N-(5,6-difluoro-9H-pyrido[2,3-b]indol-8-yl)-N-methyl-carbamate (35.0 g, 22.2% yield) as a white solid. In total, 145g of compound A9.6 (91.9% yield) was obtained. MS (ESI): 334.1 ([M+H]⁺).

### Step (c) Preparation of tert-butyl N-(3-bromo-5,6-difluoro-9H-pyrido[2,3-b]indol-8-yl)-N-methyl-carbamate (compound A9.7)

To a stirred solution of *tert-butyl* N-(5,6-difluoro-9H-pyrido[2,3-b]indol-8-yl)-N-methylcarbamate (140.0 g, 0.420 mol) and pyridine (166.1 g, 2.1 mol) in THF (2.0 L) was added bromine (335.6 g, 2.1 mol) at -60 °C. After the addition, the mixture was warmed up and stirred at 15 °C for 2 h. After LC-MS showed the reaction was completed, the mixture was added dropwise into aq. sodium sulfite solution (5.0 L, 10%) and adjusted to pH = 8.0 with aq. sodium carbonate solution at 0 °C and stirred at 25 °C for additional 1 h. The mixture was then extracted by EtOAc (2.0 L) three times. Combined organics was then washed with aq. sodium carbonate solution (1.0 L) twice and brine (1.0 L) three times. Separated organic layer was dried over anhy. Na₂SO₄, filtered, and concentrated *in vacuo* to give a crude product, which was recrystallized in MeOH (1.0 L) to give *tert-butyl* N-(3-bromo-5,6-difluoro-9H-pyrido[2,3-b]indol-8-yl)-N-methyl-carbamate (150.0 g, 86.6% yield) as a yellow solid. MS (ESI): 412.1 ([{⁷⁹Br}M+H]⁺), 414.1 ([{⁸¹Br}M+H]⁺).

### Step (d) Preparation of tert-butyl N-(3-bromo-5,6-difluoro-1-oxido-9H-pyrido[2,3-b]indol-1-ium-8-yl)-N-methyl-carbamate (compound A9.8)

To a stirred solution of *tert-butyl* N-(3-bromo-5,6-difluoro-9H-pyrido[2,3-b]indol-8-yl)-N-methyl-carbamate (150.0 g, 363.9 mmol) in THF (1.2 L) was added 3-chlorobenzoperoxoic acid (148.0 g, 727.8 mmol, 85% wt). The mixture was degassed with nitrogen for 2 min before stirred at 50 °C for 2 h. After TLC (petroleum ether:EtOAc = 2:1) showed the starting material was consumed completely, the reaction was cooled back to r.t. and the mixture was poured into aq. sodium sulfite solution (6.0 L, 10%) and stirred for 1 h. The resulting precipitate was collected by filtration and washed by aq. sodium sulfite solution (1.0 L, 5%). The filter cake was evaporated to dryness under the reduced pressure to give *tert-butyl* N-(3-bromo-5,6-difluoro-1-oxido-9H-pyrido[2,3-b]indol-1-ium-8-yl)-N-methyl-carbamate (150.0 g, 96.3% yield) as a yellow solid. MS (ESI): 428.1 ([{⁷⁹Br}M+H]⁺), 430.1 ([{⁸¹Br}M+H]⁺).

### Step (e) Preparation of tert-Butyl N-(3-bromo-4-chloro-5,6-difluoro-9H-pyrido[2,3-b]indol-8-yl)-N-methyl-carbamate (Intermediate 9)

To a stirred solution of *tert-butyl* N-(3-bromo-5,6-difluoro-1-oxido-9H-pyrido[2,3-b]indol-1-ium-8-yl)-N-methyl-carbamate (150 g, 0.35 mol) in DMF / THF (2.5 L, v/v = 1:1) was added phosphorus(V) oxychloride (421.3 g, 2.75 mol) dropwise under ice-salt bath. After the addition, the mixture was stirred at -5 °C~ 0 °C for 4 h until TLC (petroleum ether: EtOAc = 2:1) showed the starting material was consumed completely. The mixture was then poured into satd. aq. solution of sodium carbonate (5.0 L) at 0 °C and extracted with EtOAc (3.0 L) twice. Combined organics was then washed with satd. aq. solution of Na₂CO₃ (1.0 L) twice, aq. CaCl₂ solution (1N, 1.5 L) four times, and brine (1.0 L) twice. The organic layer was dried over anhy. Na₂SO₄, filtered, and concentrated *in vacuo* to give a crude product, which was recrystallized in MeOH (1.2 L) to give *tert*-Butyl N-(3-bromo-4-chloro-5,6-difluoro-9H-pyrido[2,3-b]indol-8-yl)-N-methyl-carbamate (142.0 g, 90.8% yield) as a white solid. ¹H NMR (400 MHz, DMSO-d6) δ: 12.85 (br. s., 1 H), 8.75 (s, 1 H), 7.69 ~ 7.74 (dd, *J*=11.6, 6.8 Hz, 1 H), 3.22 (s, 3 H), 1.22 ~ 1.50 (m, 9 H). MS (ESI): 446.0 ([⁷⁹Br} M+H]⁺), 448.0 ([{⁸¹Br} M+H]⁺).

### Intermediate B1 [2-(1-Ethoxycarbonylcyclopropyl)pyrimidin-5-yl]boronic acid

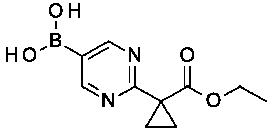

The titled compound was synthesized according to the following scheme:

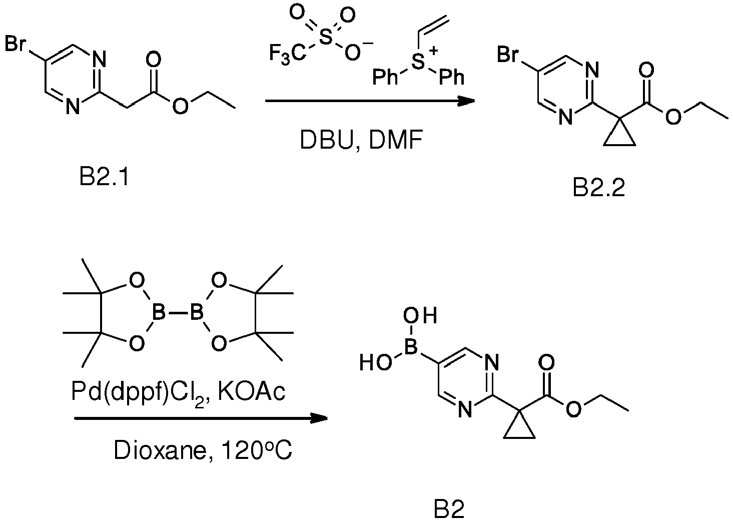

### Step (a) Ethyl 1-(5-bromopyrimidin-2-yl)cyclopropanecarboxylate (compound B2.2)

Ethyl 2-(5-Bromo-2-pyrimidinyl)acetate (compound B2.1, 245 mg, 1.0 mmol, Accela ChemBio, catalog number: SY009334), diphenylvinylsulfonium triflate (724 mg, 1.0 mmol, Synthonix, catalog number: D6369), and DBU (456 mg, 3.0 mmol) were dissolved in DMF (1.5 mL). The solution was stirred under nitrogen atmophere at 100 °C for 1h, cooled to room temperature, and concentrated *in vacuo.* The residue was dissolved in DCM (20mL) and washed with water (10 mL). The organic layer was dried over Na₂SO₄ and concentrated *in vacuo.* The residue was purified by flash chromatography to give ethyl 1-(5-bromopyrimidin-2-yl)cyclopropanecarboxylate (240 mg, 89% yield) as colorless oil. MS (ESI): 273.1 ([{⁸¹Br}M+H]⁺), 271.1 ([{⁷⁹Br}M+H]⁺

### Step (b) [2-(1-Ethoxycarbonylcyclopropyl)pyrimidin-5-yl]boronic acid (compound B2)

Ethyl 1-(5-bromopyrimidin-2-yl)cyclopropanecarboxylate (compound B2.2, 500mg, 1.84mmol), bis(pinacolato)diboron (702 mg, 2.77 mmol), [1,1'-bis(diphenylphosphino)ferrocene]dichloropalladium(II) (135 mg, 0.18 mmol), and potassium acetate (362mg, 3.69 mmol) were dissolved in dioxane (20 mL). The solution was degassed with argon for 5min. Reaction continued at refluxing temperature for 12 h. The reaction mixture was cooled to room temperature. Precipitates were removed by filtration. The filtrate was concentrated *in vacuo.* The residue was purified by reverse phase chromatography to give [2-(1-ethoxycarbonylcyclopropyl)pyrimidin-5-yl]boronic acid as yellow solid (221 mg, 51% yield). MS (ESI): 231.1 ([M+H]⁺).

### Example 1.01 5-[4-[(3R)-3-Aminopyrrolidin-1-yl]-6-fluoro-8-(methylamino)-9H-pyrido[2,3-b]indol-3-yl]pyridine-3-carbonitrile

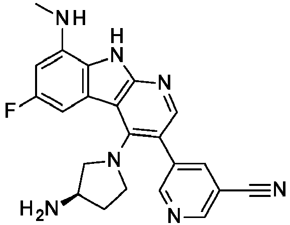

### Step (a) Preparation of (R)-tert-Butyl N-[4-[3-(tert-butoxycarbonylamino)pyrrolidin-1-yl]-3-chloro-6-fluoro-9H-pyrido[2,3-b]indol-8-yl]-N-methyl-carbamate

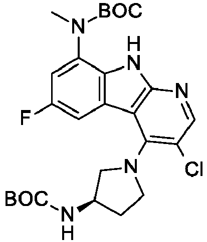

To the solution of *tert-butyl* N-(3,4-dichloro-6-fluoro-9H-pyrido[2,3-b]indol-8-yl)-N-methyl-carbamate (**Intermediate A1,** 100.0 mg, 0.261 mmol, as the "CORE" in table 1) in DMSO (5.0 mL) were added (*R*)-3-(Boc-amino)pyrrolidine (53.0 mg, 0.287 mmol, as the "**AMINE**" in table 1) and triethylamine (53.0 mg, 0.522 mmol). The mixture was stirred at 120 °C for 12 h. The reaction mixture was cooled to room temperature and concentrated *in vacuo.* The residue was purified by prep-HPLC to give (*R*)-*tert*-butyl N-[4-[3-(*tert-*butoxycarbonylamino)pyrrolidin-1-yl]-3-chloro-6-fluoro-9H-pyrido[2,3-b]indol-8-yl]-N-methylcarbamate (110.0 mg, 78% yield) as yellow oil. MS (ESI): 536.2 ([{³⁷Cl}M+H]⁺), 534.2 ([{³⁵Cl}M+H]⁺).

### Step (b) Preparation of (R)-tert-Butyl N-[4-[3-(tert-butoxycarbonylamino)pyrrolidin-1-yl]-3-(5-cyano-3-pyridyl)-6-fluoro-9H-pyrido[2,3-b]indol-8-yl]-N-methyl-carbamate

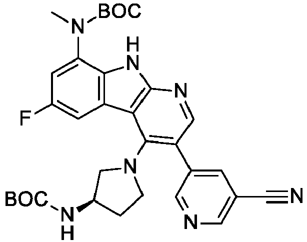

A solution of (*R*)-*tert*-butyl N-[4-[3-(*tert*-butoxycarbonylamino)pyrrolidin-1-yl]-3-chloro-6-fluoro-9H-pyrido[2,3-b]indol-8-yl]-N-methyl-carbamate (100.0 mg, 0.188 mmol), 3-cyanopyridine-5-boronic acid pinacol ester (86.0 mg, 0.375 mmol, as the "**BORONIC REAGENT**" in table 1), tris(dibenzylideneacetone)dipalladium(0) (34.0 mg, 0.038 mmol), XPhos (18.0 mg, 0.038 mmol), and potassium phosphate tribasic (119.0 mg, 0.564 mmol) in dioxane (5.0 mL) and water (3 drops) was stirred at 100 °C for 12 h under nitrogen atmosphere. The mixture was cooled to room temperature and poured into water (50.0 mL). Then the mixture was extracted with EtOAc (100 mL) for three times. The combined organic layer was dried over Na₂SO₄ and concentrated *in vacuo.* The residue was purified by prep-HPLC to give (*R*)*-tert-*butyl N-[4-[3-(*tert-*butoxycarbonylamino)pyrrolidin-1-yl]-3-(5-cyano-3-pyridyl)-6-fluoro-9H-pyrido[2,3-b]indol-8-yl]-N-methyl-carbamate (50.0 mg, 44% yield) as a yellow solid. MS (ESI): 624.3 ([M+Na]⁺), 602.3 ([M+H]⁺).

### Step (c) Preparation of 5-[4-[(3R)-3-aminopyrrolidin-1-yl]-6-fluoro-8-(methylamino)-9H-pyrido[2,3-b]indol-3-yl]pyridine-3-carbonitrile

To the solution of (R)-tert-butyl N-[4-[3-(*tert*-butoxycarbonylamino)pyrrolidin-1-yl]-3-(5-cyano-3-pyridyl)-6-fluoro-9H-pyrido[2,3-b]indol-8-yl]-N-methyl-carbamate (50.0 mg, 0.083 mmol) in dichloromethane (2.0 mL) was added TFA (1.0 mL). The solution was stirred for 2 h at room temperature, and concentrated *in vacuo.* The residue was purified by prep-HPLC to give 5-[4-[(3*R*)-3-aminopyrrolidin-1-yl]-6-fluoro-8-(methylamino)-9H-pyrido[2,3-b]indol-3-yl]pyridine-3-carbonitrile (8.0 mg, 24% yield) as yellow solid. ¹H NMR (Methanol-d⁴, 400MHz) δppm: 8.96 (s, 1H), 8.87 (d, J= 1.6Hz, 1H), 8.29 (s, 1H), 8.09 (s, 1H), 6.89 (d, J=7.6Hz, 1H), 6.49 (d, J=11.6Hz, 1H),3.41-3.60 (m, 4H), 3.01 (s, 3H), 2.80 (m, 1H), 2.32 (m, 1H), 1.79 (m, 1H). MS (ESI): 402.1 ([M+H]⁺).

The following examples were prepared in analogy to **Example 1.01,** replacing *tert-Butyl* N-(3,4-dichloro-5,6-difluoro-9H-pyrido[2,3-b]indol-8-yl)-N-methyl- carbamate (**Intermediate A1**) as the "**CORE**" in step (a), (*R*)-3-(Boc-amino)pyrrolidine as the "**AMINE**" in step (a), THF/H₂O as the "**SOLVENT**" in step (b), Ad₂nBuP Biphenyl as the "**CATALYST**" in step (b) and 3-cyanopyridine-5-boronic acid pinacol ester as the "**BORONIC REAGENT**" in step (b) with the reagents indicated in Table 1 respectively.

**Table 1**

| **No.** | **Compound Name and Structure** | **CORE, AMINE, BORONIC REAGENT, CATALYST and SLOVENT** | **¹H NMR and MS (ESI)** |
|---|---|---|---|
| 1.02 | **5-[4-(3-Aminopyrrolidin-1-yl)-8-(ethylamino)-5,6-difluoro-9H-pyrido[2,3-b]indol-3-yl]pyridine-3-carbonitrile** | **CORE**: Intermediate A2 | **¹H NMR** (400 MHz, DMSO-*d6*) 8 ppm: 12.12 (br. s, 1H), 9.10 (d, J=1.2Hz, 1H), 8.93 (d, J=1.6Hz, 1H), 8.42 (s, 1H), 8.19 (s, 1H), 8.05 (br s, 3H), 6.62 (m, 1H), 3.69 (s, 1H), 3.53 (m, 1H), 3.20-3.32 (m, 4H), 2.98 (m, 1H), 2.24 (m, 1H), 1.85 (m, 1H), 1.30 (t, J=7.2Hz, 3H). |
| | | **AMINE**: 3-(Boc-amino)pyrrolidine | |
| | 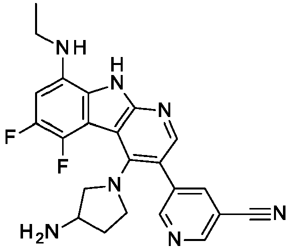 | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | |
| | | **SOLVENT:** THF/H₂O | **MS** (ESI): 434.3 ([M+H]⁺) |
| 1.03 | **5-[4-(3-Aminopyrrolidin-1-yl)-6-chloro-8-(ethylamino)-9H-pyrido[2,3-b]indol-3-yl]pyridine-3-carbonitrile** 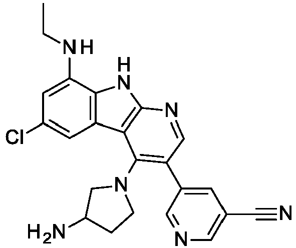 | **CORE:** Intermediate A3 | **¹H NMR** (400 MHz, MeOH-*d4*) 8 ppm: 9.01 (s, 1H), 8.95 (s, 1H), 8.36 (s, 1H), 8.12 (s, 1H), 7.19 (s, 1H), 6.72 (s, 1H), 3.94 (m, 1H), 3.79 (m, 1H) 3.63 (m, 3H), 2.49 (m, 1H), 2.07 (m, 1H), 1.42 (m, 2H), 1.20 (t, J=7.2Hz, 3H). |
| | | **AMINE:** 3-(Boc-amino)pyrrolidine | |
| | | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | |
| | | | **MS** (ESI): 432.3 ([{³⁵Cl}M+H]⁺), 434.3 ([{³⁷Cl}M+H]⁺). |
| | | **SOLVENT:** THF/H₂O | |
| 1.04 | **5-[4-[(3*R*)-3-Aminopyrrolidin-1-yl]-8-(ethylamino)-5,6-difluoro-9H-pyrido[2,3-b]indol-3-yl]pyridine-3-carbonitrile** | **CORE:** Intermediate A2 | **¹H NMR** (400 MHz, |
| | | **AMINE:** (*R*)-3-(Boc-amino)pyrrolidine | MeOH-*d4*) 8 ppm: 8.96 (d, J=2.0Hz, 1H), 8.85 (d, J=2.0Hz, 1H), 8.28 (m, 1H), |
| | 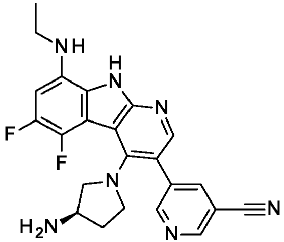 | **BORONIC REAGENT:** 3-Cyanopyridine-5-boronic acid pinacol ester | 8.11 (s, 1H), 6.59 (m, 1H), 3.52 (m, 1H), 3.35 (m, 1H), 3.28-3.33 (m, 4H), 2.76 (m, 1H), 2.25 (m, 1H), 1.73 (m, 1H), 1.41 (t, J=7.2Hz, 3H). |
| | | **CATALYST:** Pd-Ad₂nBuP Biphenyl Precat (CAS#: 1375477-29-4) | |
| | | | **MS** (ESI): 434.3 ([M+H]⁺) |
| | | **SOLVENT:** THF/H₂O | |
| 1.05 | **5-[4-[(3*R*)-3-Aminopyrrolidin-1-yl]-6-chloro-8-(ethylamino)-9H-pyrido[2,3-b]indol-3-yl]pyridine-3-carbonitrile** | **CORE:** Intermediate A3 | **¹H NMR** (400 MHz, MeOH-*d4*) δ ppm: 8.95 (d, J=1.6Hz, 1H), 8.86 (d, J=2.0Hz, 1H), 8.28 (m, 1H), 8.09 (s, 1H), 7.17 (d, J=1.6Hz, 1H), 6.66 (s, 1H), 3.60 (m, 1H), 3.43 (m, 3H), 3.32 (m, 2H), 2.77 (m, 1H), 2.32 (m, 1H), 1.79 (m, 1H), 1.42 (t, J=7.2Hz, 3H). |
| | | **AMINE:** (*R*)-3-(Boc-amino)pyrrolidine | |
| | 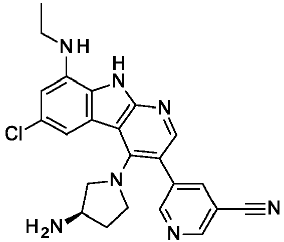 | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | |
| | | **SOLVENT:** THF/H₂O | **MS** (ESI): 432.3 ([{³⁵Cl}M+H]⁺), 434.3 ([{³⁷Cl}M+H]⁺). |
| 1.06 | **5-[4-[2-(Aminomethyl)pyrrolidin-1-yl]-8-(ethylamino)-6-fluoro-9H-pyrido[2,3-b]indol-3-yl]pyridine-3-carbonitrile** | **CORE:** Intermediate A4 | **¹H NMR** (400 MHz, DMSO-*d6*) 8 ppm: 11.75 (s, 1H), 9.10 (s, 1H), 8.90 (d, J=1.6Hz, 1H), 8.37 (s, 1H), 8.31 (s, 1H), 7.53 (br. s, 3H), 6.77 (d, J=8.0Hz, 1H), 6.51 (d, J=12.0Hz, 1H), 3.73 (s, 1H), 3.25-3.37 (m, 4H), 2.51 (m, 1H), 2.36 (m, 1H), 2.04 (m, 2H), 1.87 (m, 2H), 1.32 (t, J=7.2Hz, 3H). |
| | | **AMINE:** 2-N-Boc-Aminomethylpyrrolidine (Matrix Scientific) | |
| | 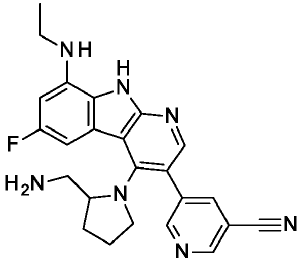 | **BORONIC REAGENT:** 3-Cyanopyridine-5-boronic acid pinacol ester | |
| | | **CATALYST**: Pd-Ad₂nBuP Biphenyl Precat (CAS#: 1375477-29-4) | **MS** (ESI): 430.2 ([M+H]⁺). |
| | | **SOLVENT:** THF/H₂O | |
| 1.07 | **5-[4-[3-(Aminomethyl)pyrrolidin-1-yl]-8-(ethylamino)-5,6-difluoro-9H-pyrido[2,3-b]indol-3-yl]pyridine-3-carbonitrile** | **CORE:** Intermediate A2 | **¹H NMR** (400 MHz, |
| | | **AMINE:** *tert-Butyl* N-(pyrrolidin-3-ylmethyl) carbamate (PharmaBlock) | MeOH-*d4*) δ ppm: 8.98 (s, 1H), 8.97 (s, 1H), 8.41 (s, 1H), 8.10 (s, 1H),6.63 (m, 1H), 3.76 (m, 1H), 3.49 (m, 2H), 3.33 (m, 3H), 2.96 (m, 2H), 2.53 (m, 1H), 2.16 (m, 1H), 1.73 (m, 1H),1.39 (t, J=7.2Hz, 3H). |
| | 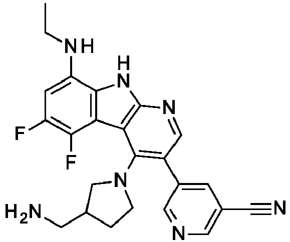 | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | **MS** (ESI): 448.3 ([M+H]⁺) |
| | | **SOLVENT:** THF/H₂O | |
| 1.08 | **4-[3-(Aminomethyl)pyrrolidin-1-yl]-N-ethyl-5,6-difluoro-3-(2-methoxypyrimidin-5-yl)-9H-pyrido[2,3-b]indol-8-amine** | **CORE:** Intermediate A2 | **¹H NMR** (400 MHz, MeOH-*d4*) δ ppm: 8.72 (s, 2H), 8.07 (s, 1H), 6.64 (m, 1H), 4.11 (s, 3H) 3.70 (m, 1H), 3.49-3.54 (m, 2H), 3.27-3.33 (m, 3H), 3.00 (m, 2H), 2.57 (m, 1H), 2.20 (m, 1H), 1.76 (m, 1H), 1.40 (t, J=7.2Hz, 3H). |
| | | **AMINE:** *tert-Butyl* N-(pyrrolidin-3-ylmethyl) carbamate (PharmaBlock) | |
| | 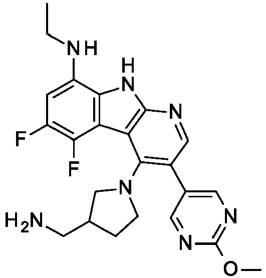 | **BORONIC REAGENT**: 2-Methoxy-5-(4,4,5,5-tetramethyl-1,3,2-dioxaborolan-2-yl)pyrimidine | |
| | | | **MS** (ESI): 454.2 ([M+H]⁺) |
| | | **CATALYST:** Pd-Ad₂nBuP Biphenyl Precat (CAS#: 1375477-29-4) | |
| | | **SOLVENT:** THF/H₂O | |
| 1.09 | **4-(3-Aminopyrrolidin-1-yl)-N-ethyl-5,6-difluoro-3-pyrazolo[1,5-a]pyrimidin-6-yl-9H-pyrido[2,3-b]indol-8-amine** | **CORE:** Intermediate A2 | **¹H NMR** (400 MHz, DMSO-*d6*) 8 ppm: 12.01 (s, 1H), 9.26 (d, J=1.2Hz, 1H), 8.60 (d, J=2.0Hz, 1H), 8.30 (m, 2H), 7.99 (br. s, 3H), 6.83 (d, J=2.0Hz, 1H), 6.63 (m, 1H), 3.63 (m, 1H), 3.60 (m, 1H), 3.38 (m, 2H), 3.24 (m, 2H), 3.00 (m, 1H), 2.27 (m, 1H), 1.86 (m, 1H), 1.31 (t, J=7.2Hz, 3H). |
| | | **AMINE:** 3-(Boc-amino)pyrrolidine | |
| | 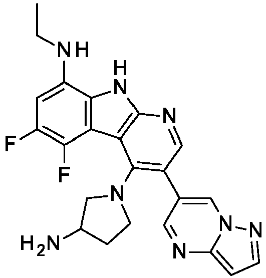 | **BORONIC REAGENT:** Pyrazolo[1,5-a]pyrimidine-6-boronic acid pinacol ester (Matrix Scientific) | |
| | | **CATALYST:** Pd-Ad₂nBuP Biphenyl Precat (CAS#: 1375477-29-4) | |
| | | | **MS** (ESI): 449.3 ([M+H]⁺) |
| | | **SOLVENT:** THF/H₂O | |
| 1.10 | **5-[4-[3-(Aminomethyl)-3-fluoro-pyrrolidin-1-yl]-8-(ethylamino)-6-fluoro-9H-pyrido[2,3-b]indol-3-yl]pyridine-3-carbonitrile** | **CORE:** Intermediate A4 | **¹H NMR** (400 MHz, DMSO-*d6*) δ ppm: 8.83 (s, 1H), 8.75 (s, 1H), 8.16 (s, 1H), 8.03 (s, 1H), 6.88 (m, 1H), 6.40 (m, 1H), 3.50-3.20 (m, 6H), 2.90 (m, 2H), 2.10 (m, 2H), 1.30 (m, 3H). |
| | 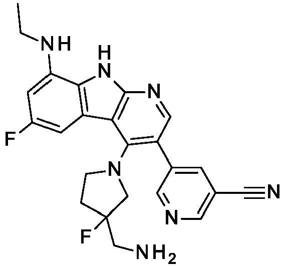 | **AMINE:** *tert*-Butyl N-[(3-fluoropyrrolidin-3-yl) methyl]carbamate (PharmaBlock) | |
| | | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | **MS** (ESI): 448.1 ([M+H]⁺). |
| | | **SOLVENT:** THF/H₂O | |
| 1.11 | **5-[8-(Ethylamino)-6-fluoro-4-[cis-2-amino-5-azaspiro[2.4]heptan-5-yl]-9H-pyrido[2,3-b]indol-3-yl]pyridine-3-carbonitrile** | **CORE:** Intermediate A4 | **¹H NMR** (400 MHz, DMSO-*d6*) 8 ppm: 12.01 (s, 1H), 9.08 (d, J=1.6Hz, 1H), 8.95 (d, J=1.6Hz, 1H), 8.43 (s, 1H), 8.19-8.22 (m, 4H), 6.91 (m, 1H), 6.49 (m, 1H), 3.36-3.44 (m, 3H), 3.24 (m, 3H), 2.73 (s, 1H), 1.93 (m, 1H), 1.85 (m, 1H), 1.32 (t, J=7.2Hz, 3H), 1.09 (m, 1H), 0.93 (m,1H). |
| | | **AMINE:** *Cis-tert*-butyl N-{5-azaspiro[2.4] heptan-1-yl}carbamate | |
| | 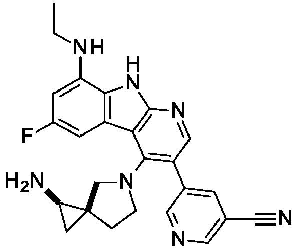 | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | |
| | | | **MS** (ESI): 442.3 ([M+H]⁺). |
| | | **SOLVENT:** THF/H₂O | |
| 1.12 | **5-[8-(Ethylamino)-6-fluoro-4-[*trans*-2-amino-5-azaspiro[2.4]heptan-5-yl]-9H-pyrido[2,3-b]indol-3-yl]pyridine-3-carbonitrile** | **CORE:** Intermediate A4 | **¹H NMR** (400 MHz, DMSO-*d6*) 8 ppm: 11.91 (s, 1H), 9.08 (s, 1H), 8.93 (d, J=2.0Hz, 1H), 8.35-8.44 (m, 4H), 8.17 (s, 1H), 6.87 (d, J=8.4Hz, 1H), 6.49 (d, J=12.0Hz, 1H), 3.42 (m, 2H), 3.26 (m, 3H), 3.06 (m, 1H), 2.68 (s, 1H), 2.07 (m, 1H), 1.95 (m, 1H), 1.32 (t, J=7.2Hz, 3H), 0.99 (m, 2H). |
| | | **AMINE:** *Trans-tert-*butyl N-{5-azaspiro[2.4] heptan-1-yl}carbamate | |
| | | **BORONIC REAGENT:** 3-Cyanopyridine-5-boronic acid pinacol ester | |
| | 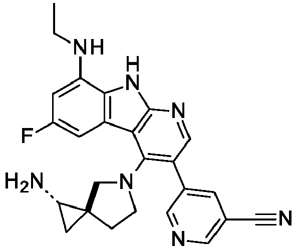 | **CATALYST** : Pd-Ad₂nBuP Biphenyl Precat (CAS#: 1375477-29-4) | |
| | | **SOLVENT:** THF/H₂O | **MS** (ESI): 442.4 ([M+H]⁺). |
| 1.13 | **5-[4-[3-(Aminomethyl)pyrrolidin-1-yl]-5,6-difluoro-8-(methylamino)-9H-pyrido[2,3-b]indol-3-yl]pyridine-3-carbonitrile** | **CORE:** Intermediate A5 | **¹H NMR** (400MHz, CDCl₃) δ ppm: 8.98 (d, J=1.6Hz, 1H), 8.94 (d, J=2.4Hz, 1H), 8.37 (m, 1H) 8.12 (s, 1H), 6.61 (m, 1H), 3.67 (m, 1H), 3.46 (m, 2H), 3.21 (m, 1H), 2.97 (s, 3H), 2.94 (m, 2H), 2.52 (m, 1H), 2.16 (m, 1H), 1.72 (m, 1H). |
| | | **AMINE:** *tert-Butyl* N-(pyrrolidin-3-ylmethyl) carbamate | |
| | 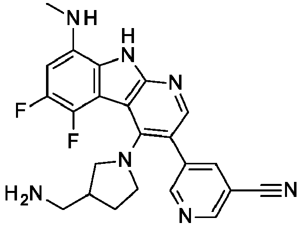 | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | **MS** (ESI): 434.1 ([M+H]⁺). |
| | | **SOLVENT:** THF/H₂O | |
| 1.14 | **5-[4-[3-(Aminomethyl)-1-piperidyl]-8-(ethylamino)-6-fluoro-9H-pyrido[2,3-b]indol-3-yl]pyridine-3-carbonitrile** | **CORE:** Intermediate A4 | **¹H NMR** (400 MHz, DMSO-*d6*) δ ppm: 8.86 (s, 1H), 8.75 (s, 1H), 8.19 (s, 1H), 7.99 (s, 1H), 7.10 (m, 1H), 6.41 (m, 1H), 3.20 (m, 4H), 2.80 (m, 1H), 2.50 (m, 2H), 2.30(m, 1H), 1.90-1.60 (m, 5H), 1.30 (m,3H). |
| | | **AMINE:** 3-(Boc-aminomethyl)piperidine | |
| | | **BORONIC REAGENT:** 3-Cyanopyridine-5-boronic acid pinacol ester | |
| | 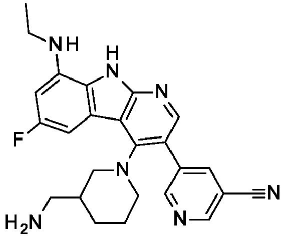 | **CATALYST:** Pd-Ad₂nBuP Biphenyl Precat (CAS#: 1375477-29-4) | **MS** (ESI): 444.2 ([M+H]⁺). |
| | | **SOLVENT:** THF/H₂O | |
| 1.15 | **5-[4-[(3*R*)-3-Aminopyrrolidin-1-yl]-5,6-difluoro-8-(methylamino)-9H-pyrido[2,3-b]indol-3-yl]pyridine-3-carbonitrile** | **CORE:** Intermediate A5 | **¹H NMR** (400 MHz, DMSO-*d6*) 8 ppm: 12.10 (br. s, 1H), 9.10 (s, 1H), 8.93 (s, 1H), 8.43 (s, 1H), 8.19 (s, 1H), 8.08 (br. s, 3H), 6.60 (m, 1H), 3.71 (m, 1H), 3.56 (m, 1H), 3.33 (m, 1H), 3.26 (m, 1H), 3.03 (m, 1H), 2.89 (s, 3H), 2.24 (m, 1H), 1.87 (m, 1H). |
| | | **AMINE:** (*R*)-3-(Boc-amino)pyrrolidine | |
| | 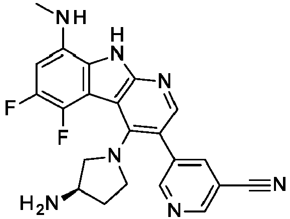 | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | |
| | | **SOLVENT:** THF/H₂O | **MS** (ESI): 420.0 ([M+H]⁺). |
| 1.16 | **5-[6-Fluoro-8-(methylamino)-4-[cis-2-amino-5-azaspiro[2.4]heptan-5-yl]-9H-pyrido[2,3-b]indol-3-yl]pyridine-3-carbonitrile** | **CORE:** Intermediate A1 | **¹H NMR** (400 MHz, |
| | | **AMINE:** *Cis-tert*-butyl N-{5-azaspiro[2.4] heptan-1-yl}carbamate | DMSO-*d6*) 8 ppm: 11.84 (s, 1H), 9.08 (s, 1H), 8.93 (d, J=1.6Hz, 1H), 8.42 (s, 1H), 8.18-8.23 (m, 4H), 6.91 (m, |
| | 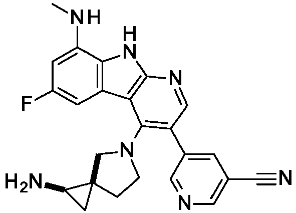 | **BORONIC REAGENT:** 3-Cyanopyridine-5-boronic acid pinacol ester | 1H), 6.47 (d, J=12.0Hz, 1H), 3.35 (m, 3H), 3.22 (m, 1H), 2.92 (s, 3H), 2.74 (s,1H), 1.96 (m, 1H), 1.84 (m,1H), 1.11 (m, 1H), 0.93 (m,1H). |
| | | **CATALYST:** Pd-Ad₂nBuP Biphenyl Precat (CAS#: 1375477- 29-4) | **MS** (ESI): 428.3 ([M+H]⁺) |
| | | **SOLVENT:** THF/H₂O | |
| 1.17 | **5-[6-Fluoro-8-(methylamino)-4-[*trans*-2-amino-5-azaspiro[2.4]heptan-5-yl]-9H-pyrido[2,3-b]indol-3-yl]pyridine-3-carbonitrile** | **CORE:** Intermediate A1 | **¹H NMR** (400 MHz, DMSO-*d6*) 8 ppm: 11.83 (s, 1H), 9.06 (d, J=1.6Hz, 1H), 8.93 (d, J=2.0Hz, 1H), 8.38-8.43 (m, 4H), 8.16 (s, 1H), 6.88 (d, J=8.0Hz, 1H), 6.47 (m, 1H), 3.41 (m, 2H), 3.24 (m, 1H), 3.05 (m, 1H), 2.92 (s, 3H), 2.68 (s, 1H), 2.07(m, 1H), 1.95 (m, 1H), 0.99(m, 2H). |
| | | **AMINE:** *Trans-tert-*butyl N-{5-azaspiro[2.4] heptan-1-yl}carbamate | |
| | 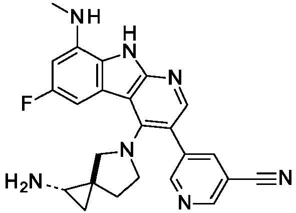 | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | **MS** (ESI): 428.3 ([M+H]⁺) |
| | | **SOLVENT:** THF/H₂O | |
| 1.18 | **4-[(3*R*)-3-Aminopyrrolidin-1-yl]-N-ethyl-5,6-difluoro-3-(2-methoxypyrimidin-5-yl)-9H-pyrido[2,3-b]indol-8-amine** | **CORE:** Intermediate A2 | **¹H NMR** (400 MHz, MeOH-*d4*) 8 ppm: 8.61 (d, J=5.2Hz, 2H), 8.10 (d, J=2.0Hz, 1H), 6.57 (m, 1H), 4.10 (s, 3H), 3.54 (m, 1H), 3.34 (m, 1H), 3.30 (m, 1H), 3.28 (m, 3H), 2.79 (m, 1H), 2.25 (m, 1H), 1.75 (m, 1H), 1.41 (t, J=7.2Hz, 3H). |
| | | **AMINE:** (*R*)-3-(Boc-amino)pyrrolidine | |
| | 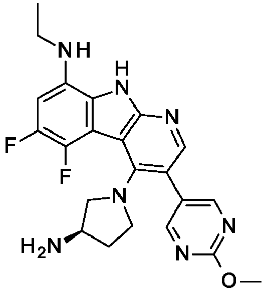 | **BORONIC REAGENT:** 2-Methoxy-5-(4,4,5,5-tetramethyl-1,3,2-dioxaborolan-2-yl)pyrimidine | |
| | | **CATALYST:** Pd-Ad₂nBuP Biphenyl Precat (CAS#: 1375477-29-4) | **MS** (ESI): 440.3 ([M+H]⁺) |
| | | **SOLVENT:** THF/H₂O | |
| 1.19 | **4-[3-(Aminomethyl)pyrrolidin-1-yl]-5,6-difluoro-3-(2-methoxypyrimidin-5-yl)-N-methyl-9H-pyrido[2,3-b]indol-8-amine** | **CORE:** Intermediate A5 | **¹H** NMR (400 MHz, DMSO-*d6*) 8 ppm: 11.91 (s, 1H), 8.66 (s, 2H), 8.17 (s, 1H), 7.79 (s, 3H), 6.59 (m, 1H), 4.00 (s, 3H), 3.22-3.34 (m, 4H), 2.89 (s, 3H), 2.82-2.86 (m, 3H), 2.06 (m, 1H), 1.62 (m, 1H). |
| | | **AMINE:** *tert-Butyl* N-(pyrrolidin-3-ylmethyl) carbamate | |
| | 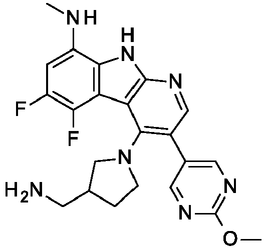 | **BORONIC REAGENT:** 2-Methoxy-5-(4,4,5,5-tetramethyl-1,3,2-dioxaborolan-2-yl)pyrimidine | |
| | | **CATALYST:** Pd-Ad₂nBuP Biphenyl Precat (CAS#: 1375477-29-4) | **MS** (ESI): 440.3 ([M+H]⁺). |
| | | **SOLVENT:** THF/H₂O | |
| 1.20 | **5-[4-(3-Aminopyrrolidin-1-yl)-8-(ethylamino)-6-(trifluoromethyl)-9H-pyrido[2,3-b]indol-3-yl]pyridine-3-carbonitrile** | **CORE:** Intermediate A6 | **¹H NMR** (400 MHz, DMSO-*d6*) δ ppm: 12.25 (d, J=10.4Hz, 1H), 9.12 (s, 1H), 8.96 (d, J=2.0Hz, 1H), 8.46 (d, J=2.0Hz, 1H), 8.20 (s, 1H), 8.11 (s, 3H), 7.37 (s, 1H), 6.79 (s, 1H), 3.78 (m, 1H), 3.46 (m, 3H), 3.30 (m, 2H), 2.95 (m, 1H), 2.32 (m, 1H), 1.94 (m, 1H), 1.34 (t, J=7.2Hz, 3H). |
| | | **AMINE:** 3-(Boc-amino)pyrrolidine | |
| | | **BORONIC REAGENT:** 3-Cyanopyridine-5-boronic acid pinacol ester | |
| | 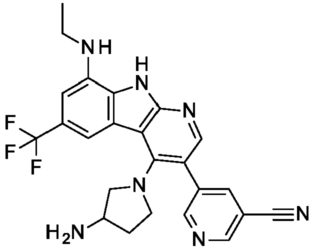 | **CATALYST:** Pd-Ad₂nBuP Biphenyl Precat (CAS#: 1375477-29-4) | |
| | | **SOLVENT:** THF/H₂O | **MS** (ESI): 466.2 ([M+H]⁺). |
| 1.21 | **5-[4-[3-(Aminomethyl)-1-piperidyl]-5,6-difluoro-8-(methylamino)-9H-pyrido[2,3-b]indol-3-yl]pyridine-3-carbonitrile** | **CORE:** Intermediate A5 | **¹H NMR** (400 MHz, MeOH-*d4*) δ ppm: 8.98 (d, 1H), 8.91 (d, 1H), 8.32 (m, 1H), 8.20 (s, 1H), 6.63 (m, 1H), 3.10-3.19 (m, 4H), 2.99 (s, 3H), 2.56 (m, 2H), 1.56-1.87 (m, 5H). |
| | | **AMINE:** 3-(Boc- aminomethyl)piperidine | |
| | 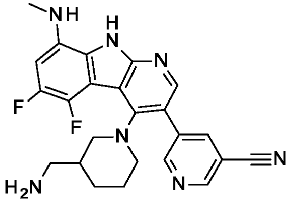 | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | **MS** (ESI): 448.1 ([M+H]⁺). |
| | | **SOLVENT:** THF/H₂O | |
| 1.22 | **5-[4-[3-(Aminomethyl)-3-fluoro-pyrrolidin-1-yl]-5,6-difluoro-8-(methylamino)-9H-pyrido[2,3-b]indol-3-yl]pyridine-3-carbonitrile** | **CORE:** Intermediate A5 | **¹H NMR** (400 MHz, MeOH-*d4*) δ ppm: 8.95 (d, 1H), 8.87 (d, 1H), 8.28 (m, 1H), 8.21 (d, 1H), 6.59 (m, 1H), 3.51-3.69 (m, 2H), 3.18-3.33 (m, 2H), 2.92-3.06 (m, 5H), 2.23-2.28 (m, 2H). |
| | | **AMINE:** *tert*-Butyl N-[(3-fluoropyrrolidin-3-yl) methyl]carbamate (PharmaBlock, Catalog number: PBN20120604) | |
| | 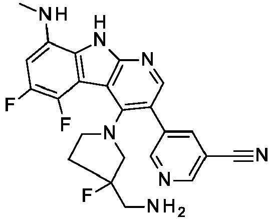 | **BORONIC REAGENT:** 3-Cyanopyridine-5-boronic acid pinacol ester | **MS** (ESI): 452.1 ([M+H]⁺). |
| | | **CATALYST:** Pd-Ad₂nBuP Biphenyl Precat (CAS#: 1375477-29-4) | |
| | | **SOLVENT:** THF/H₂O | |
| 1.23 | **5-[4-[3-(Aminomethyl)-3-fluoro-pyrrolidin-1-yl]-6-fluoro-8-(methylamino)-9H-pyrido[2,3-b]indol-3-yl]pyridine-3-carbonitrile** | **CORE:** Intermediate A1 | **¹H NMR** (400 MHz, MeOH-*d4*) δ ppm: 8.86 (d, 1H), 8.77 (d, 1H), 8.19 (m, 1H), 8.03-8.10 (m, 1H), 6.87 (m, 1H), 6.39 (m, 1H), 3.51-3.60 (m, 1H), 3.42-3.51 (m, 1H), 3.24-3.42 (m, 4H), 2.90 (s, 3H), 2.07-2.37 (m, 2H). |
| | 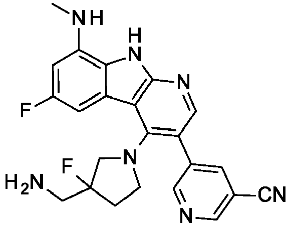 | **AMINE:** *tert*-Butyl N-[(3-fluoropyrrolidin-3-yl) methyl]carbamate (PharmaBlock, Catalog number: PBN20120604) | |
| | | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | **MS** (ESI): 434.3 ([M+H]⁺) |
| | | **SOLVENT:** THF/H₂O | |
| 1.24 | **4-[3-(Aminomethyl)-3-fluoro-pyrrolidin-1-yl]-6-fluoro-3-(2-methoxypyrimidin-5-yl)-N-methyl-9H-pyrido[2,3-b]indol-8-amine** | **CORE:** Intermediate A1 | **¹H NMR** (400 MHz, MeOH-*d4*) δ ppm: 8.64 (s, 2H), 8.13 (s, 1H), 7.04 (d, J=8Hz, 1H), 6.49 (m, 1H), 4.11 (s, 3H), 3.40-3.65 (m, 4H), 3.02 (m, 5H), 2.10-2.33 (m, 2H). |
| | | **AMINE:** *tert*-Butyl N-[(3-fluoropyrrolidin-3-yl) methyl]carbamate (PharmaBlock, Catalog number: PBN20120604) | |
| | 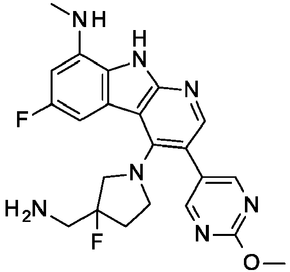 | **BORONIC REAGENT:** 2-Methoxy-5-(4,4,5,5-tetramethyl-1,3,2-dioxaborolan-2-yl)pyrimidine | **MS** (ESI): 440.3 ([M+H]⁺) |
| | | **CATALYST:** Pd-Ad₂nBuP Biphenyl Precat (CAS#: 1375477-29-4) | |
| | | **SOLVENT:** THF/H₂O | |
| 1.25 | **4-[(3*S*)-3-(Aminomethyl)-1-piperidyl]-5,6-difluoro-3-(2-methoxypyrimidin-5-yl)-N-methyl-9H-pyrido[2,3-b]indol-8-amine** | **CORE:** Intermediate A5 | **¹H NMR** (400 MHz, MeOH-*d4*) δ ppm: 8.62 (s, 2H), 8.56 (s, 1H), 8.20 (s, 1H), 6.63 (m, 1H), 4.08 (s, 3H), 3.10-3.26 (m, 3H), 2.99 (s, 3H), 2.70-2.84 (m,3H), 1.63-2.05 (m, 4H),1.16 (m, 1H). |
| | | **AMINE:** *tert*-butyl N-[(3*R*)-piperidin-3- ylmethyl] carbamate (PharmaBlock, Catalog number: PB00907) | |
| | 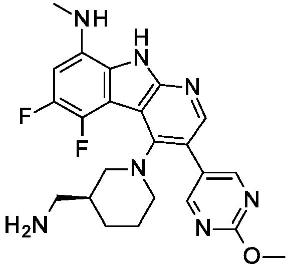 | **BORONIC REAGENT:** 2-Methoxy-5-(4,4,5,5-tetramethyl-1,3,2-dioxaborolan-2-yl)pyrimidine | **MS** (ESI): 454.2 ([M+H]⁺) |
| | | **CATALYST:** Pd-Ad₂nBuP Biphenyl Precat (CAS#: 1375477-29-4) | |
| | | **SOLVENT:** THF/H₂O | |
| 1.26 | **4-[3-(Aminomethyl)-3-methyl-1-piperidyl]-5,6-difluoro-3-(2-methoxypyrimidin-5-yl)-N-methyl-9H-pyrido[2,3-b]indol-8-amine** | **CORE:** Intermediate A5 | **¹H NMR** (400 MHz, MeOH-*d4*) δ ppm: 8.62 (s, 2H), 8.60 (s, 1H), 8.19 (s, 1H), 6.64 (m, 1H), 4.08 (s, 3H), 3.60 (m, 1H), 3.10 (m, 2H), 3.00 (s, 3H), 2.80 (m, 1H), 2.40 (m, 1H), 2.0 (m,1H), 1.58 (m, 2H), 1.36 (m,2H), 0.85 (s, 3H). |
| | | **AMINE:** *tert*-Butyl N-[(3-methylpiperidin-3-yl)methyl] carbamate (PharmaBlock, Catalog number: PBN2011460) | |
| | 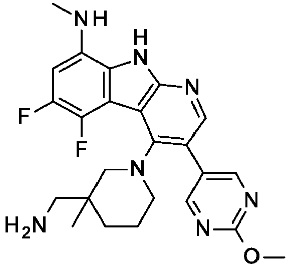 | **BORONIC REAGENT:** 2-Methoxy-5-(4,4,5,5-tetramethyl-1,3,2-dioxaborolan-2-yl)pyrimidine | **MS** (ESI): 468.3 ([M+H]⁺) |
| | | **CATALYST:** Pd-Ad₂nBuP Biphenyl Precat (CAS#: 1375477-29-4) **SOLVENT:** THF/H₂O | |
| 1.27 | **4-[1-(Aminomethyl)-3-azabicyclo[3.1.0]hexan-3-yl]-5,6-difluoro-3-(2-methoxypyrimidin-5-yl)-N-methyl-9H-pyrido[2,3-b]indol-8-amine** | **CORE:** Intermediate A5 | **¹H NMR** (400 MHz, MeOH-*d4*) δ ppm: 8.57 (s, 2H), 8.37 (s, 1H), 6.66 (m, 1H), 4.10 (s, 3H), 3.80 (m, 1H), 3.70 (m, 1H), 3.40 (m, 1H), 3.20 (m, 1H), 3.10 (m, 1H), 3.00 (s, 3H), 1.75 (m,1H), 0.85 (m, 1H), 0.70 (m,1H). |
| | | **AMINE:** 1-(Boc-amino)-3-azabicyclo[3.1.0] hexane (PharmaBlock, Catalog number: PB05399) | |
| | 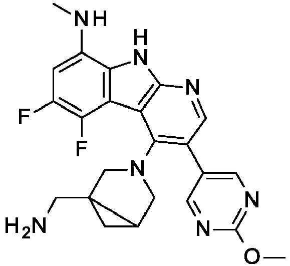 | **BORONIC REAGENT:** 2-Methoxy-5-(4,4,5,5-tetramethyl-1,3,2-dioxaborolan-2-yl)pyrimidine | **MS** (ESI): 452.1 ([M+H]⁺) |
| | | **CATALYST:** Pd-Ad₂nBuP Biphenyl Precat (CAS#: 1375477-29-4) | |
| | | SOLVENT: THF/H₂O | |
| 1.28 | **4-[3-(Aminomethyl)-3-fluoro-pyrrolidin-1-yl]-5,6-difluoro-3-(2-methoxypyrimidin-5-yl)-N-methyl-9H-pyrido[2,3-b]indol-8-amine** | **CORE:** Intermediate A5 | **¹H NMR** (400 MHz, MeOH-*d4*) δ ppm: 8.49 (s, 2H), 8.04 (s, 1H), 6.46 (m, 1H), 3.98 (s, 3H), 3.10-3.58 (m, 4H), 2.90 (m, 2H), 2.85 (s, 3H), 2.10 (m, 2H). |
| | | **AMINE:** *tert*-butyl N-[(3-fluoropyrrolidin-3-yl) methyl] carbamate (PharmaBlock, Catalog number: PBN20120604) | |
| | 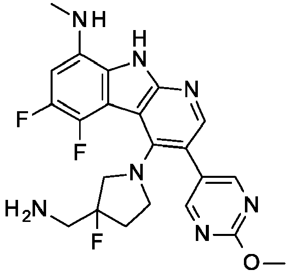 | **BORONIC REAGENT:** 2-Methoxy-5-(4,4,5,5-tetramethyl-1,3,2-dioxaborolan-2-yl)pyrimidine | **MS** (ESI): 448.1 ([M+H]⁺) |
| | | **CATALYST:** Pd-Ad₂nBuP Biphenyl Precat (CAS#: 1375477-29-4) | |
| | | **SOLVENT:** THF/H₂O | |
| 1.29 | **5-[4-[Trans-2-amino-5-azaspiro[2.4]heptan-5-yl]-5,6-difluoro-8-(methylamino)-9H-pyrido[2,3-b]indol-3-yl]pyridine-3-carbonitrile** | **CORE:** Intermediate A5 | **¹H NMR** (400 MHz, DMSO-*d6*) δ ppm: 9.06 (s, 1H), 8.89 (d, J=2Hz, 1H), 8.40 (d, J=1.6Hz, 1H), 8.33 (s, 1H), 8.18 (s, 1H), 6.57 (m, 1H), 5.79 (s, 1H), 3.28 (m, 1H), 2.92 (m, 5H), 2.17 (m, 1H), 2.11 (m, 1H), 1.80(m, 1H), 0.63 (m, 1H), 0.47(m, 1H). |
| | | **AMINE:** *Trans-tert-*butyl N-{5-azaspiro[2.4] heptan-1-yl}carbamate | |
| | | **BORONIC REAGENT:** 3-Cyanopyridine-5-boronic acid pinacol ester | |
| | 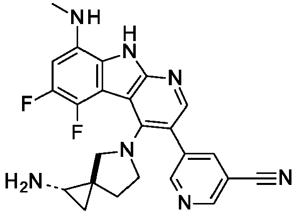 | **CATALYST:** Pd-Ad₂nBuP Biphenyl Precat (CAS#: 1375477-29-4) | **MS** (ESI): 446.2 ([M+H]⁺) |
| | | **SOLVENT:** THF/H₂O | |
| 1.30 | **5-[4-[*Cis*-2-amino-5-azaspiro[2.4]heptan-5-yl]-5,6-difluoro-8-(methylamino)-9H-pyrido[2,3-b]indol-3-yl]pyridine-3-carbonitrile** | **CORE:** Intermediate A5 | **¹H NMR** (400 MHz, DMSO-*d6*) δ ppm: 9.06 (s, 1H), 8.90 (s, 1H), 8.41 (s, 1H), 8.30 (s, 1H), 8.17 (s, 1H), 6.57 (m, 1H), 5.77 (s, 1H), 3.28 (m, 1H), 3.24 (m, 2H), 3.03 (m, 1H), 2.89 (s, 3H), 2.34 (m, 1H), 1.81 (m, 1H), 1.70 (m, 1H), 0.78 (m,1H), 0.33 (m, 1H). |
| | | **AMINE:** *Cis-tert*-butyl N-{5-azaspiro[2.4] heptan-1-yl}carbamate | |
| | 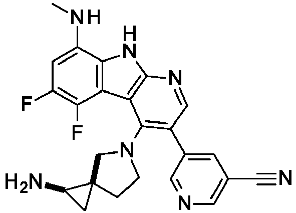 | **BORONIC REAGENT:** 3-Cyanopyridine-5-boronic acid pinacol ester | |
| | | **CATALYST:** Pd-Ad₂nBuP Biphenyl Precat (CAS#: 1375477- 29-4) | **MS** (ESI): 446.2 ([M+H]⁺) |
| | | **SOLVENT:** THF/H₂O | |
| 1.31 | **5-[4-[2-(Aminomethyl)morpholin-4-yl]-5,6-difluoro-8-(methylamino)-9H-pyrido[2,3-b]indol-3-yl]pyridine-3-carbonitrile** | **CORE:** Intermediate A5 | **¹H NMR** (400 MHz, DMSO-*d6*) δ ppm: 11.99 (s, 1H), 9.12 (d, J=2.0Hz, 1H), 8.92 (s, 1H), 8.43 (d, J=2.0Hz, 1H), 8.22 (s, 1H), 7.90 (m, 3H), 6.62 (m, 1H), 3.83 (m, 2H), 3.60 (m, 1H), 3.10(m,1H),2.93(m,6H),2.68(m,2H). |
| | | **AMINE:** *tert*-Butyl (morpholin-2-ylmethyl)carbamate (Wuxi Apptec, Catalog number: WX604306) | |
| | 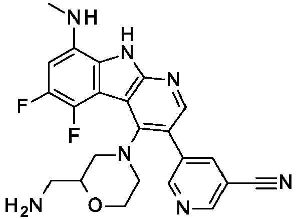 | **BORONIC REAGENT:** 3-Cyanopyridine-5-boronic acid pinacol ester | **MS** (ESI): 450.2 ([M+H]⁺) |
| | | **CATALYST:** Pd-Ad₂nBuP Biphenyl Precat (CAS#: 1375477-29-4) | |
| | | **SOLVENT:** THF/H₂O | |
| 1.32 | **5-[4-[1-(Aminomethyl)-3-azabicyclo[3.1.0]hexan-3-yl]-5,6-difluoro-8-(methylamino)-9H-pyrido[2,3-b]indol-3-yl]pyridine-3-carbonitrile** | **CORE:** Intermediate A5 | **¹H NMR** (400 MHz, *MeOH-d4)* δ ppm: 8.96 (s, 1H), 8.82 (s, 1H), 8.60 (s, 1H), 8.39 (s, 1H), 8.25 (s, 1H), 6.68 (m, 1H), 3.80 (m, 1H), 3.70 (m, 1H), 3.40 (m, 1H), 3.20 (m, 1H), 3.10 (m, 1H), 3.00 (s, 3H), 2.99 (m, 1H), 1.75 (m, 1H), 0.85 (m, 1H), 0.60 (m, 1H). |
| | | **AMINE:** 1-(Boc-amino)-3-azabicyclo[3.1.0] hexane (PharmaBlock, Catalog number: PB05399) | |
| | 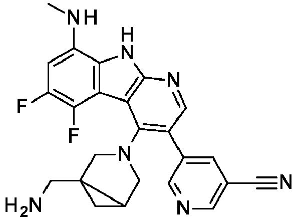 | **BORONIC REAGENT:** 3-Cyanopyridine-5-boronic acid pinacol ester | |
| | | **CATALYST:** Pd-Ad₂nBuP Biphenyl Precat (CAS#: 1375477- 29-4) | **MS** (ESI): 446.1 ([M+H]⁺) |
| | | **SOLVENT:** THF/H₂O | |
| 1.33 | **4-[2-(Aminomethyl)morpholin-4-yl]-5,6-difluoro-3-(2-methoxypyrimidin-5-yl)-N-methyl-9H-pyrido[2,3-b]indol-8-amine** | **CORE:** Intermediate A5 | **¹H NMR** (400 MHz, DMSO-*d6*) δ ppm: 11.94 (s, 1H), 8.67 (s, 2H), 8.21 (s, 1H), 7.89 (s, 3H), 8.22 (s, 1H), 6.62 (m, 1H), 4.02 (s, 3H), 3.65 (m, 3H), 3.13 (m, 1H), 2.91 (m, 6H), 2.71 (m,2H). |
| | | **AMINE:** *tert*-Butyl (morpholin-2-ylmethyl)carbamate (Wuxi Apptec, Catalog number: WX604306) | |
| | 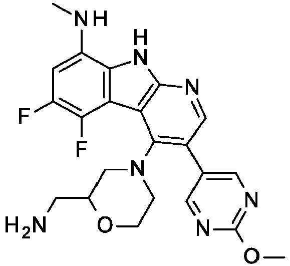 | **BORONIC REAGENT:** 2-Methoxy-5-(4,4,5,5-tetramethyl-1,3,2-dioxaborolan-2-yl)pyrimidine | **MS** (ESI): 456.2 ([M+H]⁺) |
| | | CATALYST: Pd-Ad₂nBuP Biphenyl Precat (CAS#: 1375477-29-4) | |
| | | **SOLVENT:** THF/H₂O | |
| 1.34 | **4-[*Cis*-2-amino-5-azaspiro[2.4]heptan-5-yl]-5,6-difluoro-3-(2-methoxypyrimidin-5-yl)-N-methyl-9H-pyrido[2,3-b]indol-8-amine** | **CORE:** Intermediate A5 | **¹H NMR** (400 MHz, MeOH-*d4*) δ ppm: 8.60 (s, 2H), 8.11 (s, 1H), 6.56 (m, 1H), 4.11 (s, 3H), 3.39 (m, 2H), 3.05 (m, 1H), 3.00 (m, 1H), 2.97 (s, 3H), 2.18 (m, 2H), 1.91 (m, 1H), 0.73 (m, 1H), 0.48 (m, 1H). |
| | | **AMINE:** *Cis-tert*-butyl N-{5-azaspiro[2.4] heptan-1-yl}carbamate | |
| | | **BORONIC REAGENT:** 2-Methoxy-5-(4,4,5,5-tetramethyl-1,3,2-dioxaborolan-2-yl)pyrimidine | |
| | 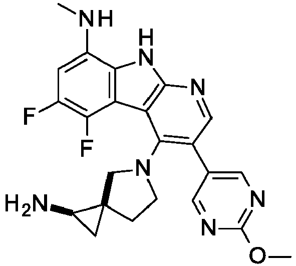 | **CATALYST:** Pd-Ad₂nBuP Biphenyl Precat (CAS#: 1375477-29-4) | **MS** (ESI): 452.3 ([M+H]⁺) |
| | | **SOLVENT:** THF/H₂O | |
| 1.35 | **4-[Trans-2-amino-5-** | **CORE:** Intermediate A5 | **¹H NMR** (400 MHz, MeOH-*d4*) δ ppm: 8.63 (s, 2H), 8.13 (s, 1H), 6.57 (m, 1H), 4.11 (s, 3H), 3.44 (m, 1H), 3.35 (m, 1H), 3.32 (m,1H), 3.20 (m, 1H), 2.98 (s,3H), 2.36 (m, 1H), 1.87 (m,2H), 0.85 (m, 1H), 0.42 (m,1H). |
| | **azaspiro[2.4]heptan-5-yl]-5,6-difluoro-3-(2-methoxypyrimidin-5-yl)-N-methyl-9H-pyrido[2,3-b]indol-8-amine** | **AMINE:** *Trans-tert-*butyl N-{5-azaspiro[2.4] heptan-1-yl}carbamate | |
| | 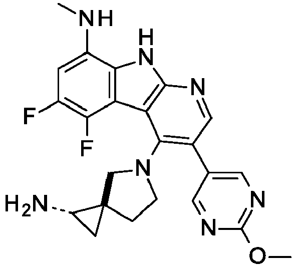 | **BORONIC REAGENT:** 2-Methoxy-5-(4,4,5,5-tetramethyl-1,3,2-dioxaborolan-2-yl)pyrimidine | **MS** (ESI): 452.3 ([M+H]⁺) |
| | | **CATALYST:** Pd-Ad₂nBuP Biphenyl Precat (CAS#: 1375477-29-4) | |
| | | **SOLVENT:** THF/H₂O | |
| 1.36 | **4-[3-(Aminomethyl)-3-methyl-pyrrolidin-1-yl]-5,6-difluoro-3-(2-methoxypyrimidin-5-yl)-N-methyl-9H-pyrido[2,3-b]indol-8-amine** | **CORE:** Intermediate A5 | **¹H NMR** (400 MHz, MeOH-*d4*) δ ppm: 8.54 (s, 2H), 8.04 (s, 1H), 6.51 (m, 1H), 3.99 (s, 3H), 3.48 (m, 1H), 3.13-3.28 (m, 3H), 2.89 (m, 5H), 1.90 (m, 1H), 1.79 (m, 1H), 1.02 (s, 3H). |
| | | **AMINE:** *tert*-Butyl (3-methylpyrrolidin-3-yl)methylcarbamate (PharmaBlock, Catalog number: PBN20120902) | |
| | 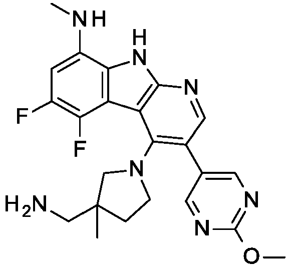 | **BORONIC REAGENT:** 2-Methoxy-5-(4,4,5,5-tetramethyl-1,3,2-dioxaborolan-2-yl)pyrimidine | **MS** (ESI): 454.2 ([M+H]⁺) |
| | | **CATALYST:** Pd-Ad₂nBuP Biphenyl Precat (CAS#: 1375477-29-4) | |
| | | **SOLVENT:** THF/H₂O | |
| 1.37 | **4-[2-(Aminomethyl)pyrrolidin-1-yl]-5,6-difluoro-3-(2-methoxypyrimidin-5-yl)-N-methyl-9H-pyrido[2,3-b]indol-8-amine** | **CORE:** Intermediate A5 | **¹H NMR:** (400 MHz, MeOH-*d4*) δ ppm: 8.67 (s, 2H), 8.28 (s, 1H), 6.66 (m, 1H), 4.12 (s, 3H), 3.83 (m, 1H), 3.65 (m, 1H), 3.34 (m, 1H), 3.00 (s, 3H), 2.74 (m,2H), 1.88-2.11 (m, 4H). |
| | | **AMINE:** 2-N-Boc-Aminomethylpyrrolidine (Matrix Scientific, Catalog number: 073333) | |
| | 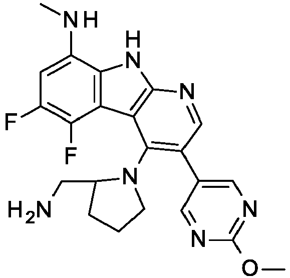 | **BORONIC REAGENT:** 2-Methoxy-5-(4,4,5,5-tetramethyl-1,3,2-dioxaborolan-2-yl)pyrimidine | **MS** (ESI): 440.3 **([M+H]⁺)** |
| | | **CATALYST:** Pd-Ad₂nBuP Biphenyl Precat (CAS#: 1375477-29-4) | |
| | | **SOLVENT:** THF/H₂O | |
| 1.38 | **5-[4-[2-(Aminomethyl)pyrrolidin-1-yl]-5,6-difluoro-8-(methylamino)-9H-pyrido[2,3-b]indol-3-yl]pyridine-3-carbonitrile** | **CORE:** Intermediate A5 | **¹H NMR:** (400 MHz, MeOH-*d4*) δ ppm: 9.023 (d, 1H), 8.90 (d, 1H), 8.32 (m, 2H), 6.68 (m, 1H), 3.82 (m, 1H), 3.53 (m, 1H), 3.33 (m, 1H), 3.01 (s, 3H), 2.76 (m, 2H), 1.86-2.11 (m, 4H). |
| | | **AMINE:** 2-N-Boc-Aminomethylpyrrolidine (Matrix Scientific, Catalog number: 073333) | |
| | 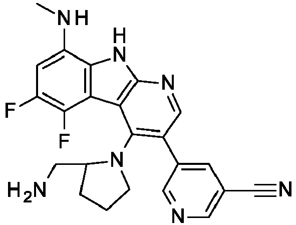 | **BORONIC REAGENT:** 3-Cyanopyridine-5-boronic acid pinacol ester | **MS** (ESI): 434.3 ([M+H]⁺) |
| | | **CATALYST:** Pd-Ad₂nBuP Biphenyl Precat (CAS#: 1375477-29-4) | |
| | | **SOLVENT:** THF/H₂O | |
| 1.40 | **5-[4-[(3S)-3-(Aminomethyl)-1-piperidyl]-5,6-difluoro-8-(methylamino)-9H-pyrido[2,3-b]indol-3-yl]pyridine-3-carbonitrile** | **CORE:** Intermediate A5 | ¹H NMR (400 MHz, MeOH-*d4*) δ ppm: 9.00 (s, 1H), 8.91 (s, 1H), 8.32 (s, 1H), 8.23 (s, 1H), 6.63 (m, 1H), 3.06-3.24 (m, 4H), 3.00 (s, 3H), 2.82 (m, 2H),1.59-1.98 (m, 4H), 1.14 (m,1H). |
| | | **AMINE:** (*R*)-3-(Boc-aminomethyl)-piperidine (Matrix Scientific, Catalog number: 112216) | |
| | 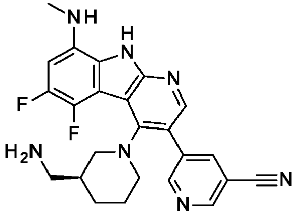 | **BORONIC REAGENT:** 3-Cyanopyridine-5-boronic acid pinacol ester | **MS** (ESI): 448.1 ([M+H]⁺) |
| | | **CATALYST:** Pd-Ad₂nBuP Biphenyl Precat (CAS#: 1375477-29-4) | |
| | | **SOLVENT:** THF/H₂O | |
| 1.41 | **Methyl 5-[4-[(3*S*)-3-(aminomethyl)-1-piperidyl]-5,6-difluoro-8-(methylamino)-9H-pyrido[2,3-b]indol-3-yl]pyridine-3-carboxylate** | **CORE**: Intermediate A5 | **¹H NMR** (400 MHz, MeOH-*d4*) δ ppm: 9.20 (s, 1H), 8.85 (s, 1H), 8.48 (s, 1H), 8.23 (s, 1H), 6.64 (m, 1H), 4.02 (s, 3H), 3.16 (m, 4H), 3.00 (s, 3H), 2.81 (m, 2H), 1.61-1.99 (m, 4H), 1.14 (m, 1H). |
| | | **AMINE:** (*R*)-3-(Boc-aminomethyl)-piperidine (Matrix Scientific, Catalog number: 112216) | |
| | 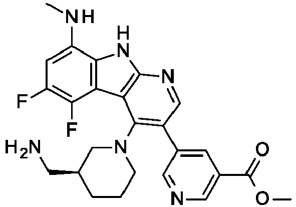 | **BORONIC REAGENT:** 5-(Methoxycarbonyl)pyridi ne-3-boronic acid | |
| | | | **MS** (ESI): 481.1 ([M+H]⁺) |
| | | **CATALYST:** Pd-Ad₂nBuP Biphenyl Precat (CAS#: 1375477-29-4) | |
| | | **SOLVENT:** THF/H₂O | |
| 1.44 | **5-(4-(3,6-Diazabicyclo[3.1.1]heptan-3-yl)-5,6-difluoro-8-(methylamino)-9H-pyrido[2,3-b]indol-3-yl)nicotinonitrile** | **CORE:** Intermediate A5 | **¹H NMR** (400 MHz, DMSO-d6) δ: 9.01-9.02 (d, *J*=2.0 Hz, 1 H), 8.90 (d, *J*=2.0 Hz, 1 H), 8.36~8.37 (t, *J*=2.0 Hz, 1 H), 8.31 (s, 1 H), 6.58~6.64 (dd, *J*=13.6, 6.4 Hz, 1 H), 5.60~5.62 (br d, *J*=4.8 Hz, 1 H), 3.53~3.57 (br d, *J*=10.8 Hz, 2 H), 3.49-3.51 (br d, *J*=6.0 Hz, 2 H), 2.93-2.94 (d, *J*=4.8 Hz, 3 H), 2.52-2.53 (m, 3 H), 2.35-2.41 (m, 1 H), 1.33-1.36 (d, *J*=8.4 Hz, 1 H). |
| | | **AMINE:** *tert-*Butyl 3,6-diazabicyclo[3.1.1]hepta ne-6-carboxylate-b]pyrrole | |
| | 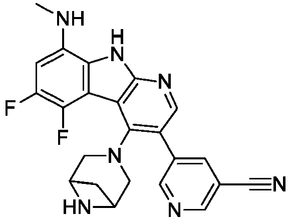 | **BORONIC REAGENT:** 5-(4,4,5,5-Tetramethyl-1,3,2-dioxaborolan-2-yl)nicotinonitrile | |
| | | **CATALYST:** Pd-Ad₂nBuP Biphenyl Precat (CAS#: 1375477-29-4) | |
| | | **SOLVENT:** THF/H₂O | MS (ESI): 432.1 (M+H)⁺. |
| 1.45 | **5-(4-(3-(Aminomethyl)pyrrolidin-1-yl)-8-(ethylamino)-6-fluoro-5-methyl-9H-pyrido[2,3-b]indol-3-yl)nicotinonitrile** | **CORE:** Intermediate A7 | **¹H NMR** (400 MHz, MeOH-*d4*): δ 9.03 (s, 1 H), 9.00 (s, 1 H), 8.47 (s, 1 H), 8.05 (s, 1 H), 6.59~6.62 (d, *J*=12.4 Hz, 1 H), 3.47~4.07 (m, 3 H), 3.29-3.32 (m, 2 H), 2.92-3.15 (m, 2 H), 2.67 (s, 3H), 1.52-2.48 (m, 2 H), 1.38-1.41 (d, *J*=7.2 Hz, 3 H). |
| | | **AMINE:** *tert*-Butyl (pyrrolidin-3-ylmethyl)carbamate | |
| | 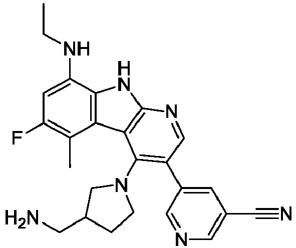 | **BROMIDE REAGENT:** 5-(4,4,5,5-Tetramethyl-1,3,2-dioxaborolan-2-yl)nicotinonitrile | |
| | | **CATALYST:** Pd₂(dba)₃, X-phos | **MS** (ESI): 444.2[M+H]⁺). |
| | | **SOLVENT:** dioxane/H₂O | |
| 1.46 | **5-(4-(3-Aminopiperidin-1-yl)-5,6-difluoro-8-(methylamino)-9H-pyrido[2,3-b]indol-3-yl)nicotinonitrile** | **CORE:** Intermediate A5 | **¹H NMR** (400 MHz, DMSO-d6) δ: 11.97 (s, 1 H), 9.13~9.14 (d, *J*=1.6 Hz, 1 H), 8.90~8.91 (d, *J*=2.0 Hz, 1 H), 8.43 (s, 1 H), 8.24 (s, 1 H), 7.81 (br. s., 3 H), 6.61~6.66(dd, *J*=13.2, 6.0 Hz, 1 H), 2.68-3.26 (m, 8 H), 1.96-1.99 (d, *J*=9.6 Hz,1 H), 1.23 -1.64 (m, 3 H). |
| | | **AMINE:** *tert*-Butyl piperidin-3-ylcarbamate | |
| | 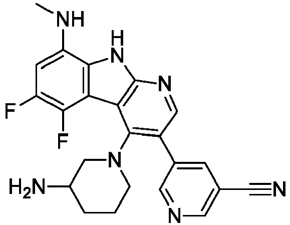 | **BORONIC REAGENT:** 5-(4,4,5,5-Tetramethyl-1,3,2-dioxaborolan-2-yl)nicotinonitrile | |
| | | **CATALYST:** Pd-Ad₂nBuP Biphenyl Precat (CAS#: 1375477-29-4) | **MS** (ESI): 434.3 (M+H)⁺, 217.8 (M/2+H)⁺. |
| | | **SOLVENT:** THF/H₂O | |
| 1.47 | **4-(3-Aminopiperidin-1-yl)-5,6-difluoro-3-(2-methoxypyrimidin-5-yl)-N-methyl-9H-pyrido[2,3-b]indol-8-amine** | **CORE:** Intermediate A5 | **¹H NMR** (400 MHz, DMSO-*d6*) δppm: 11.93 (br. s., 1 H), 8.66 (s, 2 H), 8.22 (br. s., 1 H) 7.83 (br. s., 3 H), 6.62 (dd, *J*=13.2, 6.0 Hz, 1 H), 4.02 (s, 3 H), 2.68 ~3.29 (m, 8 H), 1.99~2.02 (d, *J*=9.6 Hz, 1 H), 1.25-1.63 (m, 3 H). |
| | | **AMINE:** *tert*-Butyl piperidin-3-ylcarbamate | |
| | | **BORONIC REAGENT:** (2-Methoxypyrimidin-5-yl)boronic acid. | |
| | | **CATALYST:** Pd-Ad₂nBuP Biphenyl Precat (CAS#: 1375477-29-4) | |
| | 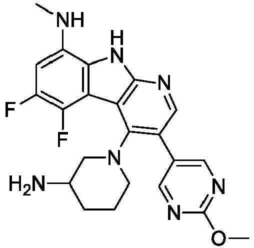 | **SOLVENT:** THF/H₂O | **MS** (ESI): 440.3 (M+H)⁺,220.8(M/2+H)⁺. |
| 1.48 | **5-[4-(7-Amino-2-azaspiro[3.3]heptan-2-yl)-5,6-difluoro-8-(methylamino)-9H-pyrido[2,3-b]indol-3-yl]pyridine-3-carbonitrile** | **CORE:** Intermediate A5 | **¹H NMR** (400MHz, CDCl₃) δ ppm: 8.83 (d, J=2.1 Hz, 1H), 8.78 (d, J=1.8 Hz, 1H), 8.23 (m, 1H), 7.98 (s, 1H), 6.42 (m, 1H), 4.12 (d, J=9.32Hz, 1H), 3.85 (d, J=9.16Hz, 1H), 3.75 (d, J=9.12Hz, 1H), 3.68 (d, J=9.24hz, 1H), 3.45 (m, 1H), 2.87 (s, 3H), 1.96 (m,3H), 1.62 (m, 1H). |
| | | **AMINE:** *tert*-Butyl N-(2-azaspiro[3.3]heptan-7-yl)carbamate (PharmaBlock) | |
| | 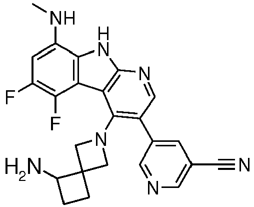 | **BORONIC REAGENT:** 3-Cyanopyridine-5-boronic acid pinacol ester | |
| | | **CATALYST:** Pd-Ad₂nBuP Biphenyl Precat (CAS#: 1375477-29-4) | **MS** (ESI): 446.1 ([M+H]⁺). |
| | | **SOLVENT:** THF/H₂O | |
| 1.49 | **5-(4-(1-Amino-6-azaspiro[3.4]octan-6-yl)-5,6-difluoro-8-(methylamino)-9H-pyrido[2,3-b]indol-3-yl)nicotinonitrile** | **CORE:** Intermediate A5 | **¹H NMR** (400 MHz, MeOH-*d4*) δ ppm 9.00 ∼8.98 (m, 1 H), 8.93 ∼8.91 (m, 1 H), 8.37 - 8.34 (m, 1 H), 8.12∼8.11 (m, 1 H), 6.65~6.59 (m, 1 H), 3.73∼3.71 (m, 1 H), 3.54~3.47 (m, 3 H), 3.32∼3.30 (m, 1 H), 2.97 (s, 3 H), 2.22 ~ 1.72 (m, 6 H). |
| | | **AMINE:** 6-Azaspiro[3.4]octan-3-amine | |
| | 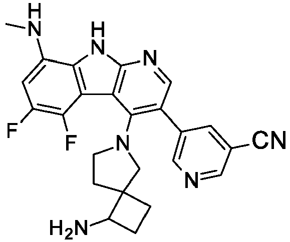 | **BORONIC REAGENT:** 5-(4,4,5,5-Tetramethyl-1,3,2-dioxaborolan-2-yl)nicotinonitrile | |
| | | **CATALYST:** Pd- Ad₂nBuP Biphenyl Precat (CAS#: 1375477-29-4) | **MS** (ESI): 460.2 ([M+H]⁺). |
| | | **SOLVENT:** dioxane/H₂O | |
| 1.50 | **4-[*Cis*-5-methyl-2,3,3a,4,6,6a-hexahydropyrrolo[2,3-c]pyrrol-1-yl]-5,6-difluoro-N-methyl-3-pyrazolo[1,5-a]pyrimidin-6-yl-9H-pyrido[2,3-b]indol-8-amine** | **CORE:** Intermediate A5 | **¹H NMR** (400MHz, CDCl₃) δ ppm: 9.20 (d, J=1.3 Hz, 1H), 8.73 (d, J=2.1Hz, 1H), 8.27 (m, 2H), 6.82 (d, J=1.68 Hz, 1H), 6.64 (m, 1H), 3.26 (m, 1H), 3.01 (s,3H), 2.66 (m, 4H), 2.51 (m,2H), 2.25 (s, 3H), 2.08 (m,2H), 1.77 (m, 1H). |
| | | **AMINE:** *Cis*-5-Methyloctahydropyrrolo [ 2,3-c]pyrrole | |
| | 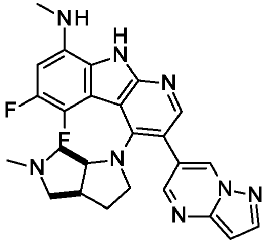 | **BORONIC REAGENT:** 6-(4,4,5,5-Tetramethyl-1,3,2-dioxaborolan-2-yl)pyrazolo[1,5-a]pyrimidine (PharmaBlock) | **MS (ESI): 475.1 ([M+H]⁺).** |
| 1.51 | **4-[4-(Dimethylamino)-5,6-difluoro-8-(methylamino)-9H-pyrido[2,3-b]indol-3-yl]-1H-pyridin-2-one** | **CORE:** Intermediate A5 | **¹H NMR** (400MHz, MeOH-*d4*) δ ppm: 8.07 (s, 1H), 7.56 (d, 1H), 6.66 (d, 1H), 6.57 (s, 1H), 6.44 (d, 1H),3.30 (s, 6H), 2.97 (s, 3H). |
| | | **AMINE:** Dimethylamine hydrochloride | |
| | 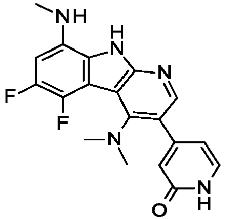 | **BORONIC REAGENT:** 4-(4,4,5,5-Tetramethyl-1,3,2-dioxaborolan-2-yl)-1H-pyridin-2-one | **MS** (ESI): 370.6 ([M+H]⁺). |
| | | **CATALYST:** Pd-Ad₂nBuP Biphenyl Precat (CAS#: 1375477-29-4) | |
| | | **SOLVENT:** THF/H₂O | |
| 1.52 | **(S)-4-(3-(Aminomethyl)piperidin-1-yl)-N-ethyl-6-fluoro-3-(2-methoxypyrimidin-5-yl)-9H-pyrido[2,3-b]indol-8-amine** | **CORE:** Intermediate A4 | **¹H NMR** (400 MHz, DMSO-d6) 11.69 (s, 1 H), 8.64 (s, 2 H), 8.14 (s, 1 H), 7.73 (br s, 3 H), 7.08∼7.11 (dd, *J*=10.0, 2.0 Hz, 1 H), 6.46∼6.50 (dd, *J*=12.0, 2.0 Hz, 1 H), 4.01 (s, 3 H), 2.68∼3.28 (m, 8 H), 1.76∼1.94 (m, 4 H), 1.30∼1.34 (t, *J*=7.2 Hz, 3 H), 1.15 (br d, *J*=10.0 Hz, 1 H). |
| | | **AMINE:** (*R*)*-tert*-Butyl (piperidin-3-ylmethyl)carbamate | |
| | 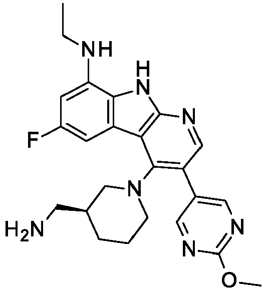 | **BORONIC REAGENT:** (2-Methoxypyrimidin-5-yl)boronic acid | |
| | | **CATALYST:** Pd-Ad₂nBuP Biphenyl Precat (CAS#: 1375477-29-4) | |
| | | **SOLVENT:** THF/H₂O | **MS** (ESI): 450.2 (M+H)⁺, 225.7 (M/2+H)⁺. |
| 1.53 | **(S)-4-(3-(Aminomethyl)piperidin-1-yl)-N-ethyl-5,6-difluoro-3-(2-methoxypyrimidin-5-yl)-9H-pyrido[2,3-b]indol-8-amine** | **CORE:** Intermediate A2 | **¹H NMR** (400 MHz, DMSO-*d6*) δ: 11.96 (s, 1 H), 8.65 (s, 2 H), 8.21 (s, 1 H), 7.69 (br s, 3 H), 6.60~6.66 (dd, *J*=13.6, 6.8 Hz, 1 H), 4.01 (s, 3 H), 3.20∼3.25 (q, *J*=7.2 Hz, 2 H), 2.65∼3.09 (m, 6 H), 1.57∼1.78 (m, 4 H), 1.29∼1.32 (t, *J*=7.2 Hz, 3 H), 1.02 (br d, *J*=9.0 Hz, 1 H). |
| | | **AMINE:** (*R*)-*tert*-Butyl (piperidin-3-ylmethyl)carbamate | |
| | 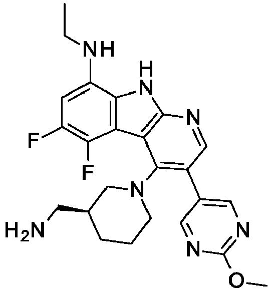 | **BORONIC REAGENT:** (2-Methoxypyrimidin-5-yl)boronic acid | |
| | | **CATALYST:** Pd-Ad₂nBuP Biphenyl Precat (CAS#: 1375477-29-4) | |
| | | **SOLVENT:** THF/H₂O | **MS** (ESI): 468.2 (M+H)⁺, 234.6(M/2+H)⁺. |
| 1.54 | **5-[4-[3-(Aminomethyl)-3-fluoro-1-piperidyl]-5,6-difluoro-8-(methylamino)-9H-pyrido[2,3-b]indol-3-yl]pyridine-3-carbonitrile** | **CORE:** Intermediate A5 | **¹H NMR** (400 MHz, MeOH-*d4*) δ: 8.86 (d, J=1.7 Hz, 1H), 8.82 (d, J=2.0 Hz, 1H), 8.28 (t, J=2.0 Hz, 1H), 8.18 (s, 1H), 6.55 (dd, J=13.1, 6.5 Hz, 1H), 3.37-3.52 (m, 2H), 3.11 (s, 1H), 3.06 (s, 1H), 2.90-3.04 (m, 2H), 2.88 (s, 3H), 1.37∼1.95 (m, 4H). |
| | | **AMINE:** *tert*-Butyl N- [(3-fluoro-3-piperidyl)methyl]carbam ate | |
| | 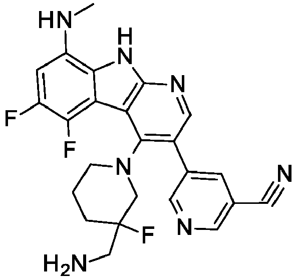 | **BORONIC REAGENT:** (5-Cyanopyridin-3-yl)boronic acid | |
| | | **CATALYST:** Xphos Pd G2(CAS: 1310584-14-5) | **MS** (ESI): 466.4([M+H]⁺). |
| | | **SOLVENT:** THF/H₂O | |
| 1.55 | 2- **[4- [4-(Dimethylamino)-5,6-difluoro-8-(methylamino)-9H-pyrido[2,3-b]indol-3-yl]-2-pyridyl]-N-methyl-acetamide** | **CORE:** Intermediate A5 | **¹H NMR** (400 MHz, MeOH-*d4*) δ ppm: 8.53 - 8.64 (m, 1 H) 8.11 (s, 1 H) 7.39 - 7.45 (m, 1 H) 7.32 - 7.38 (m, 1 H) 6.53 - 6.63 (m, 1 H) 3.81 (s, 2 H) 2.99 (s, 3 H) 2.86 (d, *J*=2.32 Hz, 6 H) 2.80 (s, 3 H). |
| | | **AMINE:** Dimethylamine hydrochloride | |
| | | **BORONIC REAGENT:** [6-[2-(Methylamino)-2-oxo-ethyl]-3-pyridyl]boronic acid | |
| | 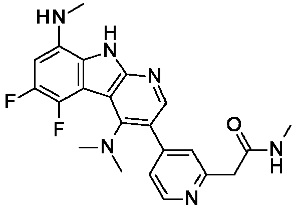 | **CATALYST:** Pd-Ad₂nBuP Biphenyl Precat (CAS#: 1375477-29-4) | **MS** (ESI): 425.4 ([M+H]⁺) |
| | | **SOLVENT:** THF/H₂O | |
| 1.57 | **4-(3,6-Diazabicyclo[3.1.1]heptan-6-yl)-5,6-difluoro-3-(2-methoxypyrimidin-5-yl)-N-methyl-9H-pyrido[2,3-b]indol-8-amine** | **CORE:** Intermediate A5 | **¹H NMR** (400 MHz, DMSO-*d₆*) δ: 8.70 (s, 2 H), 7.99 (s, 1 H), 6.42~6.47 (dd, *J*=13.6, 6.4 Hz, 1 H), 5.57~5.58 (br d, *J*=4.4 Hz, 1 H), 4.20 (br s, 2 H), 4.00 (s, 3 H), 2.89∼2.91 (d, *J*=48 Hz, 3 H), 2.57∼2.73 (m, 3 H), 2.47 (br s, 2 H), 1.69∼1.71 (d, *J*=8.0 Hz, 1H). |
| | | **AMINE:** *tert*-Butyl 3,6-diazabicyclo[3.1.1]hepta ne-6-carboxylate- b]pyrrole | |
| | 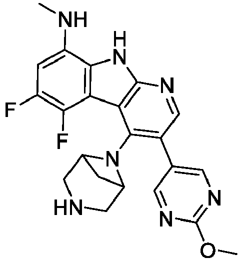 | **BORONIC REAGENT:** (2-Methoxypyrimidin-5-yl)boronic acid | |
| | | **CATALYST:** Pd-Ad₂nBuP Biphenyl Precat (CAS#: 1375477-29-4) | **MS** (ESI): 438.2 (M+H)⁺, 219.8 (M/2+H)⁺. |
| | | **SOLVENT:** THF/H₂O | |
| 1.58 | **5-(4-(3,6-Diazabicyclo[3.1.1]heptan-6-yl)-5,6-difluoro-8-(methylamino)-9H-pyrido[2,3-b]indol-3-yl)nicotinonitrile** | **CORE:** Intermediate A5 | **¹H NMR** (400 MHz, DMSO-*d₆*) δ ppm: 8.96 (s, 1 H), 8.88 (s, 1 H), 8.39 (br s, 1 H), 8.07 (s, 1 H), 6.42∼6.50 (dd, *J*=13.6, 6.4 Hz, 1 H), 5.50 (br s, 1 H), 4.16 (br s, 2 H), 2.90∼2.92 (br d, *J*=4.4 Hz, 3 H), 2.69∼2.74 (q, *J*=6.8 Hz, 1H), 2.57 - 2.61 (m, 2 H),2.47 (m, 2 H), 1.68∼1.70 (d,*J*=8.0 Hz, 1 H). |
| | | **AMINE:** *tert*-Butyl 3,6-diazabicyclo[3.1.1]hepta ne-6-carboxylate | |
| | | **BORONIC REAGENT:** 5-(4,4,5,5-Tetramethyl-1,3,2-dioxaborolan-2-yl)nicotinonitrile. | |
| | 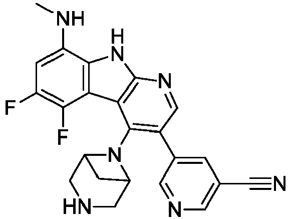 | **CATALYST:** Pd-Ad₂nBuP Biphenyl Precat (CAS#: 1375477-29-4) | **MS** (ESI): 432.3 (M+H)⁺, 216.8 (M/2+H)⁺. |
| | | **SOLVENT:** THF/H₂O | |
| 1.59 | **4-(3,6-Diazabicyclo[3.1.1]heptan-6-yl)-3-(2-ethoxypyrimidin-5-yl)-6-fluoro-N-methyl-9H-pyrido[2,3-b]indol-8-amine** | **CORE:** Intermediate A1 | **¹H NMR** (400 MHz, DMSO-*d₆*) δ ppm: 12.06 (br s, 1 H), 9.48 (br s, 1 H), 8.71~8.75 (m, 3 H), 8.19 (s, 1 H), 6.85~6.88 (m, 1 H), 6..45∼6.48 (m, 1 H), |
| | | **AMINE:** *tert*-Butyl 3,6-diazabicyclo[3.1.1]hepta ne-6-carboxylate | |
| | | **BORONIC REAGENT:** | |
| | 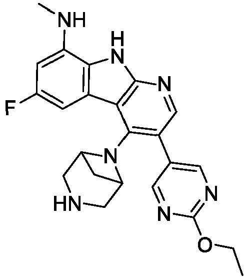 | (2-Ethoxypyrimidin-5-yl)boronic acid. | 4.40∼4.51 (m, 4 H), 2.95-3.18 (m, 5 H), 2.91 (s, 3 H), 1.84∼1.86 (br d, *J*=9.6 Hz, 1 H), 1.37∼1.40 (t, *J*=6.8 Hz, 3 H). |
| | | **CATALYST:** Pd-Ad₂nBuP Biphenyl Precat (CAS#: 1375477- | |
| | | 29-4) **SOLVENT:** THF/H₂O | **MS** (ESI): 434.1 (M+H)⁺. |
| 1.60 | **5-(4-(3,6-Diazabicyclo[3.1.1]heptan-6-yl)-6-fluoro-8-(methylamino)-9H-pyrido[2,3-b]indol-3-yl)nicotinonitrile** | **CORE:** Intermediate A1 | **¹H NMR** (400 MHz, DMSO-*d₆*) δ ppm: 8.89~8.93 (m, 2 H), 8.34~8.35 (t, *J*=2.0 Hz, 1 H), 8.01 (s, 1 H), 6.79~6.82 (dd, *J*=10.8, 2.0 Hz, 1 H), 6.37~6.40 (dd, *J*=12.0, 2.0 Hz, 1 H), 5.72 -5.73 (br d, *J*=3.6 Hz, 1 H), 4.24 (br s, 2 H), 2.93∼2.94 (br d, *J*=4.4 Hz, 3 H), 2.76 -2.80 (m, 3 H), 2.63∼2.68 (m, 2 H), 1.74 -1.76 (d, *J*=8.4 Hz, 1 H). |
| | | **AMINE:** *tert*-Butyl 3,6-diazabicyclo[3.1.1]hepta ne-6-carboxylate | |
| | | **BORONIC REAGENT:** 5-(4,4,5,5-Tetramethyl-1,3,2-dioxaborolan-2-yl)nicotinonitrile. | |
| | 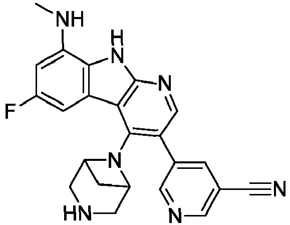 | **CATALYST:** Pd-Ad₂nBuP Biphenyl Precat (CAS#: 1375477-29-4) | |
| | | **SOLVENT:** THF/H₂O | **MS** (ESI): 414.1 (M+H)⁺. |
| 1.61 | **4-(3,6-Diazabicyclo[3.1.1]heptan-6-yl)-6-fluoro-3-(2-methoxypyrimidin-5-yl)-N-methyl-9H-pyrido[2,3-b]indol-8-amine** | **CORE**: Intermediate A1 | **¹H NMR** (400 MHz, DMSO-*d₆*) δ ppm: 11.08 (br s., 1 H), 8.66 (s, 2 H), 7.926 (s, 1 H), 6.79~6.81 (m, 1 H), 6.37 (br d, *J*=12.0 Hz, 1 H), 5.70 (br s, 1 H), 4.26~4.28 (br d, *J*=5.6 Hz, 2 H), 4.00 (s, 3 H), 2.92∼2.94 (br d, *J*=4.8 Hz, 3 H), 2.72∼2.76 |
| | | **AMINE:** *tert*-Butyl 3,6-diazabicyclo[3.1.1]hepta ne-6-carboxylate | |
| | | **BORONIC REAGENT:** (2-Methoxypyrimidin-5-yl)boronic acid | |
| | 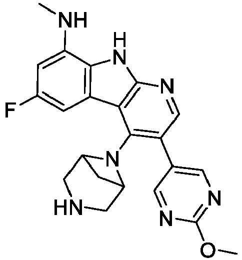 | **CATALYST** : Pd-Ad₂nBuP Biphenyl Precat (CAS#: 1375477-29-4) | (m, 3 H), 2.54∼2.57 (br d, *J*=12.8 Hz, 2 H), 1.74∼2.76 (d, *J*=8.0 Hz, 1 H). |
| | | **SOLVENT:** THF/H₂O | MS (ESI): 420.2 (M+H)⁺. |
| 1.62 | **5,6-Difluoro-N4,N4,N8-trimethyl-3-pyrazolo[1,5-a]pyrimidin-6-yl-9H-pyrido[2,3-b]indole-4,8-diamine** | **CORE:** Intermediate A5 | **¹H NMR** (400MHz, CDCl₃) δ ppm: 9.01 (m, 1H), 8.78 (m,1H), 8.24 (m, 2H), 6.81 (m, 1H), 6.61 (m, 1H), 2.99 (s, 3H), 2.96 (s, 6H). |
| | | **AMINE:** Dimethyl amine hydrochloride (PharmaBlock) | |
| | 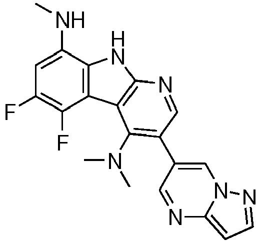 | **BORONIC REAGENT:** 6-(4,4,5,5-Tetramethyl-1,3,2-dioxaborolan-2-yl)pyrazolo[1,5-a]pyrimidine (PharmaBlock) | **MS** (ESI): 394.1 ([M+H]⁺). |
| | | **CATALYST:** Pd-Ad₂nBuP Biphenyl Precat (CAS#: 1375477-29-4) | |
| | | **SOLVENT:** THF/H₂O | |
| 1.63 | 5,6-Difluoro-3-(2-methoxypyrimidin-5-yl)-N-methyl-4-[(3aS,6aS)-5-methyl-2,3,3a,4,6,6a-hexahydropyrrolo[2,3-c]pyrrol-1-yl]-9H-pyrido[2,3-b]indol-8-amine | **CORE:** Intermediate A9 | **¹H** NMR (400 MHz, DMSO-*d6*) δ ppm: 1.62 - 1.69 (m, 1 H), 1.73 (br dd, *J*=9.72, 5.07 Hz, 1 H), 1.94 - 2.01 (m, 1 H), 2.09 (s, 3 H), 2.17 (br t, *J*=8.25 Hz, 1 H), 2.39 (br d, *J*=9.78 Hz, 1 H), 2.84 (br d, *J*=7.95 Hz, 2 H), 2.88 (br d, *J*=4.52 Hz, 3 |
| | | **AMINE:** (3aS,6aS)-5-Methyl- 2,3,3a,4,6,6a-hexahydro-1H-pyrrolo[3,4-b]pyrrole (Accela ChemBio) | |
| | | **BORONIC REAGENT:** (2-Methoxypyrimidin-5- | |
| | 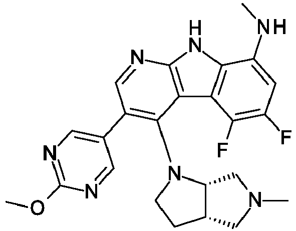 | yl)boronic acid | H), 2.95 - 2.99 (m, 2 H), 3.98 (s, 3 H), 4.45 (br s, 1 H), 5.64 (br d, *J*=4.65 Hz, 1 H), 6.55 (br dd, *J*=13.39, 5.93 Hz, 1 H), 8.06 (d, *J*=0.73 Hz, 1 H), 8.65 (s, 2 H), 11.66 (br s, 1 H). |
| | | **CATALYST:** Pd-Ad₂nBuP Biphenyl Precat (CAS#: 1375477-29-4) | |
| | | **SOLVENT:** THF/H₂O | **MS** (ESI): 466.1 ([M+H]⁺). |

### Example 2.01

### 1-[5-[4-(Dimethylamino)-8-(ethylamino)-6-fluoro-9H-pyrido[2,3-b]indol-3-yl]pyrimidin-2-yl]cyclopropanecarboxylic acid

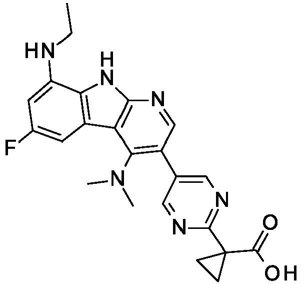

### Step (a) Preparation of ethyl 1-[5-[8-[tert-butoxycarbonyl(ethyl)aminol-4-(dimethylamino)-6-fluoro-9H-pyrido[2,3-b]indol-3-yl]pyrimidin-2-yl]cyclopropanecarboxylate (compound 2.01a)

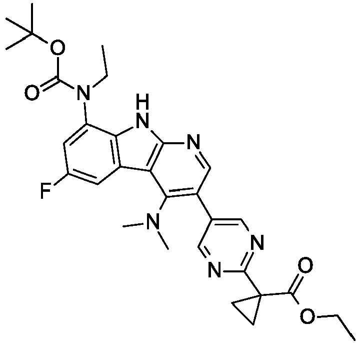

Compound 2.01a was prepared in analogy to step (a) and (b) of **Example 1.01,** by using *tert-*butyl N-(3,4-dichloro-6-fluoro-9H-pyrido[2,3-b]indol-8-yl)-N-methyl-carbamate **(intermediate A1** as the **"CORE"),** and replacing (*R*)-3-(Boc-amino)pyrrolidine with dimethylamine hydrochloride, as the **"AMINE",** in step (a), and replacing 3-cyanopyridine-5-boronic acid pinacol ester with 2-(1-ethoxycarbonylcyclopropyl) pyrimidin-5-yl]boronic **acid(intermediate B1**), as the **"BORONIC REAGENT",** in step (b).

### Step (b) Preparation of 1-[5-[4-(dimethylamino)-8-(ethylamino)-6-fluoro-9H-pyrido[2,3-b]indol-3-yl]pyrimidin-2-yl]cyclopropanecarboxylic acid (Example 2.01)

To a solution of ethyl 1-[5-[8-[*tert*-butoxycarbonyl(ethyl)amino]-4-(dimethylamino)-6-fluoro-9H-pyrido[2,3-b]indol-3-yl]pyrimidin-2-yl]cyclopropanecarboxylate (45.0 mg, 0.077mmol) in THF (0.4 mL) and water (0.1 mL) was added sodium hydroxide (154.0 mg, 3.85 mmol). Reaction continued for 12 h at 30 °C, and the reaction mixture was poured into water (10 mL) afterwards. pH of the reaction mixture was adjusted to about 7 by careful addition of HCl (IN). The mixture was then extracted with DCM/methanol (10:1, 50 mL) for three times. The combined organic layer was washed with brine (50 mL) for twice, dried over Na₂SO₄, and concentrated *in vacuo.* Further drying under high vacuum gave crude 1-[5-[8-[*tert-*butoxycarbonyl(ethyl)amino]-4-(dimethylamino)-6-fluoro-9H-pyrido[2,3-b]indol-3-yl]pyrimidin-2-yl]cyclopropanecarboxylic acid (50.0 mg) as yellow solid. MS (ESI): 535.23 ([M+H]+).

The above crude 1-[5-[8-[*tert*-butoxycarbonyl(ethyl)amino]-4-(dimethylamino)-6-fluoro-9H-pyrido[2,3-b]indol-3-yl]pyrimidin-2-yl]cyclopropanecarboxylic acid (48.0 mg, 0.070 mmol) was dissolved in dry DCM (2.0 mL), to which was added TFA (1.0 mL) dropwise. The solution was stirred for 1 h at 30 °C. LCMS analysis indicated the completion of the reaction. Then the reaction was cooled to room temperature and concentrated *in vacuo.* The residue was purified by prep-HPLC to give 1-[5-[4-(dimethylamino)-8-(ethylamino)-6-fluoro-9H-pyrido[2,3-b]indol-3-yl]pyrimidin-2-yl]cyclopropanecarboxylic acid (26.0 mg, 63% yield) as yellow solid. ¹H NMR (400 MHz, METHANOL-d4) δ ppm: 8.73 (s, 2H), 8.04 (s, 1H), 6.96 (d, 1H), 6.43 (d, 1H), 3.23 (m, 2H),2.84 (s, 6H), 1.76 (d, 4H), 1.31 (m, 3H). MS (ESI): 435.2 ([M+H]⁺).

The following examples were prepared in analogy to **Example 2.01,** replacing *tert*-butyl N-(3,4-dichloro-6-fluoro-9H-pyrido[2,3-b]indol-8-yl)-N-methyl-carbamate **(Intermediate A1**) as the **"CORE"** in step (a), dimethylamine hydrochloride as the **"AMINE"** in step (a), THF/H₂O as the **"SOLVENT",** Ad₂nBuP Biphenyl as the **"CATALYST"** in step (a) and [2-(1-Ethoxycarbonylcyclopropyl) pyrimidin-5-yl]boronic acid **(Intermediate B1**) as the **"BORONIC REAGENT"** in step (a) with the reagents indicated in Table 2 respectively.

**Table 2**

| **No.** | **Compound Name and Structure** | **CORE, AMINE, BORONIC REAGENT, CATALYST and SLOVENT** | **¹H NMR and MS (ESI)** |
|---|---|---|---|
| 2.02 | **1- [5- [4-(Dimethylamino)-5,6-difluoro-8-(methylamino)-9H-pyrido[2,3-b]indol-3-yl]pyrimidin-2-yl]cyclopropanecarboxylic acid** | **CORE:** Intermediate A5 | **¹H NMR** (400 MHz, |
| | | **AMINE:** Dimethylamine hydrochloride | MeOH-*d4*) δ ppm: 8.82 (s, 2H), 8.15 (s, 1H), 6.62 (m, 1H), 2.99 (s, 3H), 2.91 (s, 6H), 1.87 (m, 4H). |
| | | **BORONIC REAGENT:** Intermediate B1 | **MS** (ESI): 439.1 ([M+H]⁺) |
| | 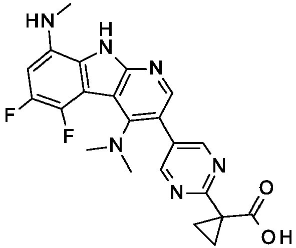 | **CATALYST:** Pd-Ad₂nBuP Biphenyl Precat (CAS#: 1375477-29-4) | |
| | | **SOLVENT:** THF/H₂O | |
| 2.03 | **1-(5-(4-(Dimethylamino)-6,7-difluoro-8-(methylamino)-9H-pyrido[2,3-b]indol-3-yl)pyrimidin-2-yl)cyclopropanecarboxylic acid** | **CORE:** Intermediate A8 | **¹H NMR** (400 MHz, DMSO-*d6*) δ ppm 11.74 (s, 1 H), 8.80 (s, 2 H), 8.17 (s, 1 H), 7.23~7.27 (dd, *J*=10.8, 6.8 Hz, 1 H), 3.11-3.12 (d, *J*=4.0 Hz, 3 H), 2.79 (s, 6 H), 1.57- 1.64 (m, 4 H). |
| | | **AMINE:** Dimethylamine hydrochloride | |
| | | **BORONIC REAGENT:** Intermediate B1 | |
| | | **CATALYST:** Pd-Ad₂nBuP Biphenyl Precat (CAS#: 1375477-29-4) | MS (ESI): 439.1 (M+H)⁺, 220.1(M/2+H)⁺. |
| | | **SOLVENT:** THF/H₂O | |
| | 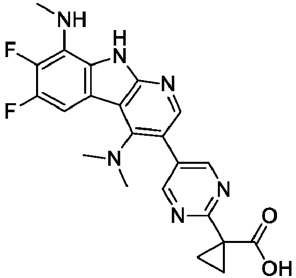 | | |

### Example 3.01

### 5-[4-[4-(Aminomethyl)-3,3-difluoro-pyrrolidin-1-yl]-6-fluoro-8-(methylamino)-9H-pyrido[2,3-b]indol-3-yl]pyridine-3-carbonitrile

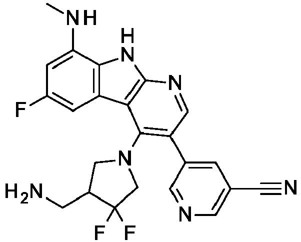

### Step (a) Preparation of tert-butyl N-[4-[4-[(tert-butoxycarbonylamino)methyl]-3,3-difluoro-pyrrolidin-1-yl]-3-chloro-6-fluoro-9H-pyrido[2,3-b]indol-8-yl]-N-methyl-carbamate

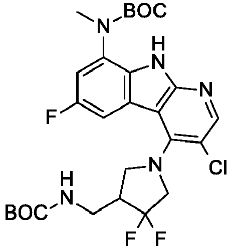

To the solution of *tert-butyl* N-(3,4-dichloro-6-fluoro-9H-pyrido[2,3-b]indol-8-yl)-N-methyl-carbamate **(Intermediate A1,** 150 mg, 0.40 mmol, as the **"CORE"** in Table 3) and *tert-*butyl N-[(4,4-difluoropyrrolidin-3-yl)methyl]carbamate (142 mg, 0.60 mmol, as the **"AMINE")** in 1,4-dioxane (5.0 mL) was added Cs₂CO₃ (260 mg, 0.60 mmol). The mixture was degassed with nitrogen for 2 min. Pd(OAc)₂ (4.6 mg, 0.02 mmol) and BINAP (24 mg, 0.04 mmol), combined as the **"CATALYST",** were added under nitrogen. The suspension was heated to 80 °C and stirred for 12 h. Then the reaction mixture was poured into water (50 mL) and extracted with ethyl acetate (50 mL). The organic layer was washed with IN CaCl₂ (50 mL) twice and brine (100 mL), dried over Na₂SO₄ and concentrated *in vacuo.* The product was purified by prep-HPLC to give the *tert*-butyl N-[4-[4-[(*tert*-butoxycarbonylamino)methyl]-3,3-difluoro-pyrrolidin-1-yl]-3-chloro-6-fluoro-9H-pyrido[2,3-b]indol-8-yl]-N-methyl-carbamate (198.2 mg, 83% yield) as a white solid. MS (ESI): 584.2 ([{³⁵Cl}M+H]+), 586.2 ([{³⁷Cl}M+H]⁺).

### Step (b) Preparation of tert-butyl N-[4-[4-[(tert-butoxycarbonylamino)methyl]-3,3-difluoro-pyrrolidin-1-yl]-3-(5-cyano-3-pyridyl)-6-fluoro-9H-pyrido[2,3-b]indol-8-yl]-N-methyl-carbamate

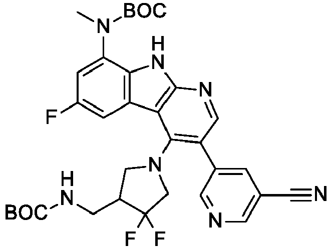

A solution of *tert*-butyl N-[4-[4-[(*tert*-butoxycarbonylamino)methyl]-3,3-difluoro-pyrrolidin-1-yl]-3-chloro-6-fluoro-9H-pyrido[2,3-b]indol-8-yl]-N-methyl-carbamate (90.0 mg, 0.154 mmol), 3-cyanopyridine-5-boronic acid pinacol ester (45.7 mg, 0.199 mmol, as the **"BORONIC REAGENT"** in Table 3), CsF (7.0 mg, 0.462 mmol),), tris(dibenzylideneacetone)dipalladium(0) (28.2 mg, 0.03 mmol, Alfa Aesar), and XPhos (14.3 mg, 0.03 mmol, Sigma-Aldrich) in the mixture of dioxane (5.0 mL) and water (0.5 mL) was stirred for 12 h at 100°C under N₂ atmosphere. Then the reaction mixture was cooled to room temperature and poured into water (50 mL). The mixture was extracted with ethyl acetate (50 mL). The organic layer was washed with IN CaCl₂ (50 mL) twice and brine (50 mL) twice, dried over Na₂SO₄ and concentrated *in vacuo.* The residue was purified by silica gel flash chromatography to give *tert*-butyl N-[4-[4-[(*tert*-butoxycarbonylamino)methyl]-3,3-difluoro-pyrrolidin-1-yl]-3-(5-cyano-3-pyridyl)-6-fluoro-9H-pyrido[2,3-b]indol-8-yl]-N-methyl-carbamate (51.2 mg, 51% yield) as a yellow solid. MS (ESI): 652.2 ([M+H]⁺).

### Step (c) Preparation of 5-[4-[4-(aminomethyl)-3,3-difluoro-pyrrolidin-1-yl]-6-fluoro-8-(methylamino)-9H-pyrido[2,3-b]indol-3-yl]pyridine-3-carbonitrile

To a solution of *tert*-butyl N-[4-[4-[(*tert*-butoxycarbonylamino)methyl]-3,3-difluoro-pyrrolidin-1-yl]-3-(5-cyano-3-pyridyl)-6-fluoro-9H-pyrido[2,3-b]indol-8-yl]-N-methyl-carbamate (45.2 mg, 0.069 mmol) in dry DCM (2.0 mL) was added TFA (0.4 mL). The solution was stirred for 0.5 h at room temperature and concentrated *in vacuo.* The residue was purified by prep-HPLC (0.5% TFA in water) to give 5-[4-[4-(aminomethyl)-3,3-difluoro-pyrrolidin-1-yl]-6-fluoro-8-(methylamino)-9H-pyrido[2,3-b]indol-3-yl]pyridine-3-carbonitrile (22.5 mg, 72 % yield) as a yellow solid. ¹H NMR (400 MHz, METHANOL-d4): δ 9.03 (d, J=1.6Hz, 1H), 8.93 (s, 1H), 8.36 (m, 1H), 8.24 (s, 1H), 6.97 (m, 1H), 6.56 (m, 1H), 3.95 (m, 1H), 3.78 (m, 1H), 3.65 (m, 1H), 3.37 (m, 1H), 3.09-3.20 (m, 3H), 3.03 (s, 3H). MS (ESI): 452.2 ([M+H]⁺)

The following examples were prepared in analogy to **Example 3.01,** replacing *tert*-butyl N-(3,4-dichloro-6-fluoro-9H-pyrido[2,3-b]indol-8-yl)-N-methyl-carbamate **(Intermediate A1**) as the **"CORE"** in step (a), Pd(OAc)₂ and BINAP as the **"CATALYST"** in step (a), *tert*-butyl N-[(4,4-difluoropyrrolidin-3-yl)methyl]carbamate as the **"AMINE"** in step (a), and 3-cyanopyridine-5-boronic acid pinacol ester as the **"BORONIC REAGENT"** in step (a) with the reagents indicated in Table 3 respectively.

**Table 3**

| **No.** | **Compound Name and Structure** | **CORE, AMINE, CATALYST** and **BORONIC REAGENT** | **¹H NMR and MS (ESI)** |
|---|---|---|---|
| 3.02 | **4-[4-(Aminomethyl)-3,3-difluoro-pyrrolidin-1-yl]-6-fluoro-3-(2-methoxypyrimidin-5-yl)-N-methyl-9H-pyrido[2,3-b]indol-8-amine** | **CORE:** Intermediate A1 | ¹H NMR (400 MHz, MeOH-*d4*) δ ppm: 8.69 (s, 2H), 8.21 (s, 1H), 6.97 (d, J=9.6Hz, 1H), 6.55 (d, J=9.6Hz, 1H), 4.13 (s, 3H), 3.96 (m, 1H), 3.82 (m, 1H), 3.73 (m, 1H), 3.37 (m, 1H), 3.08-3.25 (m, 3H), 3.02 (s, 3H). |
| | | **AMINE:** *tert*-Butyl N-[(4,4-difluoropyrrolidin-3-yl)methyl]carbamate | |
| | | **CATALYST:** Pd(OAc)₂ and BINAP | |
| | 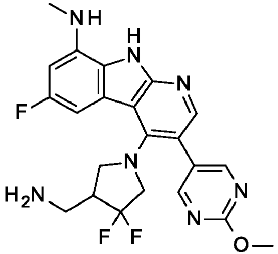 | **BORONIC REAGENT:** 2-Methoxy-5-(4,4,5,5-tetramethyl-1,3,2-dioxaborolan-2-yl)pyrimidine | |
| | | | **MS** (ESI): 458.2 ([M+H]⁺). |
| 3.03 | **4-[4-(Aminomethyl)-3,3-difluoro-pyrrolidin-1-yl]-5,6-difluoro-3-(2-methoxypyrimidin-5-yl)-N-methyl-9H-pyrido[2,3-b]indol-8-amine** | **CORE:** Intermediate A5 | ¹H NMR (400 MHz, MEOH-*d4*) δ ppm: 8.67 (s, 2H), 8.28 (s, 1H), 6.65 (m, 1H), 4.12 (s, 3H), 3.88 (m, 2H), 3.58 (m, 1H), 3.30 (m, 1H), 3.11-3.23 (m, 3H), 3.00 (s, 3H). |
| | | **AMINE:** *tert*-Butyl N-[(4,4-difluoropyrrolidin-3-yl)methyl]carbamate | |
| | | **CATALYST:** Pd(OAc)₂ and BINAP | |
| | 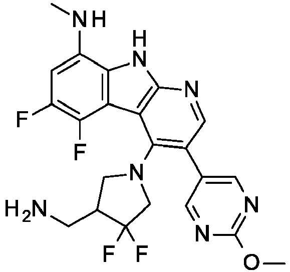 | **BORONIC REAGENT:** 2-Methoxy-5-(4,4,5,5-tetramethyl-1,3,2-dioxaborolan-2-yl)pyrimidine | **MS** (ESI): 476.2 ([M+H]⁺) |
| 3.04 | **5-[4-[4-(Aminomethyl)-3,3-difluoro-pyrrolidin-1-yl]-5,6-difluoro-8-(methylamino)-9H-pyrido[2,3-b]indol-3-yl]pyridine-3-carbonitrile** | **CORE:** Intermediate A5 | ¹H NMR (400 MHz, MeOH-*d4*) δ ppm: 9.02 (d, 1H), 8.91 (d, 1H), 8.35 (d, 1H), 8.31 (s, 1H), 6.66 (m, 1H), 3.86 (m, 2H), 3.53 (m, 1H), 3.30 (m, 1H), 3.13 (m, 3H), 3.00 (s, 3H). |
| | | **AMINE:** *tert*-Butyl N-[(4,4-difluoropyrrolidin-3-yl)methyl]carbamate | |
| | | **CATALYST:** Pd(OAc)₂ and BINAP | |
| | 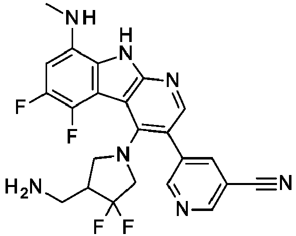 | **BORONIC REAGENT:** 3-Cyanopyridine-5-boronic acid pinacol ester | MS: 470.1 ([M+H]⁺). |

### Example 4.01

### 4-(1-(Aminomethyl)cyclopropyl)-6-fluoro-3-(2-methoxypyrimidin-5-yl)-N-methyl-9H-pyrido[2,3-b]indol-8-amine

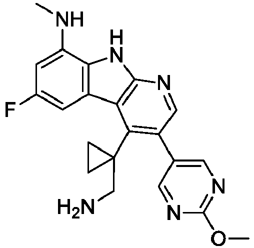

The titled compound was synthesized according to the following scheme:

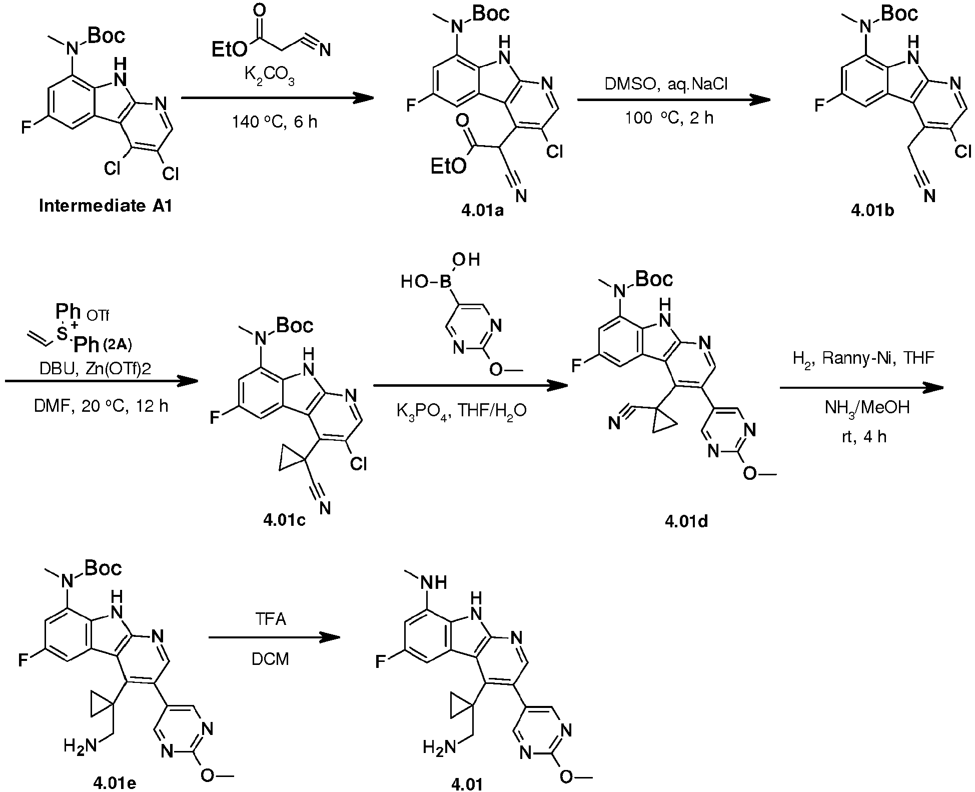

### Step (a) Preparation of ethyl 2-[8-[tert-butoxycarbonyl(methyl)aminol-3-chloro-6-fluoro-9H-pyrido[2,3-b]indol-4-yl]-2-cyano-acetate (compound 4.01a)

To a solution of *tert*-butyl N-(3,4-dichloro-6-fluoro-9H-pyrido[2,3-b]indol-8-yl)-N-methyl-carbamate (intermediate A1 3.0 g, 7.81 mmol, 1 eq) in potassium carbonate (3.2 g, 23.42 mmol, 3 eq) was added ethyl cyanoacetate (50.0 mL, 469.9 mmol, 60.18 eq), and then the mixture was stirred for 6 h at 140 °C. The mixture was diluted with EtOAc (100 mL), and poured into water (200.0 mL), and extracted with EtOAc (100.0 mL) three times. The organic layer was dried over anhydrous Na₂SO₄, and concentrated to give the crude compound of ethyl 2-[8-[*tert-*butoxycarbonyl(methyl)amino]-3-chloro-6-fluoro-9H-pyrido[2,3-b]indol-4-yl]-2-cyano-acetate (5.3 g, 11.55 mmol, 51.4% yield) as a yellow oil, which was used in next step directly. MS (ESI) : 461.1 ([{³⁵Cl}M+H]+), 405.0 ([{³⁵Cl}M-tBu+H]⁺), 361.1 ([{³⁵Cl}M-Boc+H]⁺), 483.1 ([{³⁵Cl}M+Na]⁺).

### Step (b) Preparation of tert-butyl N-[3-chloro-4-(cyanomethyl)-6-fluoro-9H-pyrido[2,3-b]indol-8-yl]-N-methyl-carbamate (compound 4.01b)

To a solution of ethyl 2-[8-[*tert*-butoxycarbonyl (methyl)amino]-3-chloro-6-fluoro-9H-pyrido[2,3-b]indol-4-yl]-2-cyano-acetate (5.3 g, 3.91 mmol, 1 eq) in DMSO (20 mL) and water (10 mL) was added sodium chloride (1.1 g, 19.55 mmol, 5 eq), and the reaction mixture was stirred for 2 h at 100 °C. The mixture was diluted with EtOAc (100 mL), and poured into water (200 mL), and extracted with EtOAc (200 mL). The organic layer was dried over anhydrous Na₂SO₄, filtered, concentrated to give the crude product, which was purified by column chromatography on silica gel eluted with (Petroleum ether: EtOAc= 3:1, v/v) to give *tert*-butyl N-[3-chloro-4-(cyanomethyl)-6-fluoro-9H-pyrido[2,3-b]indol-8-yl]-N-methyl-carbamate (1500 mg, 3.86 mmol, 98.7% yield) as a yellow solid.MS (ESI) : 389.0 ([{³⁵Cl}M+H]+), 333.0 ([{³⁵Cl}M-tBu+H]⁺), 289.0 ([{³⁵Cl}M-Boc+H]⁺).

### Step (c) Preparation of tert-butyl N-[3-chloro-4-(1-cyanocyclopropyl)-6-fluoro-9H-pyrido[2,3-b]indol-8-yl]-N-methyl-carbamate (compound 4.01c)

A mixture of *tert-butyl* N-[3-chloro-4-(cyanomethyl)-6-fluoro-9H-pyrido[2,3-b]indol-8-yl]-N-methyl-carbamate (800.0 mg, 2.06 mmol, 1 eq), diphenyl(vinyl)sulfonium; trifluoromethanesulfonate (894.74 mg, 2.47 mmol, 1.2 eq), zinc trifluoromethanesulfonate (897.55 mg, 2.47 mmol, 1.2 eq) and 1,8-diazabicyclo[5.4.0]undec-7-ene (0.92 mL, 6.17 mmol, 3 eq) in anhydrous DMF (5 mL) was stirred for 12 h at 20 °C. The mixture was diluted with EtOAc (100 mL), and poured into water (100 mL), and extracted with EtOAc (100 mL). The organic layer was dried over anhydrous Na₂SO₄, filtered, concentrated to give the crude product, which was purified by column chromatography on silica gel eluted with (Petroleum ether: EtOAc=5:1) to give *tert*-butyl N-[3-chloro-4-(1-cyanocyclopropyl)-6-fluoro-9H-pyrido[2,3-b]indol-8-yl]-N-methyl-carbamate (665 mg, 1.6 mmol, 70.4% yield) as a yellow solid. MS (ESI) : 415.1 ([{³⁵Cl}M+H]+), 359.0 ([{³⁵Cl}M-tBu+H]⁺), 315.0 ([{³⁵Cl}M-Boc+H]⁺).

### Step (d) Preparation of tert-butyl (4-(1-cyanocyclopropyl)-6-fluoro-3-(2-methoxypyrimidin-5-yl)-9H-pyrido[2,3-blindol-8-yl)(methyl)carbamate (compound 4.01d)

A solution of *tert*-butyl (3-chloro-4-(1-cyanocyclopropyl)-6-fluoro-9H-pyrido[2,3-b]indol-8-yl)(methyl)carbamate (70.0 mg, 0.169 mmol), (2-methoxypyrimidin-5-yl)boronic acid (78.0 mg, 0.506 mmol) and K₃PO₄ (143.0 mg, 0.676 mmol) in THF/H₂O (3.0 mL, v/v=10:1), and then Pd-Ad₂nBuP Biphenyl (23.0 mg, 0.064 mmol) was added to the mixture in glovebox under argon. The mixture was stirred at 70 °C for 12 h under argon. TLC showed the starting material was consumed and product was found. The mixture was poured into water (50 mL) and extracted by EA (100 mL) two times. The organic layer was washed by brine (50 ml) two times, dried with Na₂SO₄ and concentrated under reduced pressure to give crude product. The crude product was purified Prep-TLC (DCM: MeOH=30:1) to give *tert*-butyl (4-(1-cyanocyclopropyl)-6-fluoro-3-(2-methoxypyrimidin-5-yl)-9H-pyrido[2,3-b]indol-8-yl)(methyl)carbamate (compound 1.56.2) (70.0 mg, yield: 84.92%) as a yellow solid. MS (ESI): 489.1 (M+H)⁺.

### Step (e) Preparation of tert-butyl (4-(1-(aminomethyl)cyclopropyl)-6-fluoro-3-(2-methoxypyrimidin-5-yl)-9H-pyrido[2,3-b]indol-8-yl)(methyl)carbamate (compound 4.01e)

To a solution of *tert*-butyl (4-(1-cyanocyclopropyl)-6-fluoro-3-(2-methoxypyrimidin-5-yl)-9H-pyrido[2,3-b]indol-8-yl)(methyl)carbamate (compound 1.56.2) (70.0 mg, 0.143 mmol) and NH₃/MeOH (2.0 mL, 1M) in THF (2.0 mL) was added was added Raney-Ni (20.0 mg) under N₂, then the suspension was degassed under vacuum and purged with H₂ several times. The reaction mixture was stirred at 28 °C for 4 h under H₂ (50 psi). LCMS showed the SM was consumed, and product formed. The mixture was filtered and the filtrate was concentrated to give *tert*-butyl (4-(1-(aminomethyl)cyclopropyl)-6-fluoro-3-(2-methoxypyrimidin-5-yl)-9H-pyrido[2,3-b]indol-8-yl)(methyl)carbamate_(compound 1.56.3) (60.0 mg, yield: 92.10%) as a yellow solid. MS (ESI):493.2 (M+H)⁺.

### Step (f) Preparation of 4-(1-(aminomethyl)cycloprol)yl)-6-fluoro-3-(2-methoxyprimidin-5-yl)-N-methyl-9H-pyrido[2,3-b]indol-8-amine (Example 4.01)

To a solution of *tert*-butyl (4-(1-(aminomethyl)cyclopropyl)-6-fluoro-3-(2-methoxypyrimidin-5-yl)-9H-pyrido[2,3-b]indol-8-yl)(methyl)carbamate (compound 4.01e) (60.0 mg, 0.122 mmol) in dry DCM (1.5 mL) was added TFA (1.5 mL), and stirred for 1 h at 28 °C. LCMS showed the reaction was completed. The mixture was concentrated to give the crude product, which was purified by HPLC (0.5% TFA as additive) to give 4-(1-(aminomethyl)cyclopropyl)-6-fluoro-3-(2-methoxypyrimidin-5-yl)-N-methyl-9H-pyrido[2,3-b]indol-8-amine (32.0 mg, yield: 66.94%) as a yellow solid. 1H NMR (400 MHz, DMSO-*d6*) δppm: 11.72 (s, 1 H), 8.71 (s, 2 H), 8.23 (s, 1 H), 7.94 (br s, 3 H), 7.37~7.36 (dd, *J*=12.0, 2.0 Hz, 1 H), 6.49~6.53 (dd, *J*=12.4, 2.0 Hz, 1 H), 4.03 (s, 3 H), 3.30~3.84 (m, 2 H), 2.94 (s, 3 H), 1.58 ~1.62 (m, 1 H), 0.87 ~1.06 (m, 2 H), 0.60~0.62 (m, 1 H) MS (ESI): 393.2 (M+H)⁺, 197.2(M/2+H)⁺.

### Example 5.01

### Cis-4-(2-amino-5-azaspiro[2.4]heptan-5-yl)-5,6-difluoro-N-methyl-3-pyrazolo[1,5-a]pyrimidin-6-yl-9H-pyrido[2,3-b]indol-8-amine

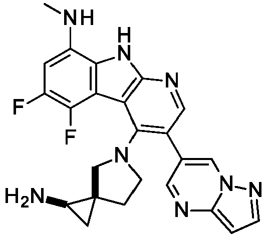

### Step (a) Preparation of cis-tert-butyl N-[4-[2-(tert-butoxycarbonylamino)-5-azaspiro[2.4]heptan 5-yl]-3-chloro-5,6-difluoro-9H-pyrido[2,3-b]indol-8-yl]-N-methyl-carbamate and trans-tert-butyl N-[4-[2-(tert-butoxycarbonylamino)-5-azaspiro[2.4]heptan-5-yl]-3-chloro-5,6-difluoro-9H-pyrido[2,3-b]indol-8-yl]-N-methyl-carbamate

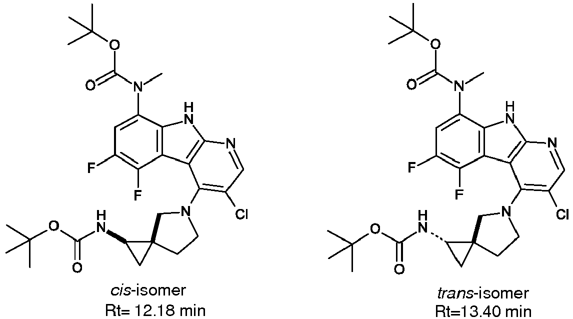

To a solution of *tert*-butyl N-(3,4-dichloro-5,6-difluoro-9H-pyrido[2,3-b]indol-8-yl)-N-methyl-carbamate (778 mg, 1.94 mmol, Intermediate **A5**), *tert*-butyl 5-azaspiro[2.4]heptan-1-ylcarbamate (500 mg, 2.36 mmol) in sulfolane (10 mL) was added DIPEA (750 mg, 5.80 mmol), and the solution was stirred at 100 °C for 2 h. After cooled back to r.t., the mixture was poured into water (100 mL), and extracted with EtOAc (80 mL) two times. Combined organics was dried over anhy. Na₂SO₄, filtered, and concentrated *in vacuo* to give a crude product, which was purified by prep-HPLC (H₂O (0.225% TFA)-ACN) to give *cis-tert-*butyl N-[4-[2-(*tert-*butoxycarbonylamino)-5-azaspiro[2.4]heptan-5-yl]-3-chloro-5,6-difluoro-9H-pyrido[2,3-b]indol-8-yl]-N-methyl-carbamate (**peak 1,** Rt = 12.18 min, 515 mg, 46.0% yield) as a yellow solid and *trans-tert-* butyl N-[4-[2-(*tert*-butoxycarbonylamino)-5-azaspiro[2.4]heptan-5-yl]-3-chloro-5,6-difluoro-9H-pyrido[2,3-b]indol-8-yl]-N-methyl-carbamate (**peak 2,** Rt = 13.40 min, 448 mg, 40.0% yield) as another yellow solid. MS (ESI): 578.1 ([{³⁵Cl}M+H]⁺).

### Step (b) Preparation of cis-tert-butyl N-[4-[2-(tert-butoxycarbonylamino)-5-azaspiro[2.4]heptan-5-yl]-5,6-difluoro-3-pyrazolo[1,5-a]pyrimidin-6-yl-9H-pyrido[2,3-b]indol-8-yl]-N-methyl-carbamate

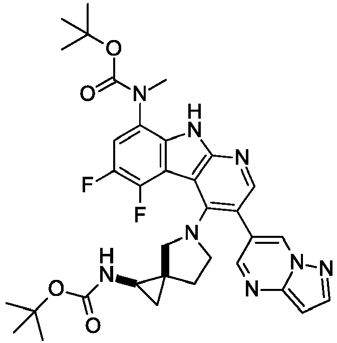

To a solution of *cis-tert-*butyl N-[4-[2-(*tert*-butoxycarbonylamino)-5-azaspiro[2.4]heptan-5-yl]-3-chloro-5,6-difluoro-9H-pyrido[2,3-b]indol-8-yl]-N-methyl-carbamate (50 mg, 0.087 mmol) in dioxane (2 mL) and water (0.5 mL) were added 6-(4,4,5,5-tetramethyl-1,3,2-dioxaborolan-2-yl)pyrazolo[1,5-a]pyrimidine, cataCXium A-Pd-G2 (Sigma-Aldrich, Catalog #: 761311) (12 mg, 0.017 mmol), and K₃PO₄ (55.1 mg, 0.26 mmol) under Argon atmosphere, and the resulting solution was stirred at 70 °C for 16 h. After cooled back to r.t., the reaction mixture was diluted with water (10 mL) and extracted with EtOAc (10 mL) three times. Combined organics was washed with brine (20 mL), dried over anhy. Na₂SO₄, filtered, and concentrated *in vacuo* to give a crude product, which was purified by flash chromatography (DCM/MeOH = 10/1) to give *cis-tert-*butyl N-[4-[2-(tert-butoxycarbonylamino)-5-azaspiro[2.4]heptan-5-yl]-5,6-difluoro-3-pyrazolo[1,5-a]pyrimidin-6-yl-9H-pyrido[2,3-b]indol-8-yl]-N-methyl-carbamate (50 mg, 87.5% yield) as a yellow solid. MS (ESI): 661.6 [{³⁵Cl} (M+H)⁺].

### Step (c) Preparation of cis-4-(2-amino-5-azaspiro[2.4]heptan-5-yl)-5,6-difluoro-N-methyl-3-pyrazolo[1,5-a]pyrimidin-6-yl-9H-pyrido[2,3-b]indol-8-amine

To a solution of *cis-tert-*butyl N-[4-[2-(tert-butoxycarbonylamino)-5-azaspiro[2.4]heptan-5-yl]-5,6-difluoro-3-pyrazolo[1,5-a]pyrimidin-6-yl-9H-pyrido[2,3-b]indol-8-yl]-N-methyl-carbamate (40 mg, 0.06 mmol) in DCM (0.5 mL) was added TFA (0.07 mL), and the solution was stirred at 20 °C for 1 h. The reaction mixture was concentrated in vacuo to give a crude product, which was purified by prep-HPLC (water (1% TFA)-ACN) to give *cis*-4-(2-amino-5-azaspiro[2.4]heptan-5-yl)-5,6-difluoro-N-methyl-3-pyrazolo[1,5-a]pyrimidin-6-yl-9H-pyrido[2,3-b]indol-8-amine (11.5 mg, 41.1% yield) as a green solid. MS (ESI): 461.0 [M+H]⁺. ¹H NMR (MeOH-*d4*, 400 MHz) δ 9.09 (d, 1H, J=1.5 Hz), 8.66 (d, 1H, J=2.1 Hz), 8.2-8.4 (m, 2H), 6.83 (d, 1H, J=2.2 Hz), 6.67 (m, 1H), 3.5-3.8 (m, 2H), 3.3-3.5 (m, 1H), 3.2-3.3 (m, 1H), 3.00 (s, 3H), 2.66 (m, 1H), 2.0-2.2 (m, 2H), 1.0-1.1 (m, 1H), 0.98 (m, 1H)

### Example 5.02

### Trans-4-(2-Amino-5-azaspiro[2.4]heptan-5-yl)-5,6-difluoro-N-methyl-3-pyrazolo[1,5-a]pyrimidin-6-yl-9H-pyrido[2,3-b]indol-8-amine

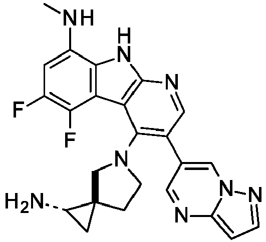

The title compound was prepared in analogy to **Example 5.01** by replacing *cis-tert-*butyl N-[4-[2-(*tert*-butoxycarbonylamino)-5-azaspiro[2.4]heptan-5-yl]-3-chloro-5,6-difluoro-9H-pyrido[2,3-b]indol-8-yl]-N-methyl-carbamate with *trans-tert-*butyl N-[4-[2-(*tert-*butoxycarbonylamino)-5-azaspiro[2.4]heptan-5-yl]-3-chloro-5,6-difluoro-9H-pyrido[2,3-b]indol-8-yl]-N-methyl-carbamate in step (b). MS (ESI): 461.0 [M+H]⁺. ¹H NMR (MeOH-*d4*, 400 MHz) δ 9.07 (d, 1H, J=1.2 Hz), 8.66 (d, 1H, J=2.1 Hz), 8.2-8.3 (m, 2H), 6.83 (d, 1H, J=2.3 Hz), 6.65 (m, 1H), 3.4-3.6 (m, 4H), 3.0 (s, 3H), 2.75 (dd, 1H, J=4.3, 7.8 Hz), 2.1-2.2 (m, 1H), 1.8-1.9 (m, 1H), 1.21 (t, 1H, J=7.3 Hz), 0.8-0.9 (m, 1H).

### Example 6.01

### 4-[(2R,3R)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine

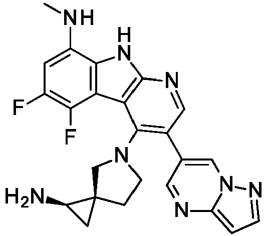

### Step (a) Preparation of tert-butyl N-[4-[(2R,3R)-2-(tert-butoxycarbonylamino)-5-azaspiro[2.4]heptan-5-yl]-3-chloro-5,6-difluoro-9H-pyrido[2,3-b]indol-8-yl]-N-methylcarbamate and tert-butyl N-[4-[(2S,3S)-2-(tert-butoxycarbonylamino)-5-azaspiro[2.4]heptan-5-yl]-3-chloro-5,6-difluoro-9H-pyrido[2,3-b]indol-8-yl]-N-methyl-carbamate and tert-butyl N-[4-[(2S,3R)-2-(tert-butoxycarbonylamino)-5-azaspiro[2.4]heptan-5-yl]-3-chloro-5,6-difluoro-9H-pyrido[2,3-b]indol-8-yl]-N-methyl-carbamate and tert-butyl N-[4-[(2R,3S)-2-(tert-butoxycarbonylamino)-5-azaspiro[2.4]heptan-5-yl]-3-chloro-5,6-difluoro-9H-pyrido[2,3-b]indol-8-yl]-N-methyl-carbamate

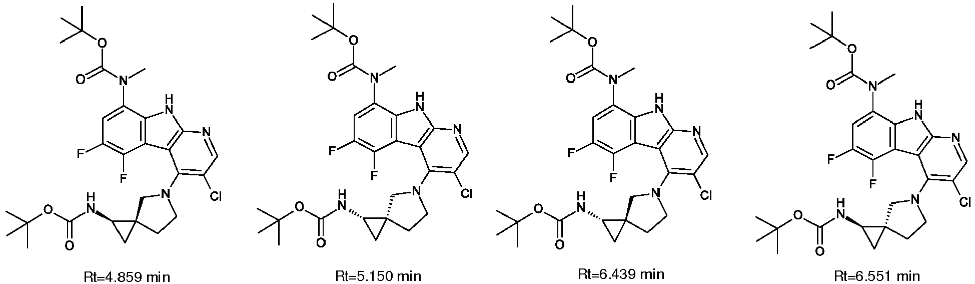

*Cis-tert-*butyl N-[4-[2-(*tert*-butoxycarbonylamino)-5-azaspiro[2.4]heptan-5-yl]-3-chloro-5,6-difluoro-9H-pyrido[2,3-b]indol-8-yl]-N-methyl-carbamate (289 mg, 0.5 mmol) was subject to chiral SFC separation. The first collected eluent peak was concentrated *in vacuo* to give *tert-*butyl N-[4-[(2R,3R)-2-(*tert*-butoxycarbonylamino)-5-azaspiro[2.4]heptan-5-yl]-3-chloro-5,6-difluoro-9H-pyrido[2,3-b]indol-8-yl]-N-methyl-carbamate (**peak 1',** Rt = 4.859 min, 120 mg) as a yellow solid and the second collected eluent peak was concentrated *in vacuo* to give *tert*-butyl N-[4-[(2S,3S)-2-(*tert*-butoxycarbonylamino)-5-azaspiro[2.4]heptan-5-yl]-3-chloro-5,6-difluoro-9H-pyrido[2,3-b]indol-8-yl]-N-methyl-carbamate (**peak 2',** Rt =5.150 min, 130 mg) as another yellow solid. SFC condition: Column: ChiralPak AD (150×4.6mm, 3µm); Mobile Phase: A for CO2 and B for Isopropanol (0.05%DEA); Flow Rate (mL/min): 2.5 mL/min.

*Trans-tert-* butyl N-[4-[2-(tert-butoxycarbonylamino)-5-azaspiro[2.4]heptan-5-yl]-3-chloro-5,6-difluoro-9H-pyrido[2,3-b]indol-8-yl]-N-methyl-carbamate (289 mg, 0.5 mmol) was subject to chiral SFC separation. The first collected eluent peak was concentrated *in vacuo* to give *tert*-butyl N-[4-[(2S,3R)-2-(*tert*-butoxycarbonylamino)-5-azaspiro[2.4]heptan-5-yl]-3-chloro-5,6-difluoro-9H-pyrido[2,3-b]indol-8-yl]-N-methyl-carbamate (**peak 3**', Rt = 6.439 min, 120 mg) as a yellow solid and the second collected eluent peak was concentrated *in vacuo* to give *tert*-butyl N-[4-[(2R,3S)-2-(*tert*-butoxycarbonylamino)-5-azaspiro[2.4]heptan-5-yl]-3-chloro-5,6-difluoro-9H-pyrido[2,3-b]indol-8-yl]-N-methyl-carbamate- (**peak 4',** Rt = 6.551 min, 140 mg) as another yellow solid. SFC condition: Column: ChiralPak AD (150×4.6mm, 3µm); Mobile Phase: A for CO2 and B for Isopropanol (0.05%DEA); Flow Rate (mL/min): 2.5 mL/min.

### Step (b) Preparation of tert-butyl N-[4-[(2R,3R)-2-(tert-butoxycarbonylamino)-5-azaspiro[2.4]heptan-5-yl]-5,6-difluoro-3-pyrazolo[1,5-a]pyrimidin-6-yl-9H-pyrido[2,3-b]indol-8-yl]-N-methyl-carbamate

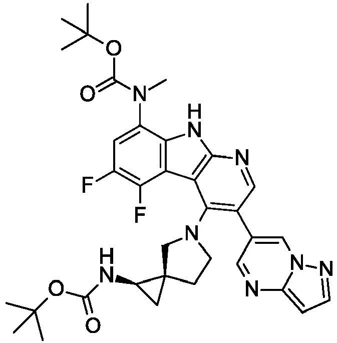

To a solution of *tert*-butyl N-[4-[(2R,3R)-2-(*tert*-butoxycarbonylamino)-5-azaspiro[2.4]heptan-5-yl]-3-chloro-5,6-difluoro-9H-pyrido[2,3-b]indol-8-yl]-N-methylcarbamate (50 mg, 0.087 mmol) in dioxane (2 mL) and water (0.5 mL) were added 6-(4,4,5,5-tetramethyl-1,3,2-dioxaborolan-2-yl)pyrazolo[1,5-a]pyrimidine, cataCXium A-Pd-G2 (Sigma-Aldrich, Catalog #: 761311) (12 mg, 0.017 mmol), and K₃PO₄ (55.1 mg, 0.26 mmol) under Argon atmosphere, and the resulting solution was stirred at 70 °C for 16 h. After cooled back to r.t., the reaction mixture was diluted with water (10 mL) and extracted with EtOAc (10 mL) three times. Combined organics was washed with brine (20 mL), dried over anhy. Na₂SO₄, filtered, and concentrated *in vacuo* to give a crude product, which was purified by flash chromatography (DCM/MeOH = 10/1) to give *tert*-butyl N-[4-[(2R,3R)-2-(*tert-*butoxycarbonylamino)-5-azaspiro[2.4]heptan-5-yl]-5,6-difluoro-3-pyrazolo[1,5-a]pyrimidin-6-yl-9H-pyrido[2,3-b]indol-8-yl]-N-methyl-carbamate (50 mg, 87.5% yield) as a yellow solid. MS (ESI): 661.6 [{³⁵Cl}(M+H)⁺],

### Step (c) Preparation of 4-[(2R,3R)-2-amino-5-azaspiro[2.4]heptan-5-yl]-5,6-difluoro-N-methyl-3-pyrazolo[1,5-a]pyrimidin-6-yl-9H-pyrido[2,3-b]indol-8-amine

To a solution of *tert*-butyl N-[4-[(2R,3R)-2-(*tert*-butoxycarbonylamino)-5-azaspiro[2.4]heptan-5-yl]-5,6-difluoro-3-pyrazolo[1,5-a]pyrimidin-6-yl-9H-pyrido[2,3-b]indol-8-yl]-N-methyl-carbamate (40 mg, 0.06 mmol) in DCM (0.5 mL) was added TFA (0.07 mL), and the solution was stirred at 20 °C for 1 h. The reaction mixture was concentrated *in vacuo* to give a crude product, which was purified by Prep-HPLC (water (1% TFA)-ACN) to give 4-[(2R,3R)-2-amino-5-azaspiro[2.4]heptan-5-yl]-5,6-difluoro-N-methyl-3-pyrazolo[1,5-a]pyrimidin-6-yl-9H-pyrido[2,3-b]indol-8-amine (11.5 mg, 41.1% yield) as a green solid. MS (ESI): 461.0 [M+H]⁺. ¹H NMR (MeOH-*d4*, 400 MHz) δ 9.1-9.1 (m, 1H), 8.68 (d, 1H, J=2.2 Hz), 8.28 (d, 1H, J=2.4 Hz), 8.27 (s, 1H), 6.83 (d, 1H, J=2.3 Hz), 6.66 (dd, 1H, J=6.4, 13.3 Hz), 3.6-3.7 (m, 3H), 3.3-3.6 (m, 1H), 3.00 (s, 3H), 2.74 (dd, 1H, J=4.3, 7.9 Hz), 2.0-2.1 (m, 1H), 1.93 (dd, 1H, J=5.7, 12.0 Hz), 1.21 (t, 1H, J=7.4 Hz), 0.86 (dd, 1H, J=4.4, 6.8 Hz).

### Example 6.02

### 4-[(2S,3S)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine

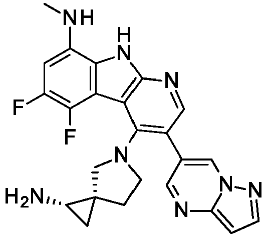

The title compound was prepared in analogy to Example 6.01 by replacing *tert*-butyl N-[4-[(2R,3R)-2-(*tert*-butoxycarbonylamino)-5-azaspiro[2.4]heptan-5-yl]-3-chloro-5,6-difluoro-9H-pyrido[2,3-b]indol-8-yl]-N-methyl-carbamate with *tert*-butyl N-[4-[(2S,3S)-2-(*tert-*butoxycarbonylamino)-5-azaspiro[2.4]heptan-5-yl]-3-chloro-5,6-difluoro-9H-pyrido[2,3-b]indol-8-yl]-N-methyl-carbamate in step (b). MS (ESI): 461.0 [M+H]⁺. ¹H NMR (METHANOL-d4, 400 MHz) δ 9.10 (dd, 1H, J=0.9, 2.1 Hz), 8.67 (d, 1H, J=2.1 Hz), 8.29 (s, 1H), 8.28 (d, 1H, J=2.4 Hz), 6.83 (dd, 1H, J=0.9, 2.4 Hz), 6.66 (dd, 1H, J=6.4, 13.2 Hz), 3.5-3.7 (m, 4H), 3.00 (s, 3H), 2.75 (dd, 1H, J=4.4, 7.9 Hz), 2.1-2.2 (m, 1H), 1.9-2.0 (m, 1H), 1.21 (t, 1H, J=7.4 Hz), 0.85 (dd, 1H, J=4.6, 6.7 Hz).

### Example 6.03

### 4-[(2S,3R)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine

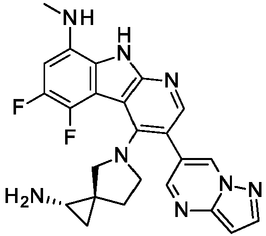

The title compound was prepared in analogy to **Example 6.01** by replacing *tert*-butyl N-[4-[(2R,3R)-2-(tert-butoxycarbonylamino)-5-azaspiro[2.4]heptan-5-yl]-3-chloro-5,6-difluoro-9H-pyrido[2,3-b]indol-8-yl]-N-methyl-carbamate with *tert*-butyl N-[4-[(2S,3R)-2-(*tert-*butoxycarbonylamino)-5-azaspiro[2.4]heptan-5-yl]-3-chloro-5,6-difluoro-9H-pyrido[2,3-b]indol-8-yl]-N-methyl-carbamate in step (b). MS (ESI): 461.0 [M+H]⁺. ¹H NMR (MeOH-*d4*, 400 MHz) δ 9.0-9.0 (m, 1H), 8.54 (d, 1H, J=2.2 Hz), 8.19 (s, 1H), 8.15 (d, 1H, J=2.3 Hz), 6.70 (d, 1H, J=2.4 Hz), 6.53 (dd, 1H, J=6.4, 13.1 Hz), 3.4-3.6 (m, 3H), 3.3-3.4 (m, 1H), 3.1-3.2 (m, 1H), 2.9 (s, 3H), 2.53 (dd, 1H, J=4.4, 7.9 Hz), 2.0-2.1 (m, 1H), 1.9-2.0 (m, 1H), 0.96 (t, 1H, J=7.4 Hz), 0.86 (dd, 1H, J=4.5, 6.7 Hz).

### Example 6.04

### 4-[(2R,3S)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine

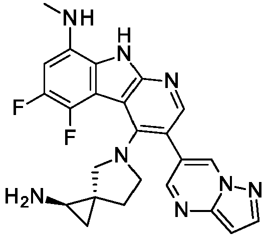

The title compound was prepared in analogy to **Example 6.01** by replacing *tert*-butyl N-[4-[(2R,3R)-2-(*tert*-butoxycarbonylamino)-5-azaspiro[2.4]heptan-5-yl]-3-chloro-5,6-difluoro-9H-pyrido[2,3-b]indol-8-yl]-N-methyl-carbamate with *tert*-butyl N-[4-[(2R,3S)-2-(*tert-*butoxycarbonylamino)-5-azaspiro[2.4]heptan-5-yl]-3-chloro-5,6-difluoro-9H-pyrido[2,3-b]indol-8-yl]-N-methyl-carbamate in step (b). MS (ESI): 461.0 [M+H]⁺. ¹H NMR (MeOH-*d4*, 400 MHz) δ 8.97 (dd, 1H, J=0.9, 2.1 Hz), 8.54 (d, 1H, J=2.1 Hz), 8.1-8.2 (m, 2H), 6.70 (dd, 1H, J=0.9, 2.4 Hz), 6.51 (m, 1H), 3.5-3.6 (m, 2H), 3.3-3.4 (m, 1H), 3.1-3.2 (m, 1H), 2.88 (s, 3H), 2.51 (m, 1H), 1.9-2.1 (m, 2H), 0.9-1.1 (m, 1H), 0.86 (m, 1H).

### BIOLOGICAL EXAMPLES

### Example 7

### 50% Growth Inhibitory Concentration (IC₅₀) Determination Assay:

The in vitro antimicrobial activity of the compounds against *S. aureus* (ATCC29213), *K*. *pneumoniae* (ATCC10031), and A. *baumannii* (ATCC17978), was determined according to the following procedure:

The assay used a 10-points Iso-Sensitest broth medium to measure quantitatively the in vitro activity of the compounds against *S. aureus* ATCC29213, *K. pneumoniae* ATCC 10031, and *A*. *baumannii* ATCC17978.

Stock compounds in DMSO were serially two-fold diluted (range from 50 to 0.097 µM final concentration) in 384 wells microtiter plates and inoculated with 49 µL the bacterial suspension in Iso-Sensitest broth medium to have a final cell concentration of ~ 5×10⁵ CFU/mL in a final volume/well of 50 µL/well. Microtiter plates were incubated at 35 ± 2 °C.

Bacterial cell growth was determined with the measurement of optical density at λ=600nm each 20 minutes over a time course of 16h.

Growth inhibition was calculated during the logarithmic growth of the bacterial cells with determination of the concentration inhibiting 50% (IC₅₀) of the growth.

GP-6 (Example 4.131 disclosed in WO 2012/125746 A1) was used as the reference compound in Table 6-8.

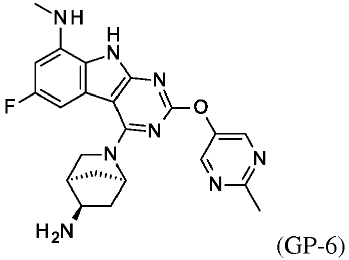

Compounds of the present invention were tested for their concentration inhibiting 50% (IC₅₀). The data of IC₅₀ over *S. aureus* (ATCC29213), *K. pneumoniae* (ATCC10031), and A. *baumannii* (ATCC17978) are illustrated in Table 4. Particular compounds of the present invention were found to have IC₅₀ ≤ 1 µM.

**Table 4: IC₅₀ values of the compounds of this invention against S. aureus, K. pneumoniae and A. baumannii**

| **Example No.** | **IC50 (µM)** | | |
|---|---|---|---|
| | *S. aureus* ATCC29213 | *K. pneumoniae* ATCC10031 | *A. baumannii* ATCC17978 |
| **GP-6** | <0.098^{∗} | <0.098^{∗} | <0.098^{∗} |
| **1.04** | 0.380 | <0.098^{∗} | 0.212 |
| **1.05** | 1.791 | <0.098^{∗} | <0.098^{∗} |
| **1.07** | 1.090 | <0.098^{∗} | 0.110 |
| **1.08** | 0.789 | <0.098^{∗} | 0.243 |
| **1.09** | 0.238 | <0.098^{∗} | 0.147 |
| **1.10** | 0.312 | <0.098^{∗} | <0.098^{∗} |
| **1.11** | 0.863 | <0.098^{∗} | <0.098^{∗} |
| **1.13** | 1.500 | <0.098^{∗} | 0.764 |
| **1.14** | 1.211 | <0.098^{∗} | <0.098^{∗} |
| **1.15** | 0.526 | <0.098^{∗} | 0.672 |
| **1.16** | 0.867 | <0.098^{∗} | 0.104 |
| **1.18** | 0.567 | <0.098^{∗} | 0.249 |
| **1.19** | 0.712 | <0.098^{∗} | 0.683 |
| **1.20** | 1.270 | <0.098^{∗} | <0.098^{∗} |
| **1.22** | 0.224 | <0.098^{∗} | 0.112 |
| **1.27** | 0.522 | <0.098^{∗} | 0.340 |
| **1.28** | 0.227 | <0.098^{∗} | 0.115 |
| **1.29** | 1.238 | N.A. | 0.100 |
| **1.30** | 1.015 | N.A. | <0.098^{∗} |
| **1.32** | 1.240 | <0.098^{∗} | 0.472 |
| **1.34** | 0.603 | <0.098^{∗} | 0.157 |
| **1.35** | 0.184 | <0.098^{∗} | <0.098^{∗} |
| **1.45** | 0.992 | 0.172 | 0.528 |
| **1.46** | 0.206 | <0.098^{∗} | 0.387 |
| **1.47** | 0.275 | <0.098^{∗} | 0.443 |
| **1.49** | 1.315 | <0.098^{∗} | 0.143 |
| **1.50** | 0.369 | <0.098^{∗} | <0.098^{∗} |
| **1.51** | 1.037 | <0.098^{∗} | <0.098^{∗} |
| **1.52** | 0.993 | <0.098^{∗} | <0.098^{∗} |
| **1.53** | 0.394 | <0.098^{∗} | <0.098^{∗} |
| **1.54** | 0.477 | <0.098^{∗} | <0.098^{∗} |
| **1.57** | 0.286 | <0.098^{∗} | 0.314 |
| **1.58** | 0.231 | N.A. | 0.182 |
| **1.59** | 0.631 | <0.098^{∗} | 0.499 |
| **1.60** | 0.842 | N.A. | N.A. |
| **1.61** | 0.650 | <0.098^{∗} | 0.792 |
| **1.62** | 1.378 | 0.238 | <0.098^{∗} |
| **2.02** | 0.474 | 0.391 | <0.098^{∗} |
| **2.03** | 1.111 | 0.189 | <0.098^{∗} |
| **3.03** | 0.559 | <0.098^{∗} | 0.411 |
| **3.04** | 0.649 | <0.098^{∗} | 0.485 |
| **4.01** | 0.631 | <0.098^{∗} | 0.847 |

| | | | |
|---|---|---|---|
| "^{∗}": the detection limit. | | | |

### Example 8

### Lyophilisation Solubility Assay (LYSA):

The solubility of compounds of present invention was assessed by LYSA assay. Samples were prepared in duplicate from 10 mM DMSO stock solutions (20µL) diluted in 30µL pure DMSO. After evaporation of DMSO with a centrifugal vacuum evaporator, the residue was dissolved in 0.05 M phosphate buffer (150µL, pH 6.5), which was stirred for one hour and shook for two hours. After one night, the solution was filtered using a microtiter filter plate. Then the filtrate and its 1/10 dilution were analyzed by HPLC-UV. In addition, a four-point calibration curve was prepared from the 10 mM stock solutions and used for the solubility determination of the compounds. The results were in µg/mL and summarized in Table 5. Particular compounds of the present invention were found to have LYSA > 10 µg/mL with much improved solubility compared to GP-6.

**Table 5: Solubility data of Examples**

| **Example No.** | **Lysa (µg/mL)** | **Example No.** | **Lysa (µg/mL)** |
|---|---|---|---|
| **GP-6** | <1.0^{∗} | **1.33** | 475 |
| **1.01** | 59 | **1.34** | 93 |
| **1.03** | 32.7 | **1.35** | 90 |
| **1.04** | 145 | **1.36** | 140 |
| **1.05** | 28 | **1.37** | 545 |
| **1.06** | >625 | **1.38** | >610 |
| **1.07** | 39 | **1.40** | 49 |
| **1.08** | 83 | **1.44** | 270 |
| **1.09** | 190 | **1.46** | 17 |
| **1.13** | 46 | **1.47** | 19 |
| **1.14** | 10 | **1.53** | 241 |
| **1.15** | 169 | **1.54** | 39 |
| **1.16** | 32 | **1.57** | 91 |
| **1.18** | 195 | **1.58** | 320 |
| **1.19** | 31 | **1.59** | 395 |
| **1.20** | 124 | **1.60** | 53 |
| **1.21** | 44 | **1.61** | 62 |
| **1.22** | 11 | **2.01** | 129 |
| **1.23** | 30 | **2.02** | 160 |
| **1.25** | 194 | **2.03** | 410 |
| **1.27** | 74 | **3.02** | 196 |
| **1.28** | 88 | **3.03** | 125 |
| **1.29** | 16 | **3.04** | 16 |
| **1.32** | 10 | **4.01** | >575 |

| | | | |
|---|---|---|---|
| "^{∗}": the detection limit. | | | |

## Claims

1. A compound of formula (I), 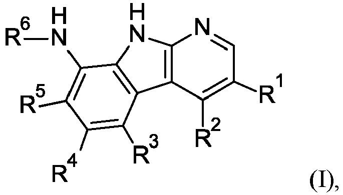 wherein
R¹ is oxopyridinyl; pyrazolo[1,5-a]pyrimidinyl; pyridinyl substituted by cyano, C₁₋₆alkoxycarbonyl or C₁₋₆alkylaminocarbonylC₁₋₆alkyl; pyrimidinyl substituted by C₁₋₆alkoxy or carboxyC₃₋₇cycloalkyl;
R² is (aminoC₁₋₆alkyl)C₃₋₇cycloalkyl;
(C₁₋₆alkyl)₂amino;
2,3,3a,4,6,6a-hexahydropyrrolo[2,3-c]pyrrolyl substituted by C₁₋₆alkyl;
3,6-diazabicyclo[3 .1.1]heptanyl;
azabicyclo[3.1.0]hexanyl substituted by aminoC₁₋₆alkyl;
azaspiro[2.4]heptanyl substituted by amino;
azaspiro[3.3]heptanyl substituted by amino;
azaspiro[3.4]octanyl substituted by amino;
diazabicyclo[3. 1. 1]heptanyl;
morpholinyl substituted by aminoC₁₋₆alkyl;
piperidinyl substituted once or twice by substituents independently selected from amino, aminoC₁₋₆alkyl, C₁₋₆alkyl and halogen; or
pyrrolidinyl substituted once, twice or three times by substituents independently selected from amino, aminoC₁₋₆alkyl, C₁₋₆alkyl and halogen;
R³ is H, C₁₋₆alkyl or halogen;
R⁴ is halogen or haloC₁₋₆alkyl;
R⁵ is H or halogen;
R⁶ is C₁₋₆alkyl;
or pharmaceutically acceptable salt thereof.

2. A compound according to claim 1, wherein
R¹ is oxopyridinyl; pyrazolo[1,5-a]pyrimidinyl; cyanopyridinyl; methoxycarbonylpyridinyl; methylaminocarbonylmethylpyridinyl; ethoxypyrimidinyl; methoxypyrimidinyl or carboxycyclopropylpyrimidinyl;
R² is (aminomethyl)azabicyclo[3.1.0]hexanyl; (aminomethyl)cyclopropyl; (aminomethyl)morpholinyl; (aminomethyl)piperidinyl; (aminomethyl)pyrrolidinyl; 3,6-diazabicyclo[3.1.1]heptanyl; aminoazaspiro[2.4]heptanyl; aminoazaspiro[3.3]heptanyl; aminoazaspiro[3.4]octanyl; aminopiperidinyl; aminopyrrolidinyl; diazabicyclo[3.1.1]heptanyl; difluoro(aminomethyl)pyrrolidinyl; dimethylamino; fluoro(aminomethyl)piperidinyl; fluoro(aminomethyl)pyrrolidinyl; methyl(aminomethyl)piperidinyl; methyl(aminomethyl)pyrrolidinyl or methyl-2,3,3a,4,6,6a-hexahydropyrrolo[2,3-c]pyrrolyl;
R³ is H, methyl or fluoro;
R⁴ is chloro, fluoro or trifluoromethyl;
R⁵ is H or fluoro;
R⁶ is ethyl or methyl;
or pharmaceutically acceptable salt thereof.

3. A compound according to claim 1 , or pharmaceutically acceptable salt thereof, wherein R¹ is cyanopyridinyl or C₁₋₆alkoxypyrimidinyl.

4. A compound according to claim 3, or pharmaceutically acceptable salt thereof, wherein R¹ is cyanopyridinyl or methoxypyrimidinyl.

5. A compound according to claim 3 or 4, or pharmaceutically acceptable salt thereof, wherein R² is (C₁₋₆alkyl)₂amino; aminoazaspiro[2.4]heptanyl; piperidinyl substituted once or twice by substituents independently selected from amino and aminoC₁₋₆alkyl; or pyrrolidinyl substituted once or twice by substituents independently selected from amino and aminoC₁₋₆alkyl.

6. A compound according to claim 5, or pharmaceutically acceptable salt thereof, wherein R² is dimethylamino, aminopiperidinyl, (aminomethyl)piperidinyl, methyl(aminomethyl)piperidinyl, aminopyrrolidinyl, (aminomethyl)pyrrolidinyl or methyl(aminomethyl)pyrrolidinyl.

7. A compound according to claim 5, wherein
R¹ is cyanopyridinyl or C₁₋₆alkoxypyrimidinyl;
R² is (C₁₋₆alkyl)₂amino; aminoazaspiro[2.4]heptanyl; piperidinyl substituted once or twice by substituents independently selected from amino and aminoC₁₋₆alkyl; or pyrrolidinyl substituted once or twice by substituents independently selected from amino and aminoC₁₋₆alkyl;
R³ is halogen;
R⁴ is halogen;
R⁵ is H;
R⁶ is C₁₋₆alkyl;
or pharmaceutically acceptable salt thereof.

8. A compound according to claim 7, wherein
R¹ is cyanopyridinyl or methoxypyrimidinyl;
R² is dimethylamino, aminopiperidinyl, (aminomethyl)piperidinyl, methyl(aminomethyl)piperidinyl, aminopyrrolidinyl, (aminomethyl)pyrrolidinyl or methyl(aminomethyl)pyrrolidinyl;
R³ is fluoro;
R⁴ is fluoro or chloro;
R⁵ is H;
R⁶ is methyl or ethyl;
or pharmaceutically acceptable salt thereof.

9. A compound according to claims 1 to 8 selected from:
5-[4-[(3R)-3-Aminopyrrolidin-1-yl]-6-fluoro-8-(methylamino)-9H-pyrido[2,3-b]indol-3-yl]pyridine-3-carbonitrile;
5-[4-(3-Aminopyrrolidin-1-yl)-8-(ethylamino)-5,6-difluoro-9H-pyrido[2,3-b]indol-3-yl]pyridine-3-carbonitrile;
5-[4-(3-Aminopyrrolidin-l-yl)-6-chloro-8-(ethylamino)-9H-pyrido[2,3-b]indol-3-yl]pyridine-3-carbonitrile;
5-[4-[(3*R*)-3-aminopyrrolidin-1-yl]-8-(ethylamino)-5,6-difluoro-9H-pyrido[2,3-b]indol-3-yl]pyridine-3-carbonitrile;
5-[4-[(3*R*)-3-aminopyrrolidin-1-yl]-6-chloro-8-(ethylamino)-9H-pyrido[2,3-b]indol-3 -yl]pyri dine-3 -carbonitrile;
5-[4-[2-(Aminomethyl)pyrrolidin-1-yl]-8-(ethylamino)-6-fluoro-9H-pyrido[2,3-b]indol-3-yl]pyridine-3-carbonitrile;
5-[4-[3-(Aminomethyl)pyrrolidin-1-yl]-8-(ethylamino)-5,6-difluoro-9H-pyrido[2,3-b]indol-3-yl]pyridine-3-carbonitrile;
4-[3-(aminomethyl)pyrrolidin-1-yl]-N-ethyl-5,6-difluoro-3-(2-methoxypyrimidin-5-yl)-9H-pyrido[2,3 -b]indol-8-amine;
4-(3-Aminopyrrolidin-1-yl)-N-ethyl-5,6-difluoro-3-pyrazolo[1,5-a]pyrimidin-6-yl-9H-pyrido[2,3 -b]indol-8-amine;
5-[4-[3-(aminomethyl)-3-fluoro-pyrrolidin-1-yl]-8-(ethylamino)-6-fluoro-9H-pyrido[2,3-b]indol-3-yl]pyridine-3-carbonitrile;
5-[8-(ethylamino)-6-fluoro-4-[*cis*-2-amino-5-azaspiro[2.4]heptan-5-yl]-9H-pyrido[2,3-b]indol-3-yl]pyridine-3-carbonitrile;
5-[8-(ethylamino)-6-fluoro-4-[*trans*-2-amino-5-azaspiro[2.4]heptan-5-yl]-9H-pyrido[2,3-b]indol-3-yl]pyridine-3-carbonitrile;
5-[4-[3-(Aminomethyl)pyrrolidin-1-yl]-5,6-difluoro-8-(methylamino)-9H-pyrido[2,3-b]indol-3-yl]pyridine-3-carbonitrile;
5-[4-[3-(Aminomethyl)-1-piperidyl]-8-(ethylamino)-6-fluoro-9H-pyrido[2,3-b]indol-3 -yl] pyri dine-3 -carbonitrile;
5-[4-[(3*R*)-3-Aminopyrrolidin-1-yl]-5,6-difluoro-8-(methylamino)-9H-pyrido[2,3-b]indol-3-yl]pyridine-3-carbonitrile;
5-[6-fluoro-8-(methylamino)-4-[*cis*-2-amino-5-azaspiro[2.4]heptan-5-yl]-9H-pyrido[2,3-b]indol-3-yl]pyridine-3-carbonitrile;
5-[6-fluoro-8-(methylamino)-4-[*trans*-2-amino-5-azaspiro[2.4]heptan-5-yl]-9H-pyrido[2,3-b]indol-3-yl]pyridine-3-carbonitrile;
4-[(3*R*)-3-Aminopyrrolidin-1-yl]-N-ethyl-5,6-difluoro-3-(2-methoxypyrimidin-5-yl)-9H-pyrido[2,3 -b]indol-8-amine;
4-[3-(Aminomethyl)pyrrolidin-1-yl]-5,6-difluoro-3-(2-methoxypyrimidin-5-yl)-N-methyl-9H-pyrido[2,3-b]indol-8-amine;
5-[4-(3-Aminopyrrolidin-1-yl)-8-(ethylamino)-6-(trifluoromethyl)-9H-pyrido[2,3-b]indol-3-yl]pyridine-3-carbonitrile;
5-[4-[3-(Aminomethyl)-1-piperidyl]-5,6-difluoro-8-(methylamino)-9H-pyrido[2,3-b]indol-3-yl]pyridine-3-carbonitrile;
5-[4-[3-(Aminomethyl)-3-fluoro-pyrrolidin-1-yl]-5,6-difluoro-8-(methylamino)-9H-pyrido[2,3-b]indol-3-yl]pyridine-3-carbonitrile;
5-[4-[3-(Aminomethyl)-3-fluoro-pyrrolidin-1-yl]-6-fluoro-8-(methylamino)-9H-pyrido[2,3-b]indol-3-yl]pyridine-3-carbonitrile;
4-[3-(aminomethyl)-3-fluoro-pyrrolidin-1-yl]-6-fluoro-3-(2-methoxypyrimidin-5-yl)-N-methyl-9H-pyrido[2,3-b]indol-8-amine;
4-[(3*S*)-3-(aminomethyl)-1-piperidyl]-5,6-difluoro-3-(2-methoxypyrimidin-5-yl)-N-methyl-9H-pyrido[2,3-b]indol-8-amine;
4-[3-(aminomethyl)-3-methyl-1-piperidyl]-5,6-difluoro-3-(2-methoxypyrimidin-5-yl)-N-methyl-9H-pyrido[2,3-b]indol-8-amine;
4-[1-(aminomethyl)-3-azabicyclo[3.1.0]hexan-3-yl]-5,6-difluoro-3-(2-methoxypyrimidin-5-yl)-N-methyl-9H-pyrido[2,3-b]indol-8-amine;
4-[3-(aminomethyl)-3-fluoro-pyrrolidin-1-yl]-5,6-difluoro-3-(2-methoxypyrimidin-5-yl)-N-methyl-9H-pyrido[2,3-b]indol-8-amine;
5-[4-[*trans*-2-amino-5-azaspiro[2.4]heptan-5-yl]-5,6-difluoro-8-(methylamino)-9H-pyrido[2,3-b]indol-3-yl]pyridine-3-carbonitrile;
5-[4-[*cis*-2-amino-5-azaspiro[2.4]heptan-5-yl]-5,6-difluoro-8-(methylamino)-9H-pyrido[2,3-b]indol-3-yl]pyridine-3-carbonitrile;
5-[4-[2-(aminomethyl)morpholin-4-yl]-5,6-difluoro-8-(methylamino)-9H-pyrido[2,3-b]indol-3 -yl]pyridine-3 -carbonitrile;
5-[4-[1-(aminomethyl)-3-azabicyclo[3.1.0]hexan-3-yl]-5,6-difluoro-8-(methylamino)-9H-pyrido[2,3-b]indol-3-yl]pyridine-3-carbonitrile;
4-[2-(aminomethyl)morpholin-4-yl]-5,6-difluoro-3-(2-methoxypyrimidin-5-yl)-N-methyl-9H-pyrido[2,3-b]indol-8-amine;
4-[*cis*-2-amino-5-azaspiro[2.4]heptan-5-yl]-5,6-difluoro-3-(2-methoxypyrimidin-5-yl)-N-methyl-9H-pyrido[2,3-b]indol-8-amine;
4-[*trans*-2-amino-5-azaspiro[2.4]heptan-5-yl]-5,6-difluoro-3-(2-methoxypyrimidin-5-yl)-N-methyl-9H-pyrido[2,3-b]indol-8-amine;
4-[3-(aminomethyl)-3-methyl-pyrrolidin-1-yl]-5,6-difluoro-3-(2-methoxypyrimidin-5-yl)-N-methyl-9H-pyrido[2,3-b]indol-8-amine;
4-[2-(aminomethyl)pyrrolidin-1-yl]-5,6-difluoro-3-(2-methoxypyrimidin-5-yl)-N-methyl-9H-pyrido[2,3-b]indol-8-amine;
5-[4-[2-(aminomethyl)pyrrolidin-1-yl]-5,6-difluoro-8-(methylamino)-9H-pyrido[2,3-b]indol-3-yl]pyridine-3-carbonitrile;
5-[4-[(3*S*)-3-(aminomethyl)-1-piperidyl]-5,6-difluoro-8-(methylamino)-9H-pyrido[2,3-b]indol-3-yl]pyridine-3-carbonitrile;
Methyl 5-[4-[(3*S*)-3-(aminomethyl)-1-piperidyl]-5,6-difluoro-8-(methylamino)-9H-pyrido[2,3-b]indol-3-yl]pyridine-3-carboxylate;
5-(4-(3,6-diazabicyclo[3.1.1]heptan-3-yl)-5,6-difluoro-8-(methylamino)-9H-pyrido[2,3 -b]indol-3 -yl)nicotinonitrile;
5-(4-(3-(aminomethyl)pyrrolidin-1-yl)-8-(ethylamino)-6-fluoro-5-methyl-9H-pyrido[2,3-b]indol-3 -yl)nicotinonitrile;
5-(4-(3-aminopiperidin-1-yl)-5,6-difluoro-8-(methylamino)-9H-pyrido[2,3-b]indol-3 -yl)nicotinonitrile;
4-(3-aminopiperidin-1-yl)-5,6-difluoro-3-(2-methoxypyrimidin-5-yl)-N-methyl-9H-pyrido[2,3-b]indol-8-amine;
5-[4-(7-amino-2-azaspiro[3.3]heptan-2-yl)-5,6-difluoro-8-(methylamino)-9H-pyrido[2,3-b]indol-3-yl]pyridine-3-carbonitrile;
5-(4-(1-amino-6-azaspiro[3.4]octan-6-yl)-5,6-difluoro-8-(methylamino)-9H-pyrido[2,3-b]indol-3 -yl)nicotinonitrile;
4-[*cis*-5-methyl-2,3,3a,4,6,6a-hexahydropyrrolo[2,3-c]pyrrol-1-yl]-5,6-difluoro-N-methyl-3-pyrazolo[1,5-a]pyrimidin-6-yl-9H-pyrido[2,3-b]indol-8-amine;
4-[4-(dimethylamino)-5,6-difluoro-8-(methylamino)-9H-pyrido[2,3-b]indol-3-yl]-1H-pyridin-2-one;
(*S*)-4-(3-(aminomethyl)piperidin-1-yl)-N-ethyl-6-fluoro-3-(2-methoxypyrimidin-5-yl)-9H-pyrido[2,3-b]indol-8-amine;
(*S*)-4-(3-(aminomethyl)piperidin-1-yl)-N-ethyl-5,6-difluoro-3-(2-methoxypyrimidin-5-yl)-9H-pyrido[2,3-b]indol-8-amine;
5-[4-[3-(aminomethyl)-3-fluoro-1-piperidyl]-5,6-difluoro-8-(methylamino)-9H-pyrido[2,3-b]indol-3-yl]pyridine-3-carbonitrile;
2-[4-[4-(dimethylamino)-5,6-difluoro-8-(methylamino)-9H-pyrido[2,3-b]indol-3-yl]-2-pyridyl]-N-methyl-acetamide;
4-(3,6-diazabicyclo[3.1.1]heptan-6-yl)-5,6-difluoro-3-(2-methoxypyrimidin-5-yl)-N-methyl-9H-pyrido[2,3-b]indol-8-amine;
5-(4-(3,6-diazabicyclo[3.1.1]heptan-6-yl)-5,6-difluoro-8-(methylamino)-9H-pyrido[2,3-b]indol-3-yl)nicotinonitrile;
4-(3,6-diazabicyclo[3.1.1]heptan-6-yl)-3-(2-ethoxypyrimidin-5-yl)-6-fluoro-N-methyl-9H-pyrido[2,3-b]indol-8-amine;
5-(4-(3,6-diazabicyclo[3.1.1]heptan-6-yl)-6-fluoro-8-(methylamino)-9H-pyrido[2,3-b]indol-3-yl)nicotinonitrile;
4-(3,6-diazabicyclo[3.1.1]heptan-6-yl)-6-fluoro-3-(2-methoxypyrimidin-5-yl)-N-methyl-9H-pyrido[2,3-b]indol-8-amine;
5,6-difluoro-N4,N4,N8-trimethyl-3-pyrazolo[1,5-a]pyrimidin-6-yl-9H-pyrido[2,3-b]indole-4,8-diamine;
5,6-Difluoro-3-(2-methoxypyrimidin-5-yl)-N-methyl-4-[(3aS,6aS)-5-methyl-2,3,3a,4,6,6a-hexahydropyrrolo[2,3-c]pyrrol-1-yl]-9H-pyrido[2,3-b]indol-8-amine;
1-[5-[4-(dimethylamino)-8-(ethylamino)-6-fluoro-9H-pyrido[2,3-b]indol-3-yl]pyrimidin-2-yl]cyclopropanecarboxylic acid;
1-[5-[4-(dimethylamino)-5,6-difluoro-8-(methylamino)-9H-pyrido[2,3-b]indol-3-yl]pyrimidin-2-yl]cyclopropanecarboxylic acid;
1-(5-(4-(dimethylamino)-6,7-difluoro-8-(methylamino)-9H-pyrido[2,3-b]indol-3-yl)pyrimidin-2-yl)cyclopropanecarboxylic acid;
5-[4-[4-(Aminomethyl)-3,3-difluoro-pyrrolidin-1-yl]-6-fluoro-8-(methylamino)-9H-pyrido[2,3-b]indol-3-yl]pyridine-3-carbonitrile;
4-[4-(aminomethyl)-3,3-difluoro-pyrrolidin-1-yl]-6-fluoro-3-(2-methoxypyrimidin-5-yl)-N-methyl-9H-pyrido[2,3-b]indol-8-amine;
4-[4-(aminomethyl)-3,3-difluoro-pyrrolidin-1-yl]-5,6-difluoro-3-(2-methoxypyrimidin-5-yl)-N-methyl-9H-pyrido[2,3-b]indol-8-amine;
5-[4-[4-(aminomethyl)-3,3-difluoro-pyrrolidin-1-yl]-5,6-difluoro-8-(methylamino)-9H-pyrido[2,3-b]indol-3-yl]pyridine-3-carbonitrile;
4-(1-(aminomethyl)cyclopropyl)-6-fluoro-3-(2-methoxypyrimidin-5-yl)-N-methyl-9H-pyrido[2,3-b]indol-8-amine;
*Cis*-4-(2-amino-5-azaspiro[2.4]heptan-5-yl)-5,6-difluoro-N-methyl-3-pyrazolo[1,5-a]pyrimidin-6-yl-9H-pyrido[2,3-b]indol-8-amine;
*Trans*-4-(2-Amino-5-azaspiro[2.4]heptan-5-yl)-5,6-difluoro-N-methyl-3-pyrazolo[1,5-a]pyrimidin-6-yl-9H-pyrido[2,3-b]indol-8-amine;
4-[(2R,3R)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine;
4-[(2S,3S)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine;
4-[(2S,3R)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine;and
4-[(2R,3S)-2-Amino-5-azaspiro[2.4]heptan-5-yl]-5,6-difluoro-N-methyl-3-pyrazolo[1,5-a]pyrimidin-6-yl-9H-pyrido[2,3-b]indol-8-amine;
or pharmaceutically acceptable salt thereof.

10. A process for the preparation of a compound according to any one of claims 1 to 9 comprising the reaction of compound of formula (Ii), 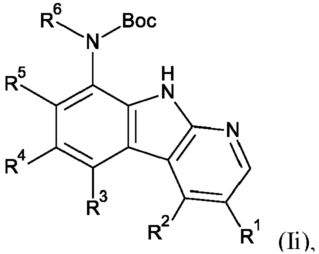 with an acid, wherein R¹ to R⁶ are defined as in any one of claims 1 to 8.

11. A compound according to any one of claims 1 to 9 for use as therapeutically active substance.

12. A pharmaceutical composition comprising a compound in accordance with any one of claims 1 to 9 and a therapeutically inert carrier.

13. A compound according to any one of claims 1 to 9 for use in the treatment or prophylaxis of bacterial infection.

## Patentansprüche

1. Verbindung der Formel (I) 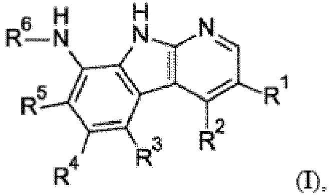 wobei
R¹ Oxopyridinyl; Pyrazolo[1,5-a]pyrimidinyl; Pyridinyl, substituiert mit Cyano, C₁₋₆-Alkoxycarbonyl oder C₁₋₆-Alkylaminocarbonyl-C₁₋₆-alkyl; Pyrimidinyl, substituiert mit C₁₋₆-Alkoxy oder Carboxy-C₃₋₇-cycloalkyl, ist;
R² (Amino-C₁₋₆-alkyl)-C₃₋₇-cycloalkyl;
(C₁₋₆-Alkyl)₂-amino;
2,3,3a,4,6,6a-Hexahydropyrrolo[2,3-c]pyrrolyl, substituiert mit C₁₋₆-Alkyl;
3,6-Diazabicyclo[3.1.1]heptanyl,
Azabicyclo[3.1.0]hexanyl, substituiert mit Amino-C₁₋₆-alkyl;
Azaspiro[2.4]heptanyl, substituiert mit Amino;
Azaspiro[3.3]heptanyl, substituiert mit Amino;
Azaspiro[3.4]octanyl, substituiert mit Amino;
Diazabicyclo[3.1.1]heptanyl;
Morpholinyl, substituiert mit Amino-C₁₋₆-alkyl;
Piperidinyl, einmal oder zweimal substituiert mit Substituenten, die unabhängig voneinander aus Amino, Amino-C₁₋₆-alkyl, C₁₋₆-Alkyl und Halogen ausgewählt sind; oder
Pyrrolidinyl, einmal, zweimal oder dreimal substituiert mit Substituenten, die unabhängig voneinander aus Amino, Amino-C₁₋₆-alkyl, C₁₋₆-Alkyl und Halogen ausgewählt sind, ist;
R³ H, C₁₋₆-Alkyl oder Halogen ist;
R⁴ Halogen oder Halogen-C₁₋₆-alkyl ist;
R⁵ H oder Halogen ist;
R⁶ C₁₋₆-Alkyl ist;
oder ein pharmazeutisch unbedenkliches Salz davon.

2. Verbindung nach Anspruch 1, wobei
R¹ Oxopyridinyl; Pyrazolo[1,5-a]pyrimidinyl; Cyanopyridinyl; Methoxycarbonylpyridinyl; Methylaminocarbonylmethylpyridinyl; Ethoxypyrimidinyl; Methoxypyrimidinyl oder Carboxycyclopropylpyrimidinyl ist;
R² (Aminomethyl)azabicyclo[3. 1.0]hexanyl; (Aminomethyl)cyclopropyl; (Aminomethyl)morpholinyl; (Aminomethyl)piperidinyl; (Aminomethyl)pyrrolidinyl; 3,6-Diazabicyclo[3 .1.1]heptanyl; Aminoazaspiro[2.4]heptanyl; Aminoazaspiro[3.3]heptanyl; Aminoazaspiro[3.4]octanyl; Aminopiperidinyl; Aminopyrrolidinyl; Diazabicyclo[3.1.1]heptanyl; Difluor(aminomethyl)pyrrolidinyl; Dimethylamino; Fluor(aminomethyl)piperidinyl; Fluor(aminomethyl)pyrrolidinyl; Methyl(aminomethyl)piperidinyl; Methyl(aminomethyl)pyrrolidinyl oder Methyl-2,3,3a,4,6,6a-hexahydropyrrolo[2,3-c]pyrrolyl ist;
R³ H, Methyl oder Fluor ist;
R⁴ Chlor, Fluor oder Trifluormethyl ist;
R⁵ H oder Fluor ist;
R⁶ Ethyl oder Methyl ist;
oder ein pharmazeutisch unbedenkliches Salz davon.

3. Verbindung nach Anspruch 1 oder pharmazeutisch unbedenkliches Salz davon, wobei R¹ Cyanopyridinyl oder C₁₋₆-Alkoxypyrimidinyl ist.

4. Verbindung nach Anspruch 3 oder pharmazeutisch unbedenkliches Salz davon, wobei R¹ Cyanopyridinyl oder Methoxypyrimidinyl ist.

5. Verbindung nach Anspruch 3 oder 4 oder pharmazeutisch unbedenkliches Salz davon, wobei R² (C₁₋₆-Alkyl)₂-amino; Aminoazaspiro[2.4]heptanyl; Piperidinyl, einmal oder zweimal substituiert mit Substituenten, die unabhängig voneinander aus Amino und Amino-C₁₋₆-alkyl ausgewählt sind; oder Pyrrolidinyl, einmal oder zweimal substituiert mit Substituenten, die unabhängig voneinander aus Amino und Amino-C₁₋₆-alkyl ausgewählt sind, ist.

6. Verbindung nach Anspruch 5 oder pharmazeutisch unbedenkliches Salz davon, wobei R² Dimethylamino, Aminopiperidinyl, (Aminomethyl)piperidinyl, Methyl(aminomethyl)piperidinyl, Aminopyrrolidinyl, (Aminomethyl)pyrrolidinyl oder Methyl(aminomethyl)pyrrolidinyl ist.

7. Verbindung nach Anspruch 5, wobei
R¹ Cyanopyridinyl oder C₁₋₆-Alkoxypyrimidinyl ist;
R² (C₁₋₆-Alkyl)₂-amino; Aminoazaspiro[2.4]heptanyl; Piperidinyl, einmal oder zweimal substituiert mit Substituenten, die unabhängig voneinander aus Amino und Amino-C₁₋₆-alkyl ausgewählt sind; oder Pyrrolidinyl, einmal oder zweimal substituiert mit Substituenten, die unabhängig voneinander aus Amino und Amino-C₁₋₆-alkyl ausgewählt sind, ist;
R³ Halogen ist;
R⁴ Halogen ist;
R⁵ H ist;
R⁶ C₁₋₆-Alkyl ist;
oder ein pharmazeutisch unbedenkliches Salz davon.

8. Verbindung nach Anspruch 7, wobei
R¹ Cyanopyridinyl oder Methoxypyrimidinyl ist;
R² Dimethylamino, Aminopiperidinyl, (Aminomethyl)piperidinyl, Methyl(aminomethyl)piperidinyl, Aminopyrrolidinyl, (Aminomethyl)pyrrolidinyl oder Methyl(aminomethyl)pyrrolidinyl ist;
R³ Fluor ist;
R⁴ Fluor oder Chlor ist;
R⁵ H ist;
R⁶ Methyl oder Ethyl ist;
oder ein pharmazeutisch unbedenkliches Salz davon.

9. Verbindung nach den Ansprüchen 1 bis 8, ausgewählt aus:
5-[4-[(3*R*)-3-Aminopyrrolidin-1-yl]-6-fluor-8-(methylamino)-9H-pyrido[2,3-b]indol-3-yl]pyridin-3-carbonitril;
5-[4-(3-Aminopyrrolidin-1-yl)-8-(ethylamino)-5,6-difluor-9H-pyrido[2,3-b]indol-3-yl]pyridin-3 -carbonitril;
5-[4-(3-Aminopyrrolidin-1-yl)-6-chlor-8-(ethylamino)-9H-pyrido[2,3-b]indol-3-yl]pyridin-3 -carbonitril;
5-[4-[(3*R*)-3-Aminopyrrolidin-1-yl]-8-(ethylamino)-5,6-difluor-9H-pyrido[2,3-b]indol-3-yl]pyridin-3-carbonitril;
5-[4-[(3*R*)-3-Aminopyrrolidin-1-yl]-6-chlor-8-(ethylamino)-9H-pyrido[2,3-b]indol-3-yl]pyridin-3 -carbonitril;
5-[4-[2-(Aminomethyl)pyrrolidin-1-yl]-8-(ethylamino)-6-fluor-9H-pyrido[2,3-b]indol-3-yl]pyridin-3-carbonitril;
5-[4-[3-(Aminomethyl)pyrrolidin-1-yl]-8-(ethylamino)-5,6-difluor-9H-pyrido[2,3-b]indol-3-yl]pyridin-3-carbonitril;
4-[3-(Aminomethyl)pyrrolidin-1-yl]-N-ethyl-5,6-difluor-3-(2-methoxypyrimidin-5-yl)-9H-pyrido [2,3 -b]indol-8-amin;
4-(3-Aminopyrrolidin-1-yl)-N-ethyl-5,6-difluor-3-pyrazolo[1,5-a]pyrimidin-6-yl-9H-pyrido[2,3-b]indol-8-amin;
5-[4-[3-(Aminomethyl)-3-fluorpyrrolidin-1-yl]-8-(ethylamino)-6-fluor-9H-pyrido[2,3-b]indol-3-yl]pyridin-3-carbonitril;
5-[8-(Ethylamino)-6-fluor-4-[*cis*-2-amino-5-azaspiro[2.4]heptan-5-yl]-9H-pyrido[2,3-b]indol-3-yl]pyridin-3-carbonitril;
5-[8-(Ethylamino)-6-fluor-4-[*trans*-2-amino-5-azaspiro[2.4]heptan-5-yl]-9H-pyrido[2,3-b]indol-3-yl]pyridin-3-carbonitril;
5-[4-[3-(Aminomethyl)pyrrolidin-1-yl]-5,6-difluor-8-(methylamino)-9H-pyrido[2,3-b]indol-3-yl]pyridin-3-carbonitril;
5-[4-[3-(Aminomethyl)-1-piperidyl]-8-(ethylamino)-6-fluor-9H-pyrido[2,3-b]indol-3-yl]pyridin-3 -carbonitril;
5-[4-[(3R)-3-Aminopyrrolidin-1-yl]-5,6-difluor-8-(methylamino)-9H-pyrido[2,3-b]indol-3-yl]pyridin-3-carbonitril;
5-[6-Fluor-8-(methylamino)-4-[*cis*-2-amino-5-azaspiro[2.4]heptan-5-yl]-9H-pyrido[2,3-b]indol-3-yl]pyridin-3-carbonitril;
5-[6-Fluor-8-(methylamino)-4-[*trans*-2-amino-5-azaspiro[2.4]heptan-5-yl]-9H-pyrido [2,3 -b]indol-3 -yl]pyridin-3 -carbonitril;
4-[(3*R*)-3-Aminopyrrolidin-1-yl]-N-ethyl-5,6-difluor-3-(2-methoxypyrimidin-5-yl)-9H-pyrido[2,3-b]indol-8-amin;
4-[3-(Aminomethyl)pyrrolidin-1-yl]-5,6-difluor-3-(2-methoxypyrimidin-5-yl)-N-methyl-9H-pyrido[2,3-b]indol-8-amin;
5-[4-(3-Aminopyrrolidin-1-yl)-8-(ethylamino)-6-(trifluormethyl)-9H-pyrido[2,3-b]indol-3-yl]pyridin-3-carbonitril;
5-[4-[3-(Aminomethyl)-1-piperidyl]-5,6-difluor-8-(methylamino)-9H-pyrido[2,3-b]indol-3-yl]pyridin-3-carbonitril;
5-[4-[3-(Aminomethyl)-3-fluorpyrrolidin-1-yl]-5,6-difluor-8-(methylamino)-9H-pyrido [2,3 -b]indol-3 -yl]pyridin-3 -carbonitril;
5-[4-[3-(Aminomethyl)-3-fluorpyrrolidin-1-yl]-6-fluor-8-(methylamino)-9H-pyrido[2,3-b]indol-3-yl]pyridin-3-carbonitril;
4-[3-(Aminomethyl)-3-fluorpyrrolidin-1-yl]-6-fluor-3-(2-methoxypyrimidin-5-yl)-N-methyl-9H-pyrido[2,3-b]indol-8-amin;
4-[(3S)-3-(Aminomethyl)-1-piperidyl]-5,6-difluor-3-(2-methoxypyrimidin-5-yl)-N-methyl-9H-pyrido[2,3-b]indol-8-amin;
4-[3-(Aminomethyl)-3-methyl-1-piperidyl]-5,6-difluor-3-(2-methoxypyrimidin-5-yl)-N-methyl-9H-pyrido[2,3-b]indol-8-amin;
4-[1-(Aminomethyl)-3-azabicyclo[3.1.0]hexan-3-yl]-5,6-difluor-3-(2-methoxypyrimidin-5-yl)-N-methyl-9H-pyrido[2,3-b]indol-8-amin;
4-[3-(Aminomethyl)-3-fluorpyrrolidin-1-yl]-5,6-difluor-3-(2-methoxypyrimidin-5-yl)-N-methyl-9H-pyrido [2,3 -b]indol-8-amin;
5-[4-[*trans*-2-Amino-5-azaspiro[2.4]heptan-5-yl]-5,6-difluor-8-(methylamino)-9H-pyrido [2,3 -b]indol-3 -yl]pyridin-3 -carbonitril;
5-[4-[*cis*-2-Amino-5-azaspiro[2.4]heptan-5-yl]-5,6-difluor-8-(methylamino)-9H-pyrido [2,3 -b]indol-3 -yl]pyridin-3 -carbonitril;
5-[4-[2-(Aminomethyl)morpholin-4-yl]-5,6-difluor-8-(methylamino)-9H-pyrido[2,3-b]indol-3-yl]pyridin-3-carbonitril;
5-[4-[1-(Aminomethyl)-3-azabicyclo[3.1.0]hexan-3-yl]-5,6-difluor-8-(methylamino)-9H-pyrido [2,3 -b]indol-3 -yl]pyridin-3 -carbonitril;
4-[2-(Aminomethyl)morpholin-4-yl]-5,6-difluor-3-(2-methoxypyrimidin-5-yl)-N-methyl-9H-pyrido[2,3-b]indol-8-amin;
4-[*cis*-2-Amino-5-azaspiro[2.4]heptan-5-yl]-5,6-difluor-3-(2-methoxypyrimidin-5-yl)-N-methyl-9H-pyrido [2,3 -b]indol-8-amin;
4-[*trans*-2-Amino-5-azaspiro[2.4]heptan-5-yl]-5,6-difluor-3-(2-methoxypyrimidin-5-yl)-N-methyl-9H-pyrido[2,3-b]indol-8-amin;
4-[3-(Aminomethyl)-3-methylpyrrolidin-1-yl]-5,6-difluor-3-(2-methoxypyrimidin-5-yl)-N-methyl-9H-pyrido[2,3-b]indol-8-amin;
4-[2-(Aminomethyl)pyrrolidin-1-yl]-5,6-difluor-3-(2-methoxypyrimidin-5-yl)-N-methyl-9H-pyrido[2,3-b]indol-8-amin;
5-[4-[2-(Aminomethyl)pyrrolidin-1-yl]-5,6-difluor-8-(methylamino)-9H-pyrido[2,3-b]indol-3-yl]pyridin-3-carbonitril;
5-[4-[(3*S*)-3-(Aminomethyl)-1-piperidyl]-5,6-difluor-8-(methylamino)-9H-pyrido[2,3-b]indol-3-yl]pyridin-3-carbonitril;
Methyl-5-[4-[(3S)-3-(aminomethyl)-1-piperidyl]-5,6-difluor-8-(methylamino)-9H-pyrido[2,3-b]indol-3-yl]pyridin-3-carboxylat;
5-(4-(3,6-Diazabicyclo[3.1.1]heptan-3-yl)-5,6-difluor-8-(methylamino)-9H-pyrido[2,3-b]indol-3-yl)nicotinonitril;
5-(4-(3-(Aminomethyl)pyrrolidin-1-yl)-8-(ethylamino)-6-fluor-5-methyl-9H-pyrido [2,3 -b]indol-3 -yl)nicotinonitril;
5-(4-(3-Aminopiperidin-1-yl)-5,6-difluor-8-(methylamino)-9H-pyrido[2,3-b]indol-3-yl)nicotinonitril;
4-(3-Aminopiperidin-l-yl)-5,6-difluor-3-(2-methoxypyrimidin-5-yl)-N-methyl-9H-pyrido[2,3-b]indol-8-amin;
5-[4-(7-Amino-2-azaspiro[3.3]heptan-2-yl)-5,6-difluor-8-(methylamino)-9H-pyrido [2,3 -b]indol-3 -yl]pyridin-3 -carbonitril;
5-(4-(1-Amino-6-azaspiro[3.4]octan-6-yl)-5,6-difluor-8-(methylamino)-9H-pyrido[2,3-b]indol-3-yl)nicotinonitril;
4-[*cis*-5-Methyl-2,3,3a,4,6,6a-hexahydropyrrolo[2,3-c]pyrrol-1-yl]-5,6-difluor-N-methyl-3-pyrazolo[1,5-a]pyrimidin-6-yl-9H-pyrido[2,3-b]indol-8-amin;
4-[4-(Dimethylamino)-5,6-difluor-8-(methylamino)-9H-pyrido[2,3-b]indol-3-yl]-1H-pyridin-2-on;
(*S*)-4-(3-(Aminomethyl)piperidin-1-yl)-N-ethyl-6-fluor-3-(2-methoxypyrimidin-5-yl)-9H-pyrido [2,3 -b]indol-8-amin;
(*S*)-4-(3-(Aminomethyl)piperidin-1-yl)-N-ethyl-5,6-difluor-3-(2-methoxypyrimidin-5-yl)-9H-pyrido[2,3-b]indol-8-amin;
5-[4-[3-(Aminomethyl)-3-fluor-1-piperidyl]-5,6-difluor-8-(methylamino)-9H-pyrido [2,3 -b]indol-3 -yl]pyridin-3 -carbonitril;
2-[4-[4-(Dimethylamino)-5,6-difluor-8-(methylamino)-9H-pyrido[2,3-b]indol-3-yl]-2-pyridyl]-N-methylacetamid;
4-(3,6-Diazabicyclo[3.1.1]heptan-6-yl)-5,6-difluor-3-(2-methoxypyrimidin-5-yl)-N-methyl-9H-pyrido[2,3-b]indol-8-amin;
5-(4-(3,6-Diazabicyclo[3.1.1]heptan-6-yl)-5,6-difluor-8-(methylamino)-9H-pyrido[2,3-b]indol-3-yl)nicotinonitril;
4-(3,6-Diazabicyclo[3.1.1]heptan-6-yl)-3-(2-ethoxypyrimidin-5-yl)-6-fluor-N-methyl-9H-pyrido [2,3 -b]indol-8-amin;
5-(4-(3,6-Diazabicyclo[3.1.1]heptan-6-yl)-6-fluor-8-(methylamino)-9H-pyrido[2,3-b]indol-3-yl)nicotinonitril;
4-(3,6-Diazabicyclo[3.1.1]heptan-6-yl)-6-fluor-3-(2-methoxypyrimidin-5-yl)-N-methyl-9H-pyrido [2,3 -b]indol-8-amin;
5,6-Difluor-N4,N4,N8-trimethyl-3-pyrazolo[1,5-a]pyrimidin-6-yl-9H-pyrido[2,3-b]indol-4,8-diamin;
5,6-Difluor-3-(2-methoxypyrimidin-5-yl)-N-methyl-4-[(3aS,6aS)-5-methyl-2,3,3a,4,6,6a-hexahydropyrrolo[2,3-c]pyrrol-1-yl]-9H-pyrido[2,3-b]indol-8-amin;
1-[5-[4-(Dimethylamino)-8-(ethylamino)-6-fluor-9H-pyrido[2,3-b]indol-3-yl]pyrimidin-2-yl]cyclopropancarbonsäure;
1-[5-[4-(Dimethylamino)-5,6-difluor-8-(methylamino)-9H-pyrido[2,3-b]indol-3-yl]pyrimidin-2-yl] cyclopropancarbonsäure;
1-(5-(4-(Dimethylamino)-6,7-difluor-8-(methylamino)-9H-pyrido[2,3-b]indol-3-yl)pyrimidin-2-yl)cyclopropancarbonsäure;
5-[4-[4-(Aminomethyl)-3,3-difluorpyrrolidin-1-yl]-6-fluor-8-(methylamino)-9H-pyrido [2,3 -b]indol-3 -yl]pyridin-3 -carbonitril;
4-[4-(Aminomethyl)-3,3-difluorpyrrolidin-1-yl]-6-fluor-3-(2-methoxypyrimidin-5-yl)-N-methyl-9H-pyrido [2,3 -b]indol-8-amin;
4-[4-(Aminomethyl)-3,3 -difluorpyrrolidin-1-yl] -5,6-difluor-3 -(2-methoxypyrimidin-5-yl)-N-methyl-9H-pyrido[2,3-b]indol-8-amin;
5-[4-[4-(Aminomethyl)-3,3-difluorpyrrolidin-1-yl]-5,6-difluor-8-(methylamino)-9H-pyrido [2,3 -b]indol-3 -yl]pyridin-3 -carbonitril;
4-(1-(Aminomethyl)cyclopropyl)-6-fluor-3-(2-methoxypyrimidin-5-yl)-N-methyl-9H-pyrido[2,3-b]indol-8-amin;
*cis*-4-(2-Amino-5-azaspiro[2.4]heptan-5-yl)-5,6-difluor-N-methyl-3-pyrazolo[1,5-a]pyrimidin-6-yl-9H-pyrido[2,3-b]indol-8-amin;
*trans*-4-(2-Amino-5-azaspiro[2.4]heptan-5-yl)-5,6-difluor-N-methyl-3-pyrazolo[1,5-a]pyrimidin-6-yl-9H-pyrido[2,3-b]indol-8-amin;
4-[(2R,3R)-2-Amino-5-azaspiro[2.4]heptan-5-yl]-5,6-difluor-N-methyl-3-pyrazolo[1,5-a]pyrimidin-6-yl-9H-pyrido[2,3-b]indol-8-amin;
4-[(2S,3S)-2-Amino-5-azaspiro[2.4]heptan-5-yl]-5,6-difluor-N-methyl-3-pyrazolo[1,5-a]pyrimidin-6-yl-9H-pyrido[2,3-b]indol-8-amin;
4-[(2S,3R)-2-Amino-5-azaspiro[2.4]heptan-5-yl]-5,6-difluor-N-methyl-3-pyrazolo[1,5-a]pyrimidin-6-yl-9H-pyrido[2,3-b]indol-8-amin; und
4-[(2R,3S)-2-Amino-5-azaspiro[2.4]heptan-5-yl]-5,6-difluor-N-methyl-3-pyrazolo[1,5-a]pyrimidin-6-yl-9H-pyrido[2,3-b]indol-8-amin;
oder ein pharmazeutisch unbedenkliches Salz davon.

10. Verfahren zur Herstellung einer Verbindung nach einem der Ansprüche 1 bis 9, umfassend die Reaktion einer Verbindung der Formel (Ii), 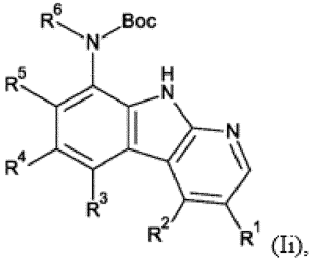 mit einer Säure, wobei R¹ bis R⁶ wie in einem der Ansprüche 1 bis 8 definiert sind.

11. Verbindung nach einem der Ansprüche 1 bis 9 zur Verwendung als therapeutisch wirksame Substanz.

12. Pharmazeutische Zusammensetzung, umfassend eine Verbindung nach einem der Ansprüche 1 bis 9 und einen therapeutisch inerten Träger.

13. Verbindung nach einem der Ansprüche 1 bis 9 zur Verwendung bei der Behandlung oder Prophylaxe einer bakteriellen Infektion.

## Revendications

1. Composé de formule (I), 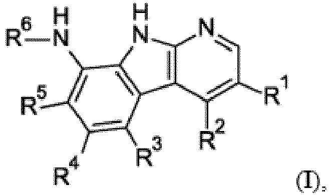 dans lequel
R¹ représente un oxopyridinyle ; un pyrazolo[1,5-a]pyrimidinyle ; un pyridinyle substitué par un cyano, un alcoxy en C₁₋₆-carbonyle ou un alkyle en C₁₋₆-aminocarbonylalkyle en C₁₋₆ ; un pyrimidinyle substitué par un alcoxy en C₁₋₆ ou un carboxycycloalkyle en C₃₋₇ ;
R² représente un (aminoalkyle en C₁₋₆)cycloalkyle en C₃₋₇ ;
un (alkyle en C₁₋₆)₂amino ;
un 2,3,3a,4,6,6a-hexahydropyrrolo[2,3-c]pyrrolyle substitué par un alkyle en C₁₋₆ ; un 3,6-diazabicyclo[3.1.1]heptanyle ;
un azabicyclo[3.1.0]hexanyle substitué par un aminoalkyle en C₁₋₆ ;
un azaspiro[2.4]heptanyle substitué par un amino ;
un azaspiro[3.3]heptanyle substitué par un amino ;
un azaspiro[3.4]octanyle substitué par un amino ;
un diazabicyclo[3.1.1]heptanyle ;
un morpholinyle substitué par un aminoalkyle en C₁₋₆ ;
un pipéridinyle substitué une ou deux fois par des substituants indépendamment choisis parmi un amino, un aminoalkyle en C₁₋₆, un alkyle en C₁₋₆ et un halogène ; ou
un pyrrolidinyle substitué une, deux ou trois fois par des substituants indépendamment choisis parmi un amino, un aminoalkyle en C₁₋₆, un alkyle en C₁₋₆ et un halogène ;
R³ représente H, un alkyle en C₁₋₆ ou un halogène ;
R⁴ représente un halogène ou un halogénoalkyle en C₁₋₆ ;
R⁵ représente H ou un halogène ;
R⁶ représente un alkyle en C₁₋₆ ;
ou un sel pharmaceutiquement acceptable de celui-ci.

2. Composé selon la revendication 1, dans lequel
R¹ représente un oxopyridinyle ; un pyrazolo[1,5-a]pyrimidinyle ; un cyanopyridinyle ; un méthoxycarbonylpyridinyle ; un méthylaminocarbonylméthylpyridinyle ; un éthoxypyrimidinyle ; un méthoxypyrimidinyle ou un carboxycyclopropylpyrimidinyle ;
R² représente un (aminométhyl)azabicyclo[3.1.0]hexanyle ; un (aminométhyl)cyclopropyle ; un (aminométhyl)morpholinyle ; un (aminométhyl)pipéridinyle ; un (aminométhyl)pyrrolidinyle ; un 3,6-diazabicyclo[3.1.1]heptanyle ; un aminoazaspiro[2.4]heptanyle ; un aminoazaspiro[3.3]heptanyle ; un aminoazaspiro[3.4]octanyle ; un aminopipéridinyle ; un aminopyrrolidinyle ; un diazabicyclo[3.1.1]heptanyle ; un difluoro(aminométhyl)pyrrolidinyle ; un diméthylamino ; un fluoro(aminométhyl)pipéridinyle ; un fluoro(aminométhyl)pyrrolidinyle ; un méthyl(aminométhyl)pipéridinyle ; un méthyl(aminométhyl)pyrrolidinyle ou un méthyl-2,3,3 a,4,6,6a-hexahydropyrrolo[2,3 -c]pyrrolyle ;
R³ représente H, un méthyle ou un fluor ;
R⁴ représente un chlore, un fluor ou un trifluorométhyle ;
R⁵ représente H ou un fluor ;
R⁶ représente un éthyle ou un méthyle ;
ou un sel pharmaceutiquement acceptable de celui-ci.

3. Composé selon la revendication 1, ou un sel pharmaceutiquement acceptable de celui-ci, dans lequel R¹ représente un cyanopyridinyle ou un alcoxy en C₁₋₆-pyrimidinyle.

4. Composé selon la revendication 3, ou un sel pharmaceutiquement acceptable de celui-ci, dans lequel R¹ représente un cyanopyridinyle ou un méthoxypyrimidinyle.

5. Composé selon la revendication 3 ou 4, ou un sel pharmaceutiquement acceptable de celui-ci, dans lequel R² représente un (alkyle en C₁₋₆)₂amino ; un aminoazaspiro[2.4]heptanyle ; un pipéridinyle substitué une ou deux fois par des substituants indépendamment choisis parmi un amino et un aminoalkyle en C₁₋₆ ; ou un pyrrolidinyle substitué une ou deux fois par des substituants indépendamment choisis parmi un amino et un aminoalkyle en C₁₋₆.

6. Composé selon la revendication 5, ou un sel pharmaceutiquement acceptable de celui-ci, dans lequel R² représente un diméthylamino, un aminopipéridinyle, un (aminométhyl)pipéridinyle, un méthyl(aminométhyl)pipéridinyle, un aminopyrrolidinyle, un (aminométhyl)pyrrolidinyle ou un méthyl(aminométhyl)pyrrolidinyle.

7. Composé selon la revendication 5, dans lequel
R¹ représente un cyanopyridinyle ou un alcoxy en C₁₋₆-pyrimidinyle ;
R² représente un (alkyle en C₁₋₆)₂amino ; un aminoazaspiro[2.4]heptanyle ; un pipéridinyle substitué une ou deux fois par des substituants indépendamment choisis parmi un amino et un aminoalkyle en C₁₋₆ ; ou un pyrrolidinyle substitué une ou deux fois par des substituants indépendamment choisis parmi un amino et un aminoalkyle en C₁₋₆ ;
R³ représente un halogène ;
R⁴ représente un halogène ;
R⁵ représente H ;
R⁶ représente un alkyle en C₁₋₆ ;
ou un sel pharmaceutiquement acceptable de celui-ci.

8. Composé selon la revendication 7, dans lequel
R¹ représente un cyanopyridinyle ou un méthoxypyrimidinyle ;
R² représente un diméthylamino, un aminopipéridinyle, un (aminométhyl)pipéridinyle, un méthyl(aminométhyl)pipéridinyle, un aminopyrrolidinyle, un (aminométhyl)pyrrolidinyle ou un méthyl(aminométhyl)pyrrolidinyle ;
R³ représente un fluor ;
R⁴ représente un fluor ou un chlore ;
R⁵ représente H ;
R⁶ représente un méthyle ou un éthyle ;
ou un sel pharmaceutiquement acceptable de celui-ci.

9. Composé selon les revendications 1 à 8 choisi parmi :
le 5-[4-[(3R)-3-aminopyrrolidin-1-yl]-6-fluoro-8-(méthylamino)-9H-pyrido[2,3-b]indol-3-yl]pyridine-3-carbonitrile ;
le 5-[4-(3-aminopyrrolidin-1-yl)-8-(éthylamino)-5,6-difluoro-9H-pyrido[2,3-b]indol-3-yl]pyridine-3-carbonitrile ;
le 5-[4-(3-aminopyrrolidin-1-yl)-6-chloro-8-(éthylamino)-9H-pyrido[2,3-b]indol-3-yl]pyridine-3-carbonitrile ;
le 5-[4-[(3*R*)-3-aminopyrrolidin-1-yl] -8-(éthylamino)-5,6-difluoro-9H-pyrido[2,3-b]indol-3-yl]pyridine-3-carbonitrile ;
le 5-[4-[(3*R*)-3-aminopyrrolidin-1-yl]-6-chloro-8-(éthylamino)-9H-pyrido[2,3-b]indol-3-yl]pyridine-3-carbonitrile ;
le 5-[4-[2-(aminométhyl)pyrrolidin-1-yl]-8-(éthylamino)-6-fluoro-9H-pyrido[2,3-b]indol-3-yl]pyridine-3-carbonitrile ;
le 5-[4-[3-(aminométhyl)pyrrolidin-1-yl]-8-(éthylamino)-5,6-difluoro-9H-pyrido[2,3-b]indol-3-yl]pyridine-3-carbonitrile ;
la 4-[3-(aminométhyl)pyrrolidin-1-yl]-N-éthyl-5,6-difluoro-3-(2-méthoxypyrimidin-5-yl)-9H-pyrido[2,3-b]indol-8-amine;
la 4-(3-aminopyrrolidin-1-yl)-N-éthyl-5,6-difluoro-3-pyrazolo[1,5-a]pyrimidin-6-yl-9H-pyrido[2,3-b]indol-8-amine ;
le 5-[4-[3-(aminométhyl)-3-fluoro-pyrrolidin-1-yl]-8-(éthylamino)-6-fluoro-9H-pyrido [2,3-b]indol-3 -yl]pyridine-3 -carbonitrile ;
le 5-[8-(éthylamino)-6-fluoro-4-[*cis*-2-amino-5-azaspiro[2.4]heptan-5-yl]-9H-pyrido [2,3-b]indol-3 -yl]pyridine-3 -carbonitrile ;
le 5-[8-(éthylamino)-6-fluoro-4-[*trans*-2-amino-5-azaspiro[2.4]heptan-5-yl]-9H-pyrido [2,3-b]indol-3 -yl]pyridine-3 -carbonitrile ;
le 5-[4-[3-(aminométhyl)pyrrolidin-1-yl]-5,6-difluoro-8-(méthylamino)-9H-pyrido[2,3-b]indol-3-yl]pyridine-3-carbonitrile ;
le 5-[4-[3-(aminométhyl)-1-pipéridyl]-8-(éthylamino)-6-fluoro-9H-pyrido[2,3-b]indol-3-yl]pyridine-3-carbonitrile ;
le 5-[4-[(3*R*)-3-aminopyrrolidin-1-yl]-5,6-difluoro-8-(méthylamino)-9H-pyrido[2,3-b]indol-3-yl]pyridine-3-carbonitrile ;
le 5-[6-fluoro-8-(méthylamino)-4-[*cis*-2-amino-5-azaspiro[2.4]heptan-5-yl]-9H-pyrido [2,3-b]indol-3 -yl]pyridine-3 -carbonitrile ;
le 5-[6-fluoro-8-(méthylamino)-4-[*trans*-2-amino-5-azaspiro[2.4]heptan-5-yl]-9H-pyrido [2,3-b]indol-3 -yl]pyridine-3 -carbonitrile ;
la 4-[(3*R*)-3-aminopyrrolidin-1-yl]-N-éthyl-5,6-difluoro-3-(2-méthoxypyrimidin-5-yl)-9H-pyrido[2,3-b]indol-8-amine ;
la 4-[3-(aminométhyl)pyrrolidin-1-yl]-5,6-difluoro-3-(2-méthoxypyrimidin-5-yl)-N-méthyl-9H-pyrido[2,3-b]indol-8-amine ;
le 5-[4-(3-aminopyrrolidin-1-yl)-8-(éthylamino)-6-(trifluorométhyl)-9H-pyrido[2,3-b]indol-3-yl]pyridine-3-carbonitrile ;
le 5-[4-[3-(aminométhyl)-1-pipéridyl]-5,6-difluoro-8-(méthylamino)-9H-pyrido[2,3-b]indol-3-yl]pyridine-3-carbonitrile ;
le 5-[4-[3-(aminométhyl)-3-fluoro-pyrrolidin-1-yl]-5,6-difluoro-8-(méthylamino)-9H-pyrido [2,3-b]indol-3 -yl]pyridine-3 -carbonitrile ;
le 5-[4-[3-(aminométhyl)-3-fluoro-pyrrolidin-1-yl]-6-fluoro-8-(méthylamino)-9H-pyrido [2,3-b]indol-3 -yl]pyridine-3 -carbonitrile ;
la 4-[3-(aminométhyl)-3-fluoro-pyrrolidin-1-yl]-6-fluoro-3-(2-méthoxypyrimidin-5-yl)-N-méthyl-9H-pyrido [2,3-b]indol-8-amine ;
la 4-[(3*S*)-3-(aminométhyl)-1-pipéridyl]-5,6-difluoro-3-(2-méthoxypyrimidin-5-yl)-N-méthyl-9H-pyrido[2,3-b]indol-8-amine ;
la 4-[3-(aminométhyl)-3-méthyl-1-pipéridyl]-5,6-difluoro-3-(2-méthoxypyrimidin-5-yl)-N-méthyl-9H-pyrido [2,3-b]indol-8-amine ;
la 4-[1-(aminométhyl)-3-azabicyclo[3.1.0]hexan-3-yl]-5,6-difluoro-3-(2-méthoxypyrimidin-5-yl)-N-méthyl-9H-pyrido[2,3-b]indol-8-amine ;
la 4-[3-(aminométhyl)-3-fluoro-pyrrolidin-1-yl]-5,6-difluoro-3-(2-méthoxypyrimidin-5-yl)-N-méthyl-9H-pyrido [2,3-b]indol-8-amine ;
le 5-[4-[*trans*-2-amino-5-azaspiro[2.4]heptan-5-yl]-5,6-difluoro-8-(méthylamino)-9H-pyrido [2,3-b]indol-3 -yl]pyridine-3 -carbonitrile ;
le 5-[4-[*cis*-2-amino-5-azaspiro[2.4]heptan-5-yl]-5,6-difluoro-8-(méthylamino)-9H-pyrido [2,3-b]indol-3 -yl]pyridine-3 -carbonitrile ;
le 5-[4-[2-(aminométhyl)morpholin-4-yl]-5,6-difluoro-8-(méthylamino)-9H-pyrido[2,3-b]indol-3-yl]pyridine-3-carbonitrile ;
le 5-[4-[1-(aminométhyl)-3-azabicyclo[3.1.0]hexan-3-yl]-5,6-difluoro-8-(méthylamino)-9H-pyrido [2,3-b]indol-3 -yl]pyridine-3 -carbonitrile ;
la 4-[2-(aminométhyl)morpholin-4-yl]-5,6-difluoro-3-(2-méthoxypyrimidin-5-yl)-N-méthyl-9H-pyrido[2,3-b]indol-8-amine ;
la 4-[*cis*-2-amino-5-azaspiro[2.4]heptan-5-yl]-5,6-difluoro-3-(2-méthoxypyrimidin-5-yl)-N-méthyl-9H-pyrido [2,3-b]indol-8-amine ;
la 4-[*trans*-2-amino-5-azaspiro[2.4]heptan-5-yl]-5,6-difluoro-3-(2-méthoxypyrimidin-5-yl)-N-méthyl-9H-pyrido [2,3-b]indol-8-amine ;
la 4-[3-(aminométhyl)-3-méthyl-pyrrolidin-1-yl]-5,6-difluoro-3 -(2-méthoxypyrimidin-5-yl)-N-méthyl-9H-pyrido[2,3-b]indol-8-amine ;
la 4-[2-(aminométhyl)pyrrolidin-1-yl]-5,6-difluoro-3-(2-méthoxypyrimidin-5-yl)-N-méthyl-9H-pyrido[2,3-b]indol-8-amine ;
le 5-[4-[2-(aminométhyl)pyrrolidin-1-yl]-5,6-difluoro-8-(méthylamino)-9H-pyrido[2,3-b]indol-3-yl]pyridine-3-carbonitrile ;
le 5-[4-[(3*S*)-3-(aminométhyl)-1-pipéridyl]-5,6-difluoro-8-(méthylamino)-9H-pyrido [2,3-b]indol-3 -yl]pyridine-3 -carbonitrile ;
le 5-[4-[(3S)-3-(aminométhyl)-1-pipéridyl]-5,6-difluoro-8-(méthylamino)-9H-pyrido[2,3-b]indol-3-yl]pyridine-3-carboxylate de méthyle ;
le 5-(4-(3,6-diazabicyclo[3. 1. 1]heptan-3-yl)-5,6-difluoro-8-(méthylamino)-9H-pyrido [2,3-b]indol-3 -yl)nicotinonitrile ;
le 5-(4-(3-(aminométhyl)pyrrolidin-1-yl)-8-(éthylamino)-6-fluoro-5-méthyl-9H-pyrido [2,3-b]indol-3 -yl)nicotinonitrile ;
le 5-(4-(3-aminopipéridin-1-yl)-5,6-difluoro-8-(méthylamino)-9H-pyrido[2,3-b]indol-3-yl)nicotinonitrile ;
la 4-(3-aminopipéridin-1-yl)-5,6-difluoro-3-(2-méthoxypyrimidin-5-yl)-N-méthyl-9H-pyrido[2,3-b]indol-8-amine ;
le 5-[4-(7-amino-2-azaspiro[3.3]heptan-2-yl)-5,6-difluoro-8-(méthylamino)-9H-pyrido [2,3-b]indol-3 -yl]pyridine-3 -carbonitrile ;
le 5-(4-(1-amino-6-azaspiro[3.4]octan-6-yl)-5,6-difluoro-8-(méthylamino)-9H-pyrido [2,3-b]indol-3 -yl)nicotinonitrile ;
la 4-[*cis*-5-méthyl-2,3,3a,4,6,6a-hexahydropyrrolo[2,3-c]pyrrol-1-yl]-5,6-difluoro-N-méthyl-3-pyrazolo[1,5-a]pyrimidin-6-yl-9H-pyrido[2,3-b]indol-8-amine ;
la 4-[4-(diméthylamino)-5,6-difluoro-8-(méthylamino)-9H-pyrido[2,3-b]indol-3-yl]-1H-pyridin-2-one ;
la (*S*)-4-(3-(aminométhyl)pipéridin-1-yl)-N-éthyl-6-fluoro-3-(2-méthoxypyrimidin-5-yl)-9H-pyrido[2,3-b]indol-8-amine;
la (*S*)-4-(3-(aminométhyl)pipéridin-1-yl)-N-éthyl-5,6-difluoro-3-(2-méthoxypyrimidin-5-yl)-9H-pyrido[2,3-b]indol-8-amine ;
le 5-[4-[3-(aminométhyl)-3-fluoro-1-pipéridyl]-5,6-difluoro-8-(méthylamino)-9H-pyrido [2,3-b]indol-3 -yl]pyridine-3 -carbonitrile ;
le 2-[4-[4-(diméthylamino)-5,6-difluoro-8-(méthylamino)-9H-pyrido[2,3-b]indol-3-yl]-2-pyridyl]-N-méthyl-acétamide ;
la 4-(3,6-diazabicyclo[3.1.1]heptan-6-yl)-5,6-difluoro-3-(2-méthoxypyrimidin-5-yl)-N-méthyl-9H-pyrido[2,3-b]indol-8-amine ;
le 5-(4-(3,6-diazabicyclo[3.1.1]heptan-6-yl)-5,6-difluoro-8-(méthylamino)-9H-pyrido [2,3-b]indol-3 -yl)nicotinonitrile ;
la 4-(3,6-diazabicyclo[3.1.1]heptan-6-yl)-3-(2-éthoxypyrimidin-5-yl)-6-fluoro-N-méthyl-9H-pyrido[2,3-b]indol-8-amine ;
le 5-(4-(3,6-diazabicyclo[3.1.1]heptan-6-yl)-6-fluoro-8-(méthylamino)-9H-pyrido[2,3-b]indol-3-yl)nicotinonitrile ;
la 4-(3,6-diazabicyclo[3.1.1]heptan-6-yl)-6-fluoro-3-(2-méthoxypyrimidin-5-yl)-N-méthyl-9H-pyrido[2,3-b]indol-8-amine ;
la 5,6-difluoro-N4,N4,N8-triméthyl-3-pyrazolo[1,5-a]pyrimidin-6-yl-9H-pyrido[2,3-b]indole-4,8-diamine ;
la 5,6-difluoro-3-(2-méthoxypyrimidin-5-yl)-N-méthyl-4-[(3aS,6aS)-5-méthyl-2,3,3a,4,6,6a-hexahydropyrrolo[2,3-c]pyrrol-1-yl]-9H-pyrido[2,3-b]indol-8-amine ;
l'acide 1-[5-[4-(diméthylamino)-8-(éthylamino)-6-fluoro-9H-pyrido[2,3-b]indol-3-yl]pyrimidin-2-yl]cyclopropanecarboxylique ;
l'acide 1-[5-[4-(diméthylamino)-5,6-difluoro-8-(méthylamino)-9H-pyrido[2,3-b]indol-3-yl]pyrimidin-2-yl]cyclopropanecarboxylique ;
l'acide 1-(5-(4-(diméthylamino)-6,7-difluoro-8-(méthylamino)-9H-pyrido[2,3-b]indol-3 -yl)pyrimidin-2-yl)cyclopropanecarboxylique ;
le 5-[4-[4-(aminométhyl)-3,3-difluoro-pyrrolidin-1-yl]-6-fluoro-8-(méthylamino)-9H-pyrido [2,3-b]indol-3 -yl]pyridine-3 -carbonitrile ;
la 4-[4-(aminométhyl)-3,3-difluoro-pyrrolidin-1-yl]-6-fluoro-3-(2-méthoxypyrimidin-5-yl)-N-méthyl-9H-pyrido [2,3-b]indol-8-amine ;
la 4-[4-(aminométhyl)-3,3-difluoro-pyrrolidin-1-yl]-5,6-difluoro-3-(2-méthoxypyrimidin-5-yl)-N-méthyl-9H-pyrido[2,3-b]indol-8-amine ;
le 5-[4-[4-(aminométhyl)-3,3-difluoro-pyrrolidin-1-yl]-5,6-difluoro-8-(méthylamino)-9H-pyrido [2,3-b]indol-3 -yl]pyridine-3 -carbonitrile ;
la 4-(1-(aminométhyl)cyclopropyl)-6-fluoro-3-(2-méthoxypyrimidin-5-yl)-N-méthyl-9H-pyrido[2,3-b]indol-8-amine ;
la *cis*-4-(2-amino-5-azaspiro[2.4]heptan-5-yl)-5,6-difluoro-N-méthyl-3-pyrazolo[1,5-a]pyrimidin-6-yl-9H-pyrido[2,3-b]indol-8-amine ;
la *trans*-4-(2-amino-5-azaspiro[2.4]heptan-5-yl)-5,6-difluoro-N-méthyl-3-pyrazolo[1,5-a]pyrimidin-6-yl-9H-pyrido[2,3-b]indol-8-amine ;
la 4- [(2R,3R)-2-amino-5-azaspiro[2.4]heptan-5-yl]-5,6-difluoro-N-méthyl-3 - pyrazolo[1,5-a]pyrimidin-6-yl-9H-pyrido[2,3-b]indol-8-amine ;
la 4-[(2S,3S)-2-amino-5 -azaspiro[2.4]heptan-5-yl]-5,6-difluoro-N-méthyl-3 - pyrazolo[1,5-a]pyrimidin-6-yl-9H-pyrido[2,3-b]indol-8-amine ;
la 4-[(2S,3R)-2-amino-5-azaspiro[2.4]heptan-5-yl]-5,6-difluoro-N-méthyl-3-pyrazolo[1,5-a]pyrimidin-6-yl-9H-pyrido[2,3-b]indol-8-amine ; et
la 4-[(2R,3 S)-2-amino-5-azaspiro[2.4]heptan-5-yl]-5,6-difluoro-N-méthyl-3-pyrazolo[1,5-a]pyrimidin-6-yl-9H-pyrido[2,3-b]indol-8-amine ;
ou un sel pharmaceutiquement acceptable de ceux-ci.

10. Procédé de préparation d'un composé selon l'une quelconque des revendications 1 à 9 comprenant la réaction d'un composé de formule (Ii), 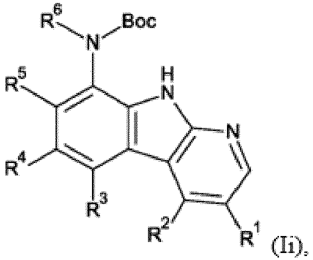 avec un acide, dans lequel R¹ à R⁶ sont tels que définis dans l'une quelconque des revendications 1 à 8.

11. Composé selon l'une quelconque des revendications 1 à 9 pour une utilisation comme substance thérapeutiquement active.

12. Composition pharmaceutique comprenant un composé selon l'une quelconque des revendications 1 à 9 et un véhicule thérapeutiquement inerte.

13. Composé selon l'une quelconque des revendications 1 à 9 pour une utilisation dans le traitement ou la prophylaxie d'une infection bactérienne.
